# EUROPEAN PATENT SPECIFICATION

(11) **EP 3 389 106 B1**
(45) Date of publication and mention of the grant of the patent: **01.12.2021**
(21) Application number: 16873048.9
(22) Date of filing: 07.12.2016
(51) Int. Cl.: H01L 51/54, C07C 201/00, C07D 403/04, C07D 491/048, C07D 495/04, C09K 11/06

(54) **ORGANIC ELECTROLUMINESCENT ELEMENT**
ORGANISCHES ELEKTROLUMINESZENTES ELEMENT
ÉLÉMENT ÉLECTROLUMINESCENT ORGANIQUE

(30) Priority: 08.12.2015 JP 2015239202
(43) Date of publication of application: 17.10.2018
(73) Proprietor: Hodogaya Chemical Co., Ltd., Tokyo 104-0028 (JP)
(72) Inventor: HAYASHI, Shuichi, Tokyo 104-0028 (JP); KABASAWA, Naoaki, Tokyo 104-0028 (JP); YAMAMOTO, Takeshi, Tokyo 104-0028 (JP); MOCHIZUKI, Shunji, Tokyo 104-0028 (JP)
(74) Representative: Müller-Boré & Partner Patentanwälte PartG mbB
(86) International application number: PCT/JP2016/086458
(87) International publication number: WO 2017/099155

(56) References cited:
- WO-A1-2013/087142
- WO-A1-2014/129201
- CN-A- 104 557 875
- JP-A- 2016 076 566
- JP-A- 2016 100 364
- KR-A- 20150 042 386
- KR-A- 20150 069 235
- KR-A- 20150 111 106
- MASAKI SHIMIZU, KENJI MOCHIDA, AND TAMEJIRO HIYAMA: "Highly Efficient Blue Fluorescence from 3,20-Silylene-Bridged2-Phenylindoles in the Solid State", THE JOURNAL OF PHYSICAL CHEMISTRY C, vol. 115, 18 May 2011 (2011-05-18), pages 11265-11274, XP002792681,

## Description

### Technical Field

This invention relates to an organic electroluminescent device (will hereinafter be referred to simply as an organic EL device) which is a self light-emitting device preferred for various displays. More specifically, the invention relates to an organic EL device using a specific arylamine compound and a specific indenoindole compound or a specific carbazole compound (and a specific anthracene derivative).

### Background Art

Since an organic EL device is a self light-emitting device, it is brighter, better in visibility, and capable of clearer display, than a liquid crystal device. Hence, energetic researches have been conducted on organic EL devices.

In 1987, C.W. Tang et al. of Eastman Kodak developed a laminated structure device sharing various roles among different materials, thereby imparting practical applicability to organic EL devices using organic materials. Such an organic EL device is formed by laminating a layer of a fluorescent body capable of transporting electrons, and a layer of an organic substance capable of transporting holes. Because of this configuration, the organic EL device injected positive charges and negative charges into the layer of the fluorescent body to perform light emission, thereby obtaining a high luminance of 1, 000 cd/m² or more at a voltage of 10V or less (see Patent Document 1 and Patent Document 2).

Many improvements have been made to date for commercialization of organic EL devices. For example, there is an electroluminescent device in which the roles of the respective layers in a laminated structure are rendered more diverse, and an anode, a hole injection layer, a hole transport layer, a luminous layer, an electron transport layer, an electron injection layer, and a cathode are provided sequentially on a substrate. High efficiency and high durability have come to be achieved by such an electroluminescent device.

For a further increase in the luminous efficiency, it has been attempted to utilize triplet excitons, and the utilization of phosphorescent compounds has been considered. Furthermore, devices utilizing light emission by thermally activated delayed fluorescence (TADF) have been developed. In 2011, Adachi et al. of Kyushu University realized an external quantum efficiency of 5.3% by a device using a thermally activated delayed fluorescence material.

The luminous layer can also be prepared by doping a charge transporting compound, generally called a host material, with a fluorescent compound, a phosphorescent compound, or a material radiating delayed fluorescence. The selection of the organic material in the organic EL device greatly affects the characteristics of the device, such as efficiency and durability.

With the organic EL device, the charges injected from both electrodes recombine in the luminous layer to obtain light emission. For this purpose, how efficiently the charges of the holes and the electrons are passed on to the luminous layer is of importance in the organic EL device, and the device needs to be excellent in carrier balance. Moreover, the hole injecting properties are enhanced, and the electron blocking properties of blocking electrons injected from the cathode are enhanced, whereby the probability of the holes and the electrons recombining is increased. Besides, excitons generated within the luminous layer are confined. By so doing, a high luminous efficiency can be obtained. Thus, the role of the hole transport material is so important that there has been a desire for a hole transport material having high hole injection properties, allowing marked hole mobility, possessing high electron blocking properties, and having high durability to electrons.

From the viewpoint of device lifetime, heat resistance and amorphousness of the material are also important. A material with low thermal resistance is thermally decomposed even at a low temperature by heat produced during device driving, and the material deteriorates. With a material having low amorphousness, crystallization of a thin film occurs even in a short time, and the device deteriorates. Thus, high resistance to heat and satisfactory amorphousness are required of the material to be used.

As hole transport materials for organic EL devices, N,N'-diphenyl-N,N'-di(α-naphthyl)benzidine (NPD) and various aromatic amine derivatives have been known (see Patent Documents 1 and 2). NPD has satisfactory hole transport capability, but its glass transition point (Tg) serving as an index of heat resistance is as low as 96°C. Under high temperature conditions, it causes decline in device characteristics due to crystallization. Among the aromatic amine derivatives described in Patent Documents 1 and 2 are compounds having excellent hole mobility of 10⁻³ cm²/Vs or more, but the electron blocking properties of such aromatic amine derivatives are insufficient. In organic EL devices formed using such aromatic amine derivatives, therefore, some of electrons pass through the luminous layer, and an increase in the luminous efficiency cannot be expected. Thus, there has been a desire for a material having higher electron blocking properties, more stable in the form of a thin film, and possessing higher resistance to heat, in order to achieve an even higher efficiency. Patent Document 3 discloses aromatic amine derivatives with high durability. However, such aromatic amine derivatives are used as charge transport materials for electrophotographic photoreceptors, and there have been no examples of their use in organic EL devices.

As compounds improved in characteristics such as heat resistance and hole injection properties, arylamine compounds having substituted carbazole structures are disclosed in Patent Documents 4 and 5. In devices using these compounds as hole injection layers or hole transport layers, heat resistance and luminous efficiency have been improved. However, the improved characteristics have been still insufficient, and an even lower driving voltage and an even higher luminous efficiency are desired.

As noted above, it is desired to combine materials excellent in hole injection/transport performance, electron injection/transport performance, stability in a thin film state, or durability, in order to improve the device characteristics, and increase the yield of device preparation of organic EL devices. Realization of a device with a high luminous efficiency, a low driving voltage, and a long lifetime through these efforts is desired. Patent Document 12 discloses a substituted triarylamine compound which is suitable as a hole transport material in organic electronic devices.

### Prior Art Documents

### Patent Documents

Patent Document 1: JP-A-H8-048656
Patent Document 2: Japanese Patent No. 3194657
Patent Document 3: Japanese Patent No. 4943840
Patent Document 4: JP-A-2006-151979
Patent Document 5: WO2008/62636
Patent Document 6: JP-T-2014-513064
Patent Document 7: WO2011/059000
Patent Document 8: WO2003/060956
Patent Document 9: Korean Unexamined Patent Publication No. 2013-060157
Patent Document 10: JP-A-H7-126615
Patent Document 11: JP-A-2005-108804
Patent Document 12: WO 2013/087142

### Summary of the Invention

### Problems to be solved by the invention

The present invention involves combining various materials for an organic EL device, which are excellent in hole injection/transport performance, electron injection/transport performance, electron blocking capability, stability in a thin film state, or durability so that the characteristics possessed by the respective materials can be exhibited effectively. It is an object of the present invention to provide, by so doing, an organic EL device which (1) is high in luminous efficiency and power efficiency, (2) is low in practical driving voltage, and (3), in particular, has a long lifetime.

### Means for solving the problems

In an attempt to attain the above object, the present inventors paid attention to the fact that arylamine-based materials were excellent in hole injection/transport capability, thin film stability, and durability. They speculated that a hole transport layer formed using such a material would be able to inject and transport holes into a luminous layer efficiently. They also noted that compounds having an indenoindole ring structure and compounds having a carbazole ring structure were excellent in luminous efficiency. The selected such a compound as a material for a luminous layer, and further combined a hole transport material with the compound for the luminous layer so that a carrier balance would be achieved in conformity with the characteristics of the material for the luminous layer. Using these combinations, they prepared various organic EL devices, and energetically evaluated the characteristics of the devices.

Moreover, the present inventors noted that compounds having an anthracene ring structure were excellent in electron injection/transport capability, thin film stability, and durability. They combined a specific compound having an anthracene ring structure, as a material for an electron transport layer, with the material for a hole transport layer and the material for a luminous layer mentioned above, thereby increasing the efficiencies of electron injection and transport into the luminous layer so that a carrier balance would further fit the characteristics of the material for the luminous layer. They energetically evaluated the characteristics of the resulting device.

Furthermore, the present inventors formed the hole transport layer into a two-layer structure composed of a first hole transport layer and a second hole transport layer. They used the above arylamine-based material having excellent electron blocking properties for the second hole transport layer, and selected specific two types of arylamine compounds as the material for the first hole transport layer, that is, they selected combinations of the materials with carrier balance elaborated, so that holes can be injected and transported efficiently into the luminous layer. Using the so selected combinations, they prepared various organic EL devices, and energetically evaluated the characteristics of the devices. As a result, they have accomplished the present invention.

That is, according to the present invention,
1) There is provided an organic electroluminescent device having at least an anode, a hole transport layer, a luminous layer, an electron transport layer, and a cathode in this order, wherein
   the hole transport layer contains an arylamine compound represented by the following general formula (1), and
   the luminous layer contains an indenoindole compound represented by the following general formula (2) or a carbazole compound represented by the following general formula (3): 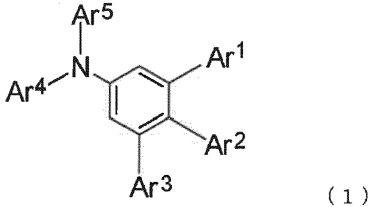 where
      Ar¹ to Ar⁵ may be identical or different, and each represents an aromatic hydrocarbon group, an aromatic heterocyclic group, or a condensed polycyclic aromatic group. 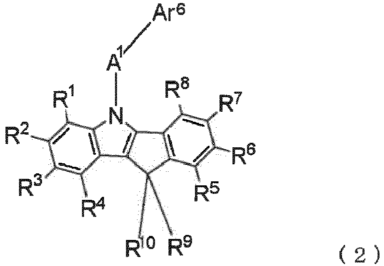 where
         A¹ represents a divalent group of an aromatic hydrocarbon, a divalent group of an aromatic heterocycle, a divalent group of a condensed polycyclic aromatic, or a single bond,
         Ar⁶ represents an aromatic hydrocarbon group, an aromatic heterocyclic group, or a condensed polycyclic aromatic group,
         R¹ to R⁸ may be identical or different, and each represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, an aryloxyl group, or a di-substituted amino group substituted by a group selected from an aromatic hydrocarbon group, an aromatic heterocyclic group, and a condensed polycyclic aromatic group; R¹ to R⁴ may bind to each other via a single bond, a substituted or unsubstituted methylene group, an oxygen atom, or a sulfur atom to form a ring; R⁵ to R⁸ may bind to each other via a single bond, a substituted or unsubstituted methylene group, an oxygen atom, or a sulfur atom to form a ring; furthermore, to a vacant site occurring upon elimination of some of R¹ to R⁴ from a benzene ring, the other group of R¹ to R⁴ may bind via a substituted or unsubstituted methylene group, an oxygen atom, a sulfur atom, or a monoarylamino group to form a ring; and to a vacant site occurring upon elimination of some of R⁵ to R⁸ from a benzene ring, the other group of R⁵ to R⁸ may bind via a substituted or unsubstituted methylene group, an oxygen atom, a sulfur atom, or a monoarylamino group to form a ring, and
         R⁹ and R¹⁰ may be identical or different, and each represents an alkyl group having 1 to 6 carbon atoms, an aromatic hydrocarbon group, an aromatic heterocyclic group, or a condensed polycyclic aromatic group, and may bind to each other via a single bond, a substituted or unsubstituted methylene group, an oxygen atom, or a sulfur atom to form a ring. 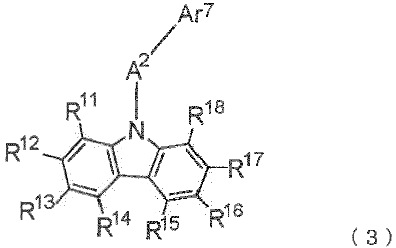 where
            A² represents a divalent group of an aromatic hydrocarbon, a divalent group of an aromatic heterocycle, a divalent group of a condensed polycyclic aromatic, or a single bond,
            Ar⁷ represents an aromatic hydrocarbon group, an aromatic heterocyclic group, or a condensed polycyclic aromatic group,
            R¹¹ to R¹⁸ may be identical or different, and each represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, an aryloxyl group, or a di-substituted amino group substituted by a group selected from an aromatic hydrocarbon group, an aromatic heterocyclic group, and a condensed polycyclic aromatic group; R¹¹ to R¹⁴ may bind to each other via a single bond, a substituted or unsubstituted methylene group, an oxygen atom, or a sulfur atom to form a ring; R¹⁵ to R¹⁸ may bind to each other via a single bond, a substituted or unsubstituted methylene group, an oxygen atom, or a sulfur atom to form a ring; furthermore, to a vacant site occurring upon elimination of some of R¹¹ to R¹⁴ from a benzene ring, the other group of R¹¹ to R¹⁴ may bind via a substituted or unsubstituted methylene group, an oxygen atom, a sulfur atom, or a monoarylamino group to form a ring; and to a vacant site occurring upon elimination of some of R¹⁵ to R¹⁸ from a benzene ring, the other group of R¹⁵ to R¹⁸ may bind via a substituted or unsubstituted methylene group, an oxygen atom, a sulfur atom, or a monoarylamino group to form a ring.

Preferred embodiments of the organic EL device according to the present invention are as follows:
2) The electron transport layer contains an anthracene derivative represented by the following general formula (4): 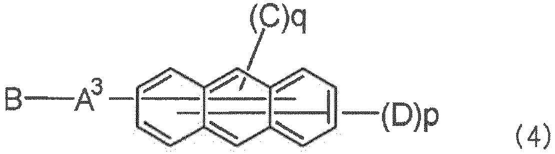 where
   A³ represents a divalent group of an aromatic hydrocarbon, a divalent group of an aromatic heterocycle, a divalent group of a condensed polycyclic aromatic, or a single bond,
   B represents an aromatic heterocyclic group,
   C represents an aromatic hydrocarbon group, an aromatic heterocyclic group, or a condensed polycyclic aromatic group and, if there are two of Cs, the two Cs may be identical or different,
   Ds may be identical or different, and each represents a hydrogen atom, a deuterium atom, a fluorine atom, a chlorine atom, a cyano group, a trifluoromethyl group, an alkyl group having 1 to 6 carbon atoms, an aromatic hydrocarbon group, an aromatic heterocyclic group, or a condensed polycyclic aromatic group, and
   provided that the sum of p and q is 9, p denotes 7 or 8, while q denotes 1 or 2.
3) The above anthracene derivative is represented by the following general formula (4a): 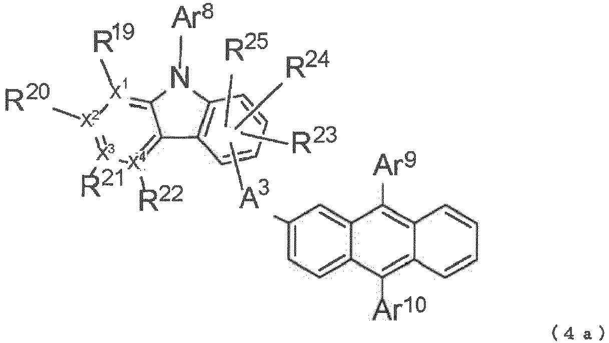 where
   A³ has a meaning as defined in the general formula (4),
   Ar⁸ to Ar¹⁰ may be identical or different, and each represents an aromatic hydrocarbon group, an aromatic heterocyclic group, or a condensed polycyclic aromatic group,
   R¹⁹ to R²⁵ may be identical or different, and each represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group, and may bind to each other via a single bond, a substituted or unsubstituted methylene group, an oxygen atom, or a sulfur atom to form a ring, and
   X¹ to X⁴ may be identical or different, and each represents a carbon atom or a nitrogen atom, and only one of X¹ to X⁴ is a nitrogen atom with the proviso that the nitrogen atom in this case does not have the hydrogen atom or substituent of R¹⁹ to R²².
4) The above anthracene derivative is represented by the following general formula (4b): 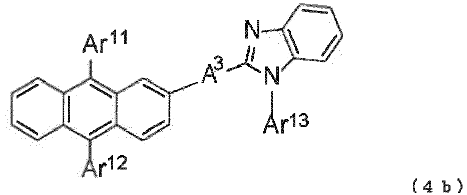 where
   A³ has a meaning as defined in the general formula (4), and
   Ar¹¹ to Ar¹³ may be identical or different, and each represents an aromatic hydrocarbon group, an aromatic heterocyclic group, or a condensed polycyclic aromatic group.
5) The above anthracene derivative is represented by the following general formula (4c): 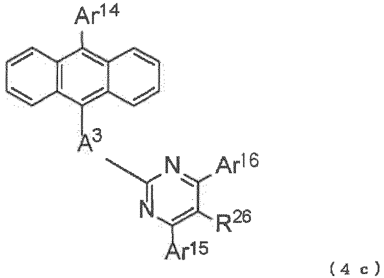 where
   A³ has a meaning as defined in the general formula (4),
   Ar¹⁴ to Ar¹⁶ may be identical or different, and each represents an aromatic hydrocarbon group, an aromatic heterocyclic group, or a condensed polycyclic aromatic group, and
   R²⁶ represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group.
6) The hole transport layer has a two-layer structure composed of a first hole transport layer and a second hole transport layer, and the second hole transport layer is located beside the luminous layer and contains the arylamine compound represented by the general formula (1).
7) The luminous layer contains a red light emitting material.
8) The luminous layer contains a phosphorescence-emitting material.
9) The phosphorescence-emitting material is a metal complex containing iridium or platinum.

### Effects of the invention

The arylamine compound represented by the general formula (1) (may hereinafter be referred to as an arylamine compound I) is higher in hole injection/transport performance and electron blocking capability than conventional hole transport materials . Moreover, the arylamine compound is stable in a thin film state and excellent in heat resistance. Thus, it is preferred as a material constituting the hole transport layer. Furthermore, it can be used as a constituent material for the hole injection layer or the electron blocking layer.

Also, with the present invention, the indenoindole compound represented by the general formula (2) (may hereinafter be referred to as an indenoindole compound II) and the carbazole compound represented by the general formula (3) (may hereinafter be referred to as a carbazole compound III) are preferably used as constituent materials for the luminous layer, preferably as host materials for the luminous layer, particularly preferably as host materials for the luminous layer having the phosphorescence-emitting material. This is because the indenoindole compound II and the carbazole compound III are excellent in luminous efficiency as compared with conventional materials.

With the present invention, moreover, the anthracene derivative represented by the general formula (4) (may hereinafter be referred to as an anthracene derivative IV) is preferably used as a constituent material for the electron transport layer. The reason is that the anthracene derivative IV is excellent in electron injection and transport capabilities, and also excels in stability in a thin film state and in durability.

With the present invention, as described above, the materials excellent in hole injection/transport performance, electron injection/transport performance, thin film stability, and durability are selected, and the hole transport layer using the arylamine compound having a specific structure is combined with the luminous layer using the material having a specific structure, with the carrier balance being taken into consideration. By so doing, the organic EL device of the present invention can transport holes from the hole transport layer to the luminous layer with good efficiency as compared with conventional organic EL devices. As a preferred embodiment, moreover, there is an embodiment in which an electron transport layer using an anthracene derivative having a specific structure is provided in an organic EL device having such a structure. Because of this feature, the efficiency of electron transport from the electron transport layer to the luminous layer is also increased.

According to the present invention, as described above, an organic EL device having a high luminous efficiency, working at a low driving voltage, excelling in durability, and possessing a particularly long lifetime has been realized.

### Brief Description of the Drawings

[Fig. 1] is a view showing the configuration of organic EL devices of Device Examples and Device Comparative Examples.
[Fig. 2] is a view showing the structural formulas of Compounds 1-1 to 1-10 which are arylamine compounds I.
[Fig. 3] is a view showing the structural formulas of Compounds 1-11 to 1-18 which are arylamine compounds I.
[Fig. 4] is a view showing the structural formulas of Compounds 1-19 to 1-28 which are arylamine compounds I.
[Fig. 5] is a view showing the structural formulas of Compounds 1-29 to 1-36 which are arylamine compounds I.
[Fig. 6] is a view showing the structural formulas of Compounds 1-37 to 1-44 which are arylamine compounds I.
[Fig. 7] is a view showing the structural formulas of Compounds 1-45 to 1-52 which are arylamine compounds I.
[Fig. 8] is a view showing the structural formulas of Compounds 1-53 to 1-60 which are arylamine compounds I.
[Fig. 9] is a view showing the structural formulas of Compounds 1-61 to 1-68 which are arylamine compounds I.
[Fig. 10] is a view showing the structural formulas of Compounds 1-69 to 1-76 which are arylamine compounds I.
[Fig. 11] is a view showing the structural formulas of Compounds 1-77 to 1-84 which are arylamine compounds I.
[Fig. 12] is a view showing the structural formulas of Compounds 1-85 to 1-94 which are arylamine compounds I.
[Fig. 13] is a view showing the structural formulas of Compounds 1-95 to 1-100 which are arylamine compounds I.
[Fig. 14] is a view showing the structural formulas of Compounds 2-1 to 2-8 which are indenoindole compounds II.
[Fig. 15] is a view showing the structural formulas of Compounds 2-9 to 2-15 which are indenoindole compounds II.
[Fig. 16] is a view showing the structural formulas of Compounds 3-1 to 3-8 which are carbazole compounds III.
[Fig. 17] is a view showing the structural formulas of Compounds 3-9 to 3-16 which are carbazole compounds III.
[Fig. 18] is a view showing the structural formulas of Compounds 3-17 to 3-23 which are carbazole compounds III.
[Fig. 19] is a view showing the structural formulas of Compounds 4a-1 to 4a-8 which are anthracene derivatives IV.
[Fig. 20] is a view showing the structural formulas of Compounds 4a-9 to 4a-16 which are anthracene derivatives IV.
[Fig. 21] is a view showing the structural formulas of Compounds 4a-17 to 4a-20 which are anthracene derivatives IV.
[Fig. 22] is a view showing the structural formulas of Compounds 4b-1 to 4b-8 which are anthracene derivatives IV.
[Fig. 23] is a view showing the structural formulas of Compounds 4b-9 to 4b-16 which are anthracene derivatives IV.
[Fig. 24] is a view showing the structural formulas of Compounds 4c-1 to 4c-6 which are anthracene derivatives IV.
[Fig. 25] is a view showing the structural formulas of Compounds 4c-7 to 4c-12 which are anthracene derivatives IV.
[Fig. 26] is a view showing the structural formulas of Compounds 4c-13 to 4c-18 which are anthracene derivatives IV.
[Fig. 27] is a view showing the structural formulas of Compounds 4c-19 to 4c-24 which are anthracene derivatives IV.
[Fig. 28] is a view showing the structural formulas of Compounds 4c-25 to 4c-30 which are anthracene derivatives IV.
[Fig. 29] is a view showing the structural formulas of Compounds 5-1 to 5-8 which are triarylamine compounds V.
[Fig. 30] is a view showing the structural formulas of Compounds 5-9 to 5-16 which are triarylamine compounds V.
[Fig. 31] is a view showing the structural formulas of Compounds 5-17 to 5-23 which are triarylamine compounds V.
[Fig. 32] is a view showing the structural formulas of compounds other than triarylamine compounds V among triarylamine compounds having two triarylamine structures.
[Fig. 33] is a view showing the structural formulas of Compounds 6-1 to 6-3 which are triarylamine compounds VI.
[Fig. 34] is a view showing the structural formulas of Compounds 6-4 to 6-6 which are triarylamine compounds VI.
[Fig. 35] is a view showing the structural formulas of Compounds 6-7 to 6-10 which are triarylamine compounds VI.
[Fig. 36] is a view showing the structural formulas of Compounds 6-11 to 6-14 which are triarylamine compounds VI.
[Fig. 37] is a view showing the structural formulas of Compounds 6-15 to 6-17 which are triarylamine compounds VI.

### Mode for Carrying Out the Invention

The organic EL device of the present invention has a basic structure in which at least an anode, a hole transport layer, a luminous layer, an electron transport layer, and a cathode are provided in this sequence on a substrate.

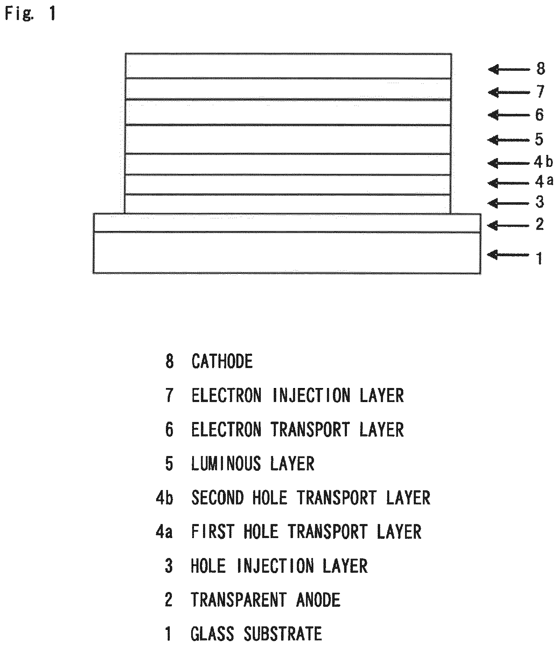

As long as the organic EL device of the present invention has such a basic structure, its layer structure can take various forms. For example, it is possible to provide a hole injection layer between the anode and the hole transport layer, provide an electron blocking layer between the hole transport layer and the luminous layer, provide a hole blocking layer between the luminous layer and the electron transport layer, or provide an electron injection layer between the electron transport layer and the cathode. Moreover, some organic layers can be omitted, or the roles of some organic layers can be fulfilled by any other layer. For example, a layer concurrently serving as the hole injection layer and the hole transport layer can be formed, or a layer concurrently serving as the electron injection layer and the electron transport layer can be formed. Furthermore, a configuration in which two or more organic layers having the same function are laminated can be adopted. For example, it is possible to adopt a configuration in which two of the hole transport layers are laminated, a configuration in which two of the luminous layers are laminated, or a configuration in which two of the electron transport layers are laminated. Fig. 1 shows a layer configuration adopted in the Examples to be described later, namely, a layer configuration in which a transparent anode 2, a hole injection layer 3, a first hole transport layer 4a, a second hole transport layer 4b, a luminous layer 5, an electron transport layer 6, an electron injection layer 7, and a cathode 8 are formed in this sequence on a glass substrate 1.

As the respective layers will be described in detail later, the present invention is importantly characterized in that the hole transport layer contains an arylamine compound I represented by the general formula (1), and that the luminous layer contains an indenoindole compound II represented by the general formula (2) or a carbazole compound III represented by the general formula (3). The arylamine compound I, the indenoindole compound II, and the carbazole compound III will be described below.

### <Arylamine compound I>

The arylamine compound I contained in the hole transport layer has a structure represented by the following general formula (1):

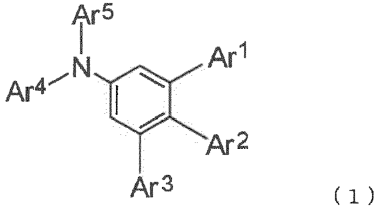

### (Ar¹ to Ar⁵)

Ar¹ to Ar⁵ may be identical or different, and each represents an aromatic hydrocarbon group, an aromatic heterocyclic group, or a condensed polycyclic aromatic group. Herein, the condensed polycyclic aromatic group does not have a heteroatom (e.g. , a nitrogen atom, an oxygen atom, or a sulfur atom) in its skeleton.

Examples of the aromatic hydrocarbon group, the aromatic heterocyclic group, or the condensed polycyclic aromatic group, represented by Ar¹ to Ar⁵, include a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, a quinazolinyl group, a benzoquinazolinyl group, and a pyridopyrimidinyl group.

The aromatic hydrocarbon group, the aromatic heterocyclic group, or the condensed polycyclic aromatic group, represented by Ar¹ to Ar⁵, may be unsubstituted, but may have a substituent. The substituent can be exemplified by the following groups, in addition to a deuterium atom, a cyano group, and a nitro group:
a halogen atom, for example, a fluorine atom, a chlorine atom, a bromine atom, or an iodine atom;
an alkyl group having 1 to 6 carbon atoms, for example, a methyl group, an ethyl group, an n-propyl group, an isopropyl group, an n-butyl group, an isobutyl group, a tert-butyl group, an n-pentyl group, an isopentyl group, a neopentyl group, or an n-hexyl group;
an alkyloxy group having 1 to 6 carbon atoms, for example, a methyloxy group, an ethyloxy group, or a propyloxy group;
an alkenyl group, for example, a vinyl group or an allyl group;
an aryloxy group, for example, a phenyloxy group or a tolyloxy group;
an arylalkyloxy group, for example, a benzyloxy group or a phenethyloxy group;
an aromatic hydrocarbon group or a condensed polycyclic aromatic group, for example, a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group;
an aromatic heterocyclic group, for example, a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, or a carbolinyl group;
an arylvinyl group, for example, a styryl group, or a naphthylvinyl group; and
an acyl group, for example, an acetyl group, or a benzoyl group,
the alkyl group having 1 to 6 carbon atoms.

The alkyloxy group having 1 to 6 carbon atoms, and the alkenyl group being optionally straight-chain or branched. Any of these substituents may be unsubstituted, but may be further substituted by the above exemplary substituent. These substituents may be present independently of each other so as not to form a ring. However, they may bind to each other via a single bond, a substituted or unsubstituted methylene group, an oxygen atom, or a sulfur atom to form a ring.

### (Preferred embodiments)

Preferred embodiments of the arylamine compound I will be described below. In the description of the preferred embodiments, the groups without the designation of "substituted"/"unsubstituted" may have a substituent or may be unsubstituted.

As Ar¹ and Ar³, an aromatic hydrocarbon group, or a condensed polycyclic aromatic group is preferred. Concretely, a phenyl group, a biphenylyl group, a terphenylyl group, a naphthyl group, a phenanthrenyl group, an anthracenyl group, or a fluorenyl group is preferred, and a phenyl group or a biphenylyl group is more preferred. These groups may be identical or different, but preferably are identical. From the viewpoint of vapor depositability, they are more preferably unsubstituted.

As Ar², an aromatic hydrocarbon group or a condensed polycyclic aromatic group is preferred. A phenyl group, a biphenylyl group, a terphenylyl group, a naphthyl group, a phenanthrenyl group, an anthracenyl group, or a fluorenyl group is more preferred. A phenyl group or a biphenylyl group is particularly preferred. As a substituent which the phenyl group may have, a condensed polycyclic aromatic group is preferred, a naphthyl group, a phenanthrenyl group or an anthracenyl group is more preferred, and a naphthyl group is particularly preferred.

Ar⁴ and Ar⁵ may be the same or different, and an aromatic hydrocarbon group or a condensed polycyclic aromatic group is preferred. From the viewpoint of vapor depositability, the number of the carbon atoms of Ar⁴ or Ar⁵ is preferably 6 to 30, and more preferably 6 to 25. Concretely, a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group is preferred. More preferred is a phenyl group, a biphenylyl group, a terphenylyl group, a naphthyl group, a phenanthrenyl group, an anthracenyl group, a fluorenyl group, or a spirobifluorenyl group. As a substituent which the phenyl group may have, a naphthyl group is preferred. As a substituent which the fluorenyl group may have, a methyl group or a phenyl group is preferred.

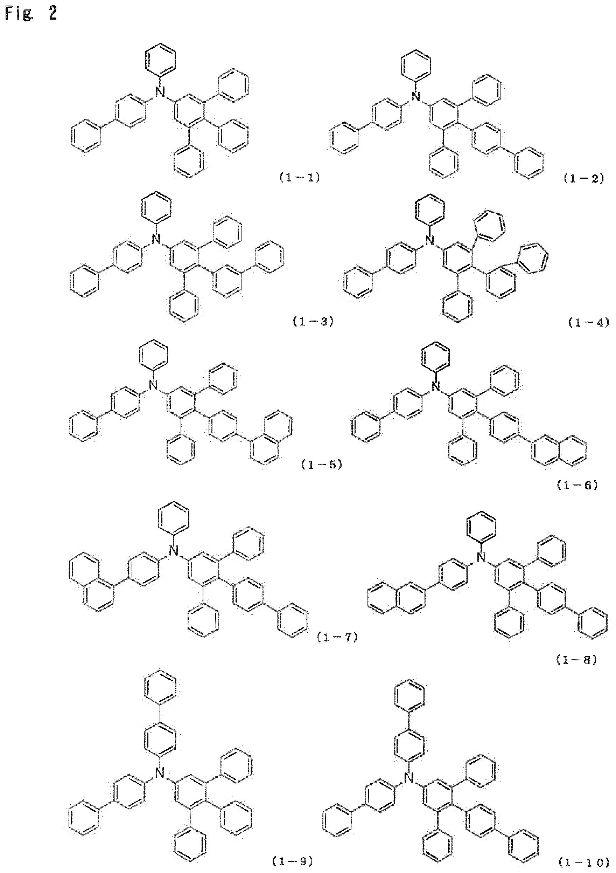

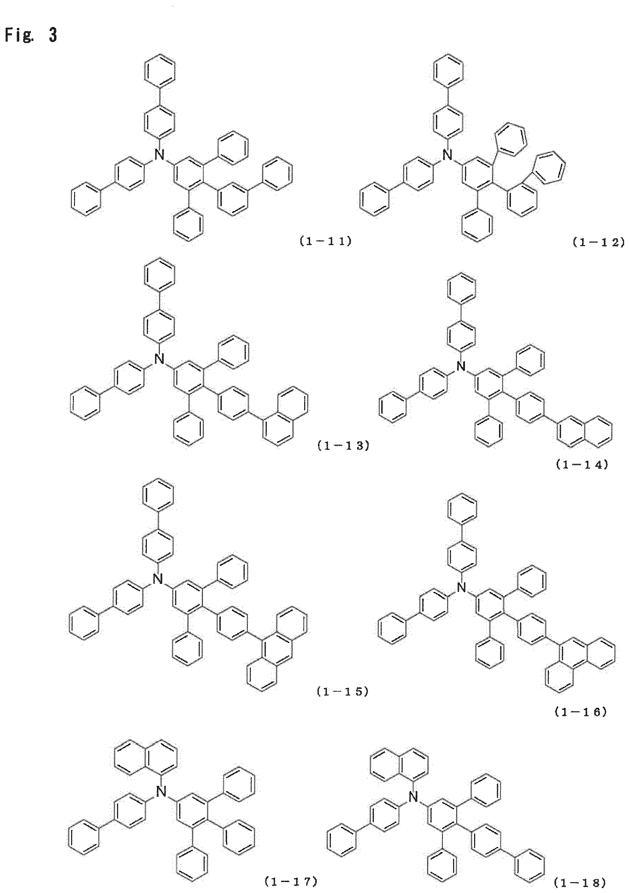

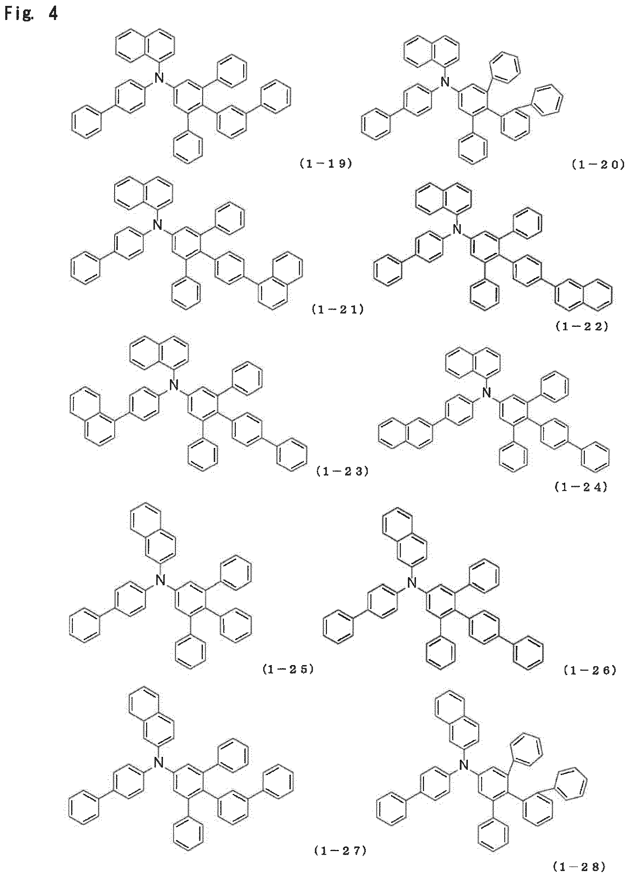

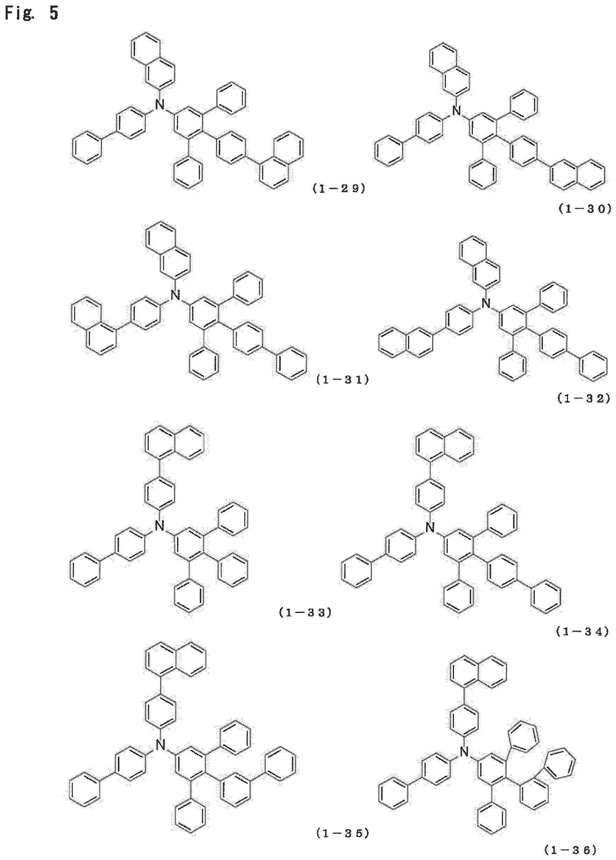

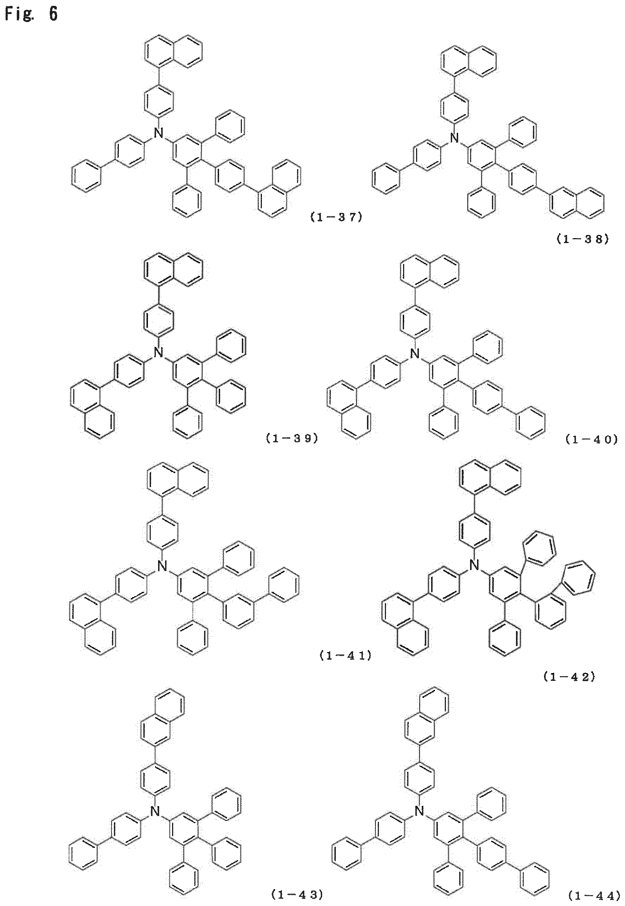

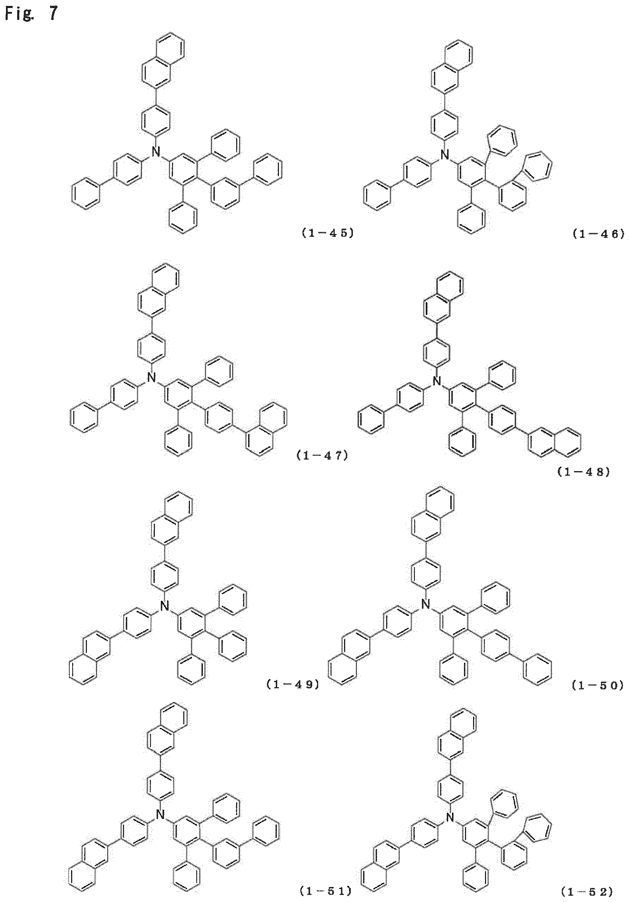

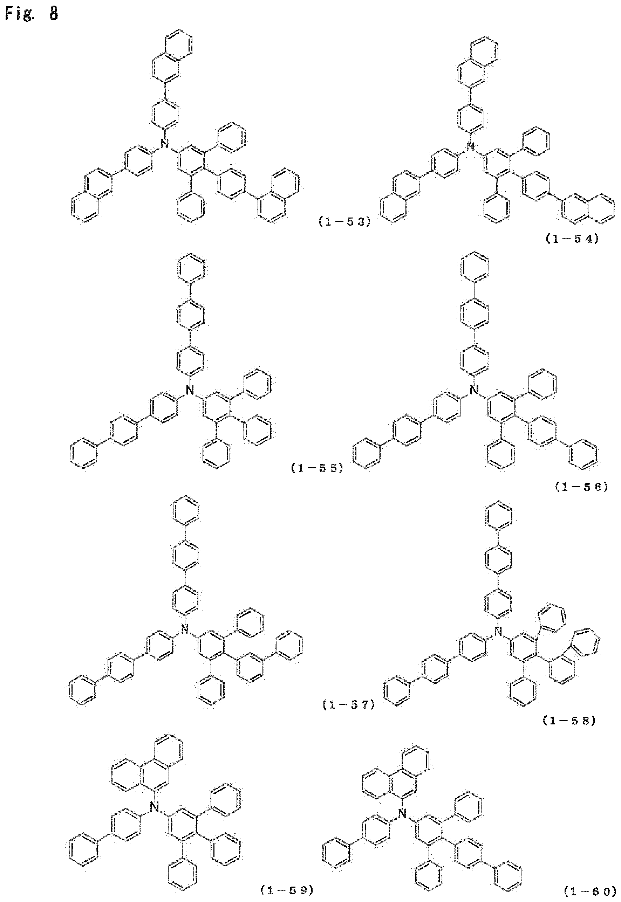

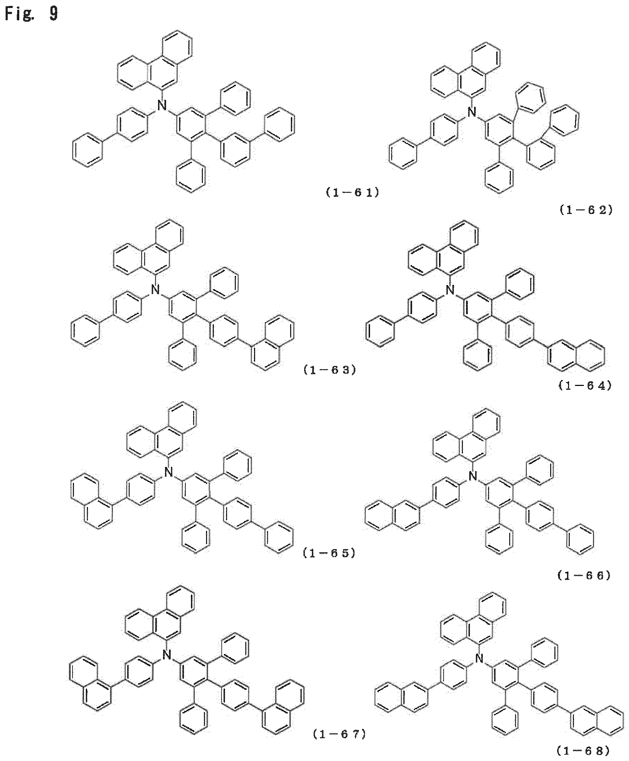

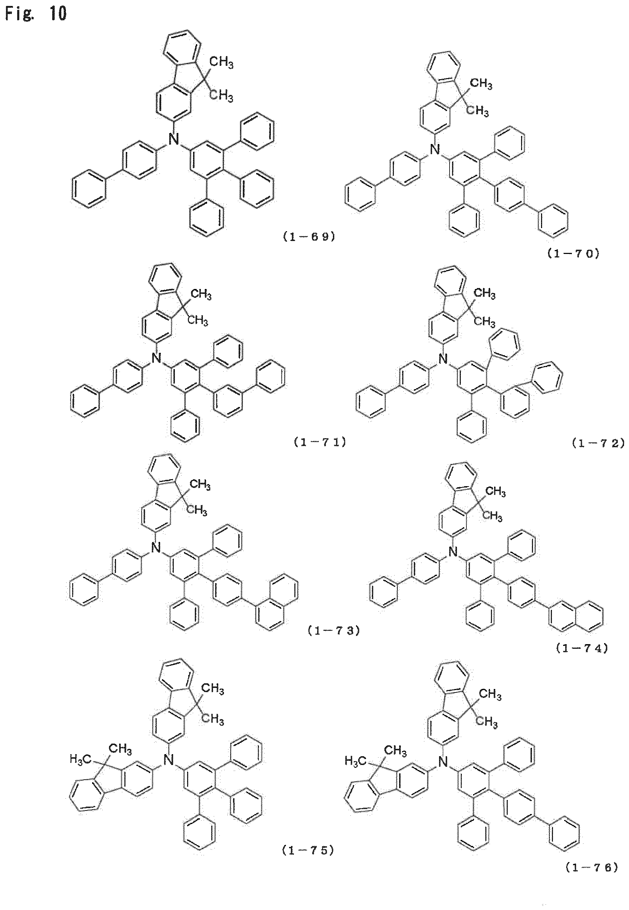

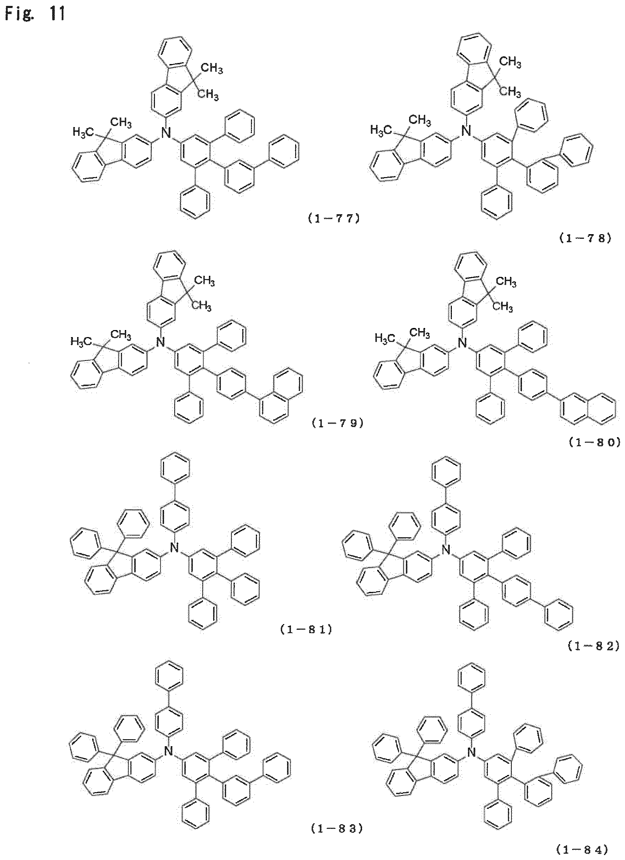

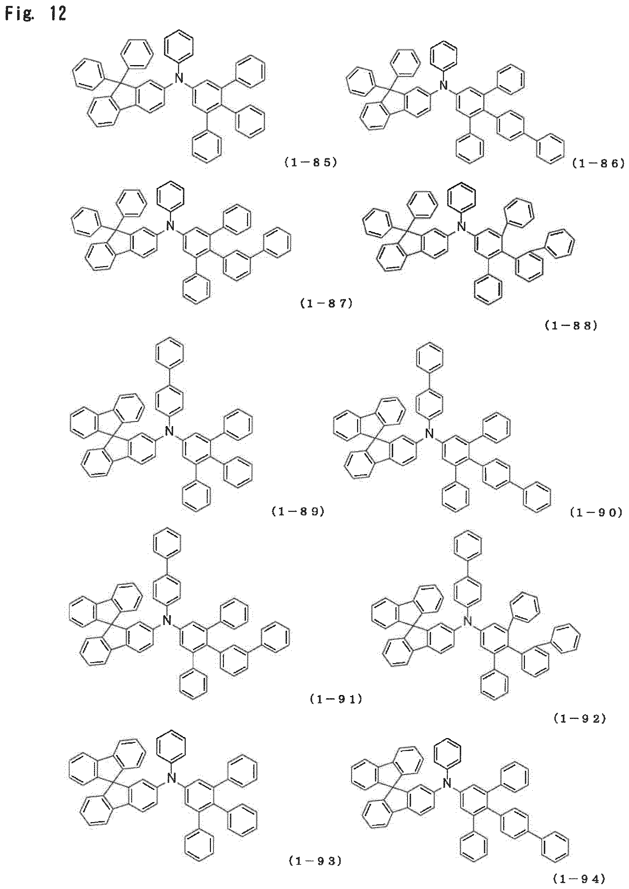

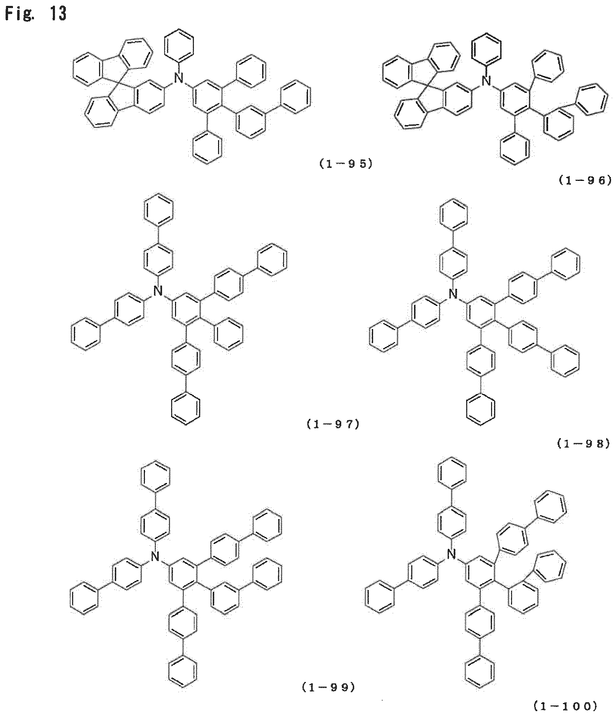

Preferred examples of the arylamine compound I are shown in Figs. 2 to 13, but the arylamine compounds I are not limited to these compounds.

The arylamine compound I can be synthesized by a publicly known method, and can be produced, for example, by cross coupling such as Suzuki coupling.

The purification of the arylamine compound I can be performed, for example, by purification using a column chromatograph, adsorption purification using silica gel, activated carbon, activated clay or the like, or recrystallization or crystallization using a solvent. Finally, purification by sublimation purification or the like may be performed. The identification of the compounds can be made by NMR analysis. As the physical properties, the glass transition point (Tg) and the work function can be measured.

The glass transition point (Tg) serves as an index to stability in a thin film state. The glass transition point (Tg) can be measured with a high sensitivity differential scanning calorimeter (DSC3100SA, manufactured by Bruker AXS) using a powder.

The work function serves as an index to hole transport properties. The work function can be measured by preparing a 100 nm thin film on an ITO substrate, and making a measurement using an ionization potential measuring device (PYS-202, manufactured by Sumitomo Heavy Industries, Ltd.).

Compounds for use in the organic EL device of the present invention, which are other than the arylamine compound I, (e.g. , indenoindole compounds II, carbazole compounds III, anthracene derivatives IV, triarylamine compounds V, and triarylamine compounds VI to be described later), can also be purified and subjected to various measurements by similar methods after their synthesis.

### <Indenoindole compound II>

The indenoindole compound II contained in the luminous layer has a structure represented by the following general formula (2):

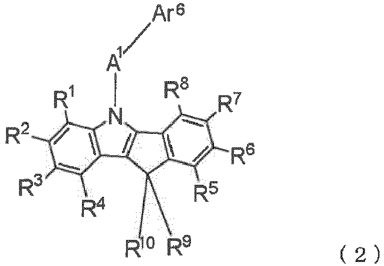

### (A¹)

A¹ represents a divalent group of an aromatic hydrocarbon, a divalent group of an aromatic heterocycle, a divalent group of a condensed polycyclic aromatic, or a single bond.

Herein, the divalent group of an aromatic hydrocarbon, the divalent group of an aromatic heterocycle, or the divalent group of a condensed polycyclic aromatic refers to a divalent group formed by removing two hydrogen atoms from an aromatic hydrocarbon, an aromatic heterocycle, or a condensed polycyclic aromatic.

Examples of the divalent group of an aromatic hydrocarbon, the divalent group of an aromatic heterocycle, or the divalent group of a condensed polycyclic aromatic, represented by A¹, include benzene, biphenyl, terphenyl, tetrakisphenyl, styrene, naphthalene, anthracene, acenaphthalene, fluorene, phenanthrene, indane, pyrene, triphenylene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

The divalent group of an aromatic hydrocarbon, the divalent group of an aromatic heterocycle, or the divalent group of a condensed polycyclic aromatic, represented by A¹, may be unsubstituted, but may have a substituent. Examples of the substituent are the same as those shown in connection with the substituent that the aromatic hydrocarbon group, the aromatic heterocyclic group, or the condensed polycyclic aromatic group represented by Ar¹ to Ar⁵ in the general formula (1) may have. The same holds true of the embodiments that the substituent can adopt.

### (Ar⁶)

Ar⁶ represents an aromatic hydrocarbon group, an aromatic heterocyclic group, or a condensed polycyclic aromatic group.

The aromatic hydrocarbon group, the aromatic heterocyclic group, or the condensed polycyclic aromatic group, represented by Ar⁶, can be exemplified by the same as those shown in connection with the aromatic hydrocarbon group, the aromatic heterocyclic group, or the condensed polycyclic aromatic group represented by Ar¹ to Ar⁵ in the general formula (1) .

The aromatic hydrocarbon group, the aromatic heterocyclic group, or the condensed polycyclic aromatic group, represented by Ar⁶, may be unsubstituted, but may have a substituent. Examples of the substituent are the same as those shown in connection with the substituent that the aromatic hydrocarbon group, the aromatic heterocyclic group, or the condensed polycyclic aromatic group represented by Ar¹ to Ar⁵ in the general formula (1) may have. The same holds true of the embodiments that the substituent can adopt.

### (R¹ to R⁸)

R¹ to R⁸ may be the same or different, and each represents a hydrogen atom; a deuterium atom; a fluorine atom; a chlorine atom; a cyano group; a nitro group; an alkyl group having 1 to 6 carbon atoms; a cycloalkyl group having 5 to 10 carbon atoms; an alkenyl group having 2 to 6 carbon atoms; an alkyloxy group having 1 to 6 carbon atoms; a cycloalkyloxy group having 5 to 10 carbon atoms; an aromatic hydrocarbon group; an aromatic heterocyclic group; a condensed polycyclic aromatic group; an aryloxyl group; or a di-substituted amino group substituted by a group selected from an aromatic hydrocarbon group, an aromatic heterocyclic group, and a condensed polycyclic aromatic group. The alkyl group having 1 to 6 carbon atoms, the alkenyl group having 2 to 6 carbon atoms, and the alkyloxy group having 1 to 6 carbon atoms may be straight-chain or branched.

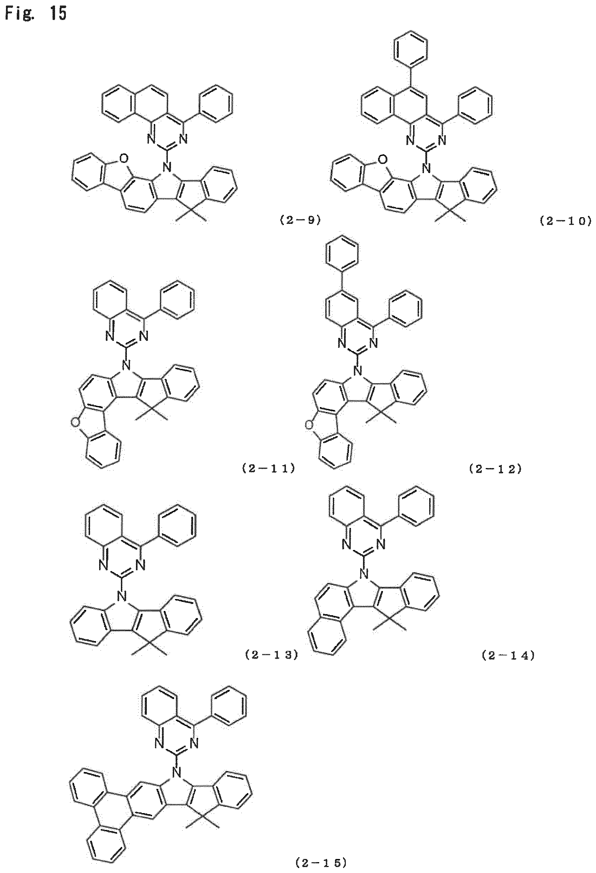

R¹ to R⁴ may be present independently of each other so as not to form a ring. However, as in Compounds 2-14 and 2-15 of Fig. 15, for example, they may bind to each other via a single bond, a substituted or unsubstituted methylene group, an oxygen atom, or a sulfur atom to form a ring. Similarly, R⁵ to R⁸ may be present independently of each other so as not to form a ring, but they may bind to each other via a single bond, a substituted or unsubstituted methylene group, an oxygen atom, or a sulfur atom to form a ring. If any of R¹ to R⁸ is the di-substituted amino group, the aromatic hydrocarbon group, aromatic heterocyclic group, or condensed polycyclic aromatic group in the di-substituted amino group contributes to ring formation.

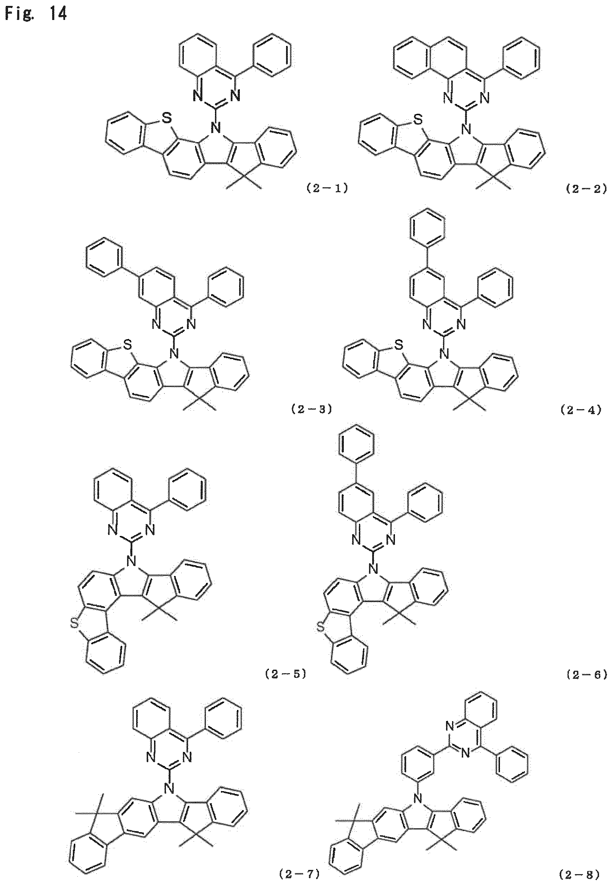

Like the compounds shown in Fig. 14, for example, to a vacant site occurring upon elimination of some of R¹ to R⁴ from the benzene ring, other group of R¹ to R⁴ may bind via a substituted or unsubstituted methylene group, an oxygen atom, a sulfur atom, or a monoarylamino group to form a ring. Similarly, to a vacant site occurring upon elimination of some of R⁵ to R⁸ from the benzene ring, other group of R⁵ to R⁸ may bind via a substituted or unsubstituted methylene group, an oxygen atom, a sulfur atom, or a monoarylamino group to form a ring. If any of R¹ to R⁸ is the di-substituted amino group, the aromatic hydrocarbon group, aromatic heterocyclic group, or condensed polycyclic aromatic group in the di-substituted amino group contributes to ring formation in cooperation with the benzene ring.

In connection with the monoarylamino group, which is one of the linking groups, the aryl group that the monoarylamino group has can be exemplified by the same ones as those shown in connection with the aromatic hydrocarbon group, the aromatic heterocyclic group, or the condensed polycyclic aromatic group represented by Ar¹ to Ar⁵ in the general formula (1). The aryl group in this case may be unsubstituted, but may have a substituent. Examples of the substituent are the same as those shown in connection with the substituent that the aromatic hydrocarbon group, the aromatic heterocyclic group, or the condensed polycyclic aromatic group represented by Ar¹ to Ar⁵ in the general formula (1) may have. The same holds true of the embodiments that the substituent can adopt.

The alkyl group having 1 to 6 carbon atoms, the cycloalkyl group having 5 to 10 carbon atoms, or the alkenyl group having 2 to 6 carbon atoms, represented by R¹ to R⁸, can be exemplified by a methyl group, an ethyl group, an n-propyl group, an isopropyl group, an n-butyl group, an isobutyl group, a tert-butyl group, an n-pentyl group, an isopentyl group, a neopentyl group, an n-hexyl group, etc.; a cyclopentyl group, a cyclohexyl group, a 1-adamantyl group, a 2-adamantyl group, etc.; and a vinyl group, an allyl group, an isopropenyl group, a 2-butenyl group, etc.

The alkyl group having 1 to 6 carbon atoms, the cycloalkyl group having 5 to 10 carbon atoms, or the alkenyl group having 2 to 6 carbon atoms, represented by R¹ to R⁸, may be unsubstituted, but may have a substituent. The substituent can be exemplified by the following groups, in addition to a deuterium atom, a cyano group, and a nitro group:
a halogen atom, for example, a fluorine atom, a chlorine atom, a bromine atom, or an iodine atom;
an alkyloxy group having 1 to 6 carbon atoms, for example, a methyloxy group, an ethyloxy group, or a propyloxy group;
an alkenyl group, for example, a vinyl group or an allyl group;
an aryloxy group, for example, a phenyloxy group or a tolyloxy group;
an arylalkyloxy group, for example, a benzyloxy group or a phenethyloxy group;
an aromatic hydrocarbon group or a condensed polycyclic aromatic group, for example, a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, or a triphenylenyl group;
an aromatic heterocyclic group, for example, a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, or a carbolinyl group;
a di-substituted amino group substituted by an aromatic hydrocarbon group or a condensed polycyclic aromatic group, for example, a diphenylamino group or a dinaphthylamino group;
a di-substituted amino group substituted by an aromatic heterocyclic group, for example, a dipyridylamino group or a dithienylamino group; and
a di-substituted amino group substituted by a substituent selected from an aromatic hydrocarbon group, a condensed polycyclic aromatic group, and an aromatic heterocyclic group.

The alkyloxy group having 1 to 6 carbon atoms and the alkenyl group may be straight-chain or branched. Any of these substituents may be unsubstituted, but may be further substituted by the above exemplary substituent. Moreover, these substituents may be present independently of each other so as not to form a ring. However, they may bind to each other via a single bond, a substituted or unsubstituted methylene group, an oxygen atom, or a sulfur atom to form a ring.

The alkyloxy group having 1 to 6 carbon atoms or the cycloalkyloxy group having 5 to 10 carbon atoms, represented by R¹ to R⁸, can be exemplified by a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

These groups, represented by R¹ to R⁸, may be unsubstituted, but may have a substituent. Examples of the substituent are the same as those shown in connection with the substituent that the alkyl group having 1 to 6 carbon atoms, the cycloalkyl group having 5 to 10 carbon atoms, or the alkenyl group having 2 to 6 carbon atoms, represented by R¹ to R⁸, may have. The same holds true of the embodiments that the substituent can adopt.

The aromatic hydrocarbon group, the aromatic heterocyclic group, or the condensed polycyclic aromatic group, represented by R¹ to R⁸, can be exemplified by the same as those shown in connection with the aromatic hydrocarbon group, the aromatic heterocyclic group, or the condensed polycyclic aromatic group represented by Ar¹ to Ar⁵ in the aforementioned general formula (1).

These groups, represented by R¹ to R⁸, may be unsubstituted, but may have a substituent. The substituent can be exemplified by the same ones as those shown in connection with the substituent that the aromatic hydrocarbon group, the aromatic heterocyclic group, or the condensed polycyclic aromatic group represented by Ar¹ to Ar⁵ in the general formula (1) may have. The same holds true of the embodiments that the substituent can adopt.

The aryloxy group represented by R¹ to R⁸ can be exemplified by a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

The aryloxy group represented by R¹ to R⁸ may be unsubstituted, but may have a substituent. The substituent can be exemplified by the same ones as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

The aromatic hydrocarbon group, aromatic heterocyclic group, or condensed polycyclic aromatic group that the di-substituted amino group, represented by R¹ to R⁸, has as its substituent can be exemplified by the same ones as shown in connection with the aromatic hydrocarbon group, aromatic heterocyclic group, or condensed polycyclic aromatic group represented by Ar¹ to Ar⁵ in the general formula (1) .

The aromatic hydrocarbon group, aromatic heterocyclic group, or condensed polycyclic aromatic group in the di-substituted amino group, represented by R¹ to R⁸, may be unsubstituted, but may have a substituent. The substituent can be exemplified by the same ones as those shown in connection with the substituent that the aromatic hydrocarbon group, aromatic heterocyclic group, or condensed polycyclic aromatic group, represented by Ar¹ to Ar⁵ in the general formula (1), may have. The same holds true of the embodiments that the substituent can adopt.

### (R⁹, R¹⁰)

R⁹ and R¹⁰ may be the same or different, and each represents an alkyl group having 1 to 6 carbon atoms, an aromatic hydrocarbon group, an aromatic heterocyclic group, or a condensed polycyclic aromatic group. The alkyl group having 1 to 6 carbon atoms may be straight-chain or branched.

These groups may be present independently of each other so as not to form a ring. However, they may bind to each other via a single bond, a substituted or unsubstituted methylene group, an oxygen atom, or a sulfur atom to form a ring.

The alkyl group having 1 to 6 carbon atoms represented by R⁹ and R¹⁰ can be exemplified by the same ones as those shown in connection with the alkyl group having 1 to 6 carbon atoms represented by R¹ to R⁸.

The alkyl group having 1 to 6 carbon atoms, represented by R⁹ and R¹⁰, may be unsubstituted, but may have a substituent. The substituent can be exemplified by the same ones as those shown in connection with the substituent that the alkyl group having 1 to 6 carbon atoms, the cycloalkyl group having 5 to 10 carbon atoms, or the alkenyl group having 2 to 6 carbon atoms, represented by R¹ to R⁸, may have. The same holds true of the embodiments that the substituent can adopt.

The aromatic hydrocarbon group, aromatic heterocyclic group, or condensed polycyclic aromatic group, represented by R⁹ and R¹⁰, can be exemplified by the same ones as those shown in connection with the aromatic hydrocarbon group, aromatic heterocyclic group, or condensed polycyclic aromatic group represented by Ar¹ to Ar⁵ in the general formula (1).

These groups represented by R⁹ and R¹⁰ may be unsubstituted, but may have a substituent. The substituent can be exemplified by the same ones as those shown in connection with the substituent that the aromatic hydrocarbon group, aromatic heterocyclic group, or condensed polycyclic aromatic group, represented by Ar¹ to Ar⁵ in the general formula (1), may have. The same holds true of the embodiments that the substituent can adopt.

### (Preferred embodiments)

Preferred embodiments of the indenoindole compound II will be described below. In the description of the preferred embodiments, the groups without the designation of "substituted"/"unsubstituted" may have a substituent or may be unsubstituted.

As A¹, a divalent group of an aromatic hydrocarbon, a divalent group of a condensed polycyclic aromatic, or a single bond is preferred; either a divalent group formed by removing two hydrogen atoms from benzene, biphenyl or naphthalene, or a single bond is more preferred; and either a divalent group formed by removing two hydrogen atoms from benzene, or a single bond is particularly preferred.

As Ar⁶, a phenyl group, a biphenylyl group, a naphthyl group, or an aromatic heterocyclic group is preferred. As the aromatic heterocyclic group, a nitrogen-containing aromatic heterocyclic group is preferred; a triazinyl group, a quinazolinyl group, a benzoquinazolinyl group, a benzimidazolyl group, a pyridopyrimidinyl group, a naphthyridinyl group, a pyridyl group, a quinolyl group, or an isoquinolyl group is more preferred; a quinazolinyl group or a benzoquinazolinyl group is particularly preferred; and a benzoquinazolinyl group is most preferred. The quinazolinyl group or the benzoquinazolinyl group preferably has a phenyl group as its substituent.

An embodiment is preferred in which as in Formulas (2a) to (2c), for example, one of R¹ to R⁴ is an aromatic hydrocarbon group, an aromatic heterocyclic group, or a condensed polycyclic aromatic group and, to a vacant site occurring upon elimination of some of R¹ to R⁴ from the benzene ring, such an aromatic hydrocarbon group, an aromatic heterocyclic group, or a condensed polycyclic aromatic group binds via a linking group, such as a substituted or unsubstituted methylene group, an oxygen atom, a sulfur atom, or a monoarylamino group, to form a ring. As the aromatic hydrocarbon group, aromatic heterocyclic group, or condensed polycyclic aromatic group in this case, a phenyl group, an indenyl group, an indolyl group, a benzofuranyl group, or a benzothienyl group is preferred. As the ring to be formed with the benzene ring in cooperation, a fluorene ring, a carbazole ring, a dibenzofuran ring, a dibenzothiophene ring, an indenoindole ring, an indenobenzofuran ring, an indenobenzothiophene ring, a benzofuroindole ring, a benzothienoindole ring, or an indoloindole ring is preferred; and a fluorene ring, a dibenzofuran ring, or a dibenzothiophene ring is more preferred.

An embodiment is also preferred in which as in Formulas (2d) to (2e) indicated below, for example, two adjacent groups among R¹ to R⁴ are each an alkenyl group having 2 to 6 carbon atoms, an aromatic hydrocarbon group, an aromatic heterocyclic group, or a condensed polycyclic aromatic group, and two such groups bind to each other via a single bond, thereby forming a condensed ring together with the benzene ring to which R¹ to R⁴ are bound. As the alkenyl group having 2 to 6 carbon atoms, aromatic hydrocarbon group, aromatic heterocyclic group, or condensed polycyclic aromatic group in this case, a vinyl group or a phenyl group is preferred. As the ring which these two groups and the benzene ring bound to R¹ to R⁴ together form, a naphthalene ring, a phenanthrene ring, or a triphenylene ring is preferred, and a naphthalene ring or a triphenylene ring is more preferred.

As the embodiment in which to a vacant site occurring upon elimination of some of R¹ to R⁴ from the benzene ring, other group of R¹ to R⁴ binds via a linking group to form a ring, the embodiment represented by the general formula (2a), (2b) or (2c) is preferred, and the embodiment represented by the general formula (2a) is particularly preferred. As the embodiment in which two adjacent groups among R¹ to R⁴ bind to each other to form a ring, the embodiment represented by the general formula (2d) or (2e) is preferred. 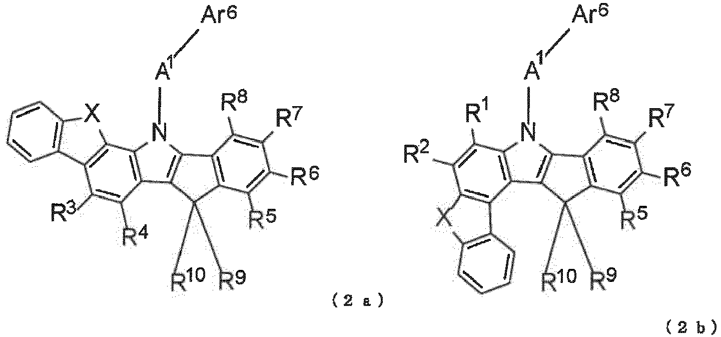 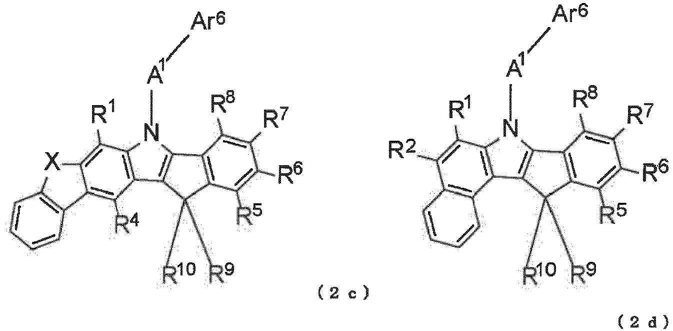 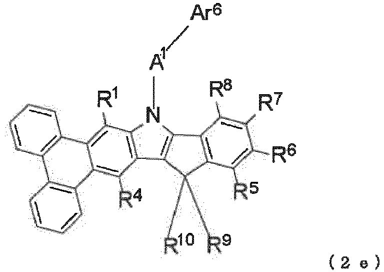 where
X represents a substituted or unsubstituted methylene group, an oxygen atom, a sulfur atom, or a monoarylamino group, and
A¹, Ar⁶, and R¹ to R¹⁰ have meanings as defined in the general formula (2).

The above general formula (2a) shows a structure in which to a position turned into a vacant site upon elimination of R¹, R² adjacent to R¹ binds via the linking group X to form a condensed ring.

The above general formula (2b) shows a structure in which to a position turned into a vacant site upon elimination of R³, R⁴ adjacent to R³ binds via the linking group X to form a condensed ring.

The above general formula (2c) shows a structure in which to a position turned into a vacant site upon elimination of R², R³ adjacent to R² binds via the linking group X to form a condensed ring.

The above general formula (2d) shows a structure in which R³ (vinyl group) and R⁴ (vinyl group) are bound to form a naphthalene ring in cooperation with the benzene ring to which R¹ to R⁴ are bound.

The above general formula (2e) shows a structure in which R² (phenyl group) and R³ (phenyl group) are bound to form a triphenylene ring in cooperation with the benzene ring to which R¹ to R⁴ are bound.

In connection with R⁵ to R⁸, preferred is an embodiment in which the adjacent two groups or all the groups are vinyl groups, and the adjacent two vinyl groups bind to each other via a single bond to form a condensed ring, namely, an embodiment in which they form a naphthalene ring or a phenanthrene ring together with the benzene ring to which R⁵ to R⁸ are bound. Alternatively, it is preferred that R⁵ to R⁸ be each a hydrogen atom.

R⁹ and R¹⁰ may be the same or different, and are each preferably an alkyl group having 1 to 6 carbon atoms, and more preferably a methyl group.

Preferred examples of the indenoindole compound II are shown in Figs. 14 and 15, but the indenoindole compound II is not limited to these compounds. In the concrete examples, Compounds 2-1 to 2-4, 2-9 and 2-10 correspond to the aforementioned general formula (2a), Compounds 2-5, 2-6, 2-11 and 2-12 correspond to the aforementioned general formula (2b), and Compounds 2-7 and 2-8 correspond to the aforementioned general formula (2c). Moreover, Compound 2-14 corresponds to the aforementioned general formula (2d), and Compound 2-15 corresponds to the aforementioned general formula (2e).

The indenoindole compound II can be synthesized in accordance with a publicly known method (see Patent Document 6). As shown in the Examples to be described later, for example, it can be synthesized by cross coupling such as Suzuki coupling.

### <Carbazole compound III>

The carbazole compound III contained in the luminous layer has a structure represented by the following general formula (3):

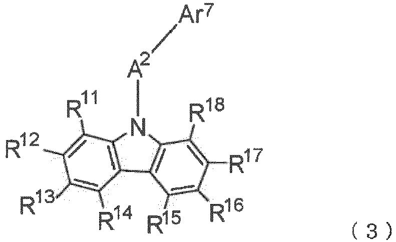

### (A²)

A² represents a divalent group of an aromatic hydrocarbon, a divalent group of an aromatic heterocycle, a divalent group of a condensed polycyclic aromatic, or a single bond.

Examples of the divalent group of an aromatic hydrocarbon, the divalent group of an aromatic heterocycle, or the divalent group of a condensed polycyclic aromatic, represented by A², are the same as those shown in connection with the divalent group of an aromatic hydrocarbon, the divalent group of an aromatic heterocycle, or the divalent group of a condensed polycyclic aromatic represented by A¹ in the aforementioned general formula (2) .

These divalent groups represented by A² may be unsubstituted, but may have a substituent. Examples of the substituent are the same as those shown in connection with the substituent that the divalent group of an aromatic hydrocarbon, the divalent group of an aromatic heterocycle, or the divalent group of a condensed polycyclic aromatic, represented by A¹ in the general formula (2), may have. The same holds true of the embodiments that the substituent can adopt.

### (Ar⁷)

Ar⁷ represents an aromatic hydrocarbon group, an aromatic heterocyclic group, or a condensed polycyclic aromatic group.

The aromatic hydrocarbon group, the aromatic heterocyclic group, or the condensed polycyclic aromatic group, represented by Ar⁷, can be exemplified by the same as those shown in connection with the aromatic hydrocarbon group, the aromatic heterocyclic group, or the condensed polycyclic aromatic group represented by Ar⁶ in the general formula (2).

These groups represented by Ar⁷ may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar⁶ in the general formula (2), may have. The same holds true of the embodiments that the substituent can adopt.

### (R¹¹ to R¹⁸)

R¹¹ to R¹⁸ may be the same or different, and each represents a hydrogen atom; a deuterium atom; a fluorine atom; a chlorine atom; a cyano group; a nitro group; an alkyl group having 1 to 6 carbon atoms; a cycloalkyl group having 5 to 10 carbon atoms; an alkenyl group having 2 to 6 carbon atoms; an alkyloxy group having 1 to 6 carbon atoms; a cycloalkyloxy group having 5 to 10 carbon atoms; an aromatic hydrocarbon group; an aromatic heterocyclic group; a condensed polycyclic aromatic group; an aryloxyl group; or a di-substituted amino group substituted by a group selected from an aromatic hydrocarbon group, an aromatic heterocyclic group, and a condensed polycyclic aromatic group. The alkyl group having 1 to 6 carbon atoms, the alkenyl group having 2 to 6 carbon atoms, and the alkyloxy group having 1 to 6 carbon atoms may be straight-chain or branched.

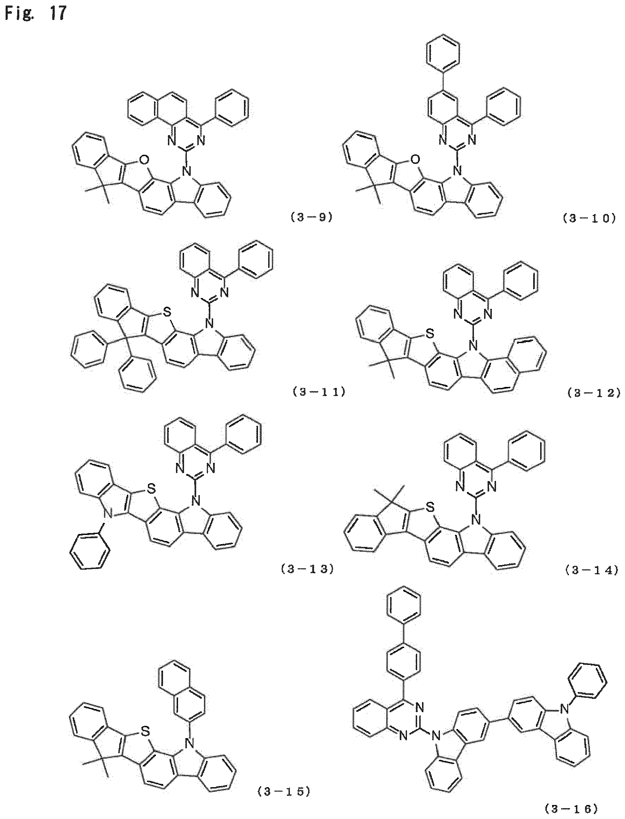

R¹¹ to R¹⁴ may be present independently of each other so as not to form a ring. However, they may bind to each other via a single bond, a substituted or unsubstituted methylene group, an oxygen atom, or a sulfur atom to form a ring. Similarly, R¹⁵ to R¹⁸ may be present independently of each other so as not to form a ring, but they may bind to each other via a single bond, a substituted or unsubstituted methylene group, an oxygen atom, or a sulfur atom to form a ring. In Compound 3-12 of Fig. 17, for example, a plurality of substituents (two vinyl groups) are bound via a single bond to form a naphthalene ring.

To a vacant site occurring upon elimination of some of R¹¹ to R¹⁴ from the benzene ring, other group of R¹¹ to R¹⁴ may bind via a substituted or unsubstituted methylene group, an oxygen atom, a sulfur atom, or a monoarylamino group to form a ring. Similarly, to a vacant site occurring upon elimination of some of R¹⁵ to R¹⁸ from the benzene ring, other group of R¹⁵ to R¹⁸ may bind via a substituted or unsubstituted methylene group, an oxygen atom, a sulfur atom, or a monoarylamino group to form a ring. In Compound 3-12 of Fig. 17, for example, to a vacant site occurring upon elimination of R¹¹ from the benzene ring, R¹² binds via a sulfur atom to form a ring.

As the aryl group in the monoarylamino group, which is one of the linking groups, the same ones as those shown in connection with the aryl group in the monoarylamino group, the linking group in the generation formula (2), can be named. The aryl group in the monoarylamino group may be unsubstituted, but may have a substituent. Examples of the substituent are the same as those shown in connection with the substituent that the aryl group in the monoarylamino group, the linking group in the general formula (2), may have. The same holds true of the embodiments that the substituent can adopt.

The alkyl group having 1 to 6 carbon atoms; the cycloalkyl group having 5 to 10 carbon atoms; the alkenyl group having 2 to 6 carbon atoms; the alkyloxy group having 1 to 6 carbon atoms; the cycloalkyloxy group having 5 to 10 carbon atoms; the aromatic hydrocarbon group; the aromatic heterocyclic group; the condensed polycyclic aromatic group; the aryloxyl group; or the di-substituted amino group substituted by a group selected from an aromatic hydrocarbon group, an aromatic heterocyclic group, and a condensed polycyclic aromatic group, represented by R¹¹ to R¹⁸, can be exemplified by the same ones as those shown in connection with the alkyl group having 1 to 6 carbon atoms; the cycloalkyl group having 5 to 10 carbon atoms; the alkenyl group having 2 to 6 carbon atoms; the alkyloxy group having 1 to 6 carbon atoms; the cycloalkyloxy group having 5 to 10 carbon atoms; the aromatic hydrocarbon group; the aromatic heterocyclic group; the condensed polycyclic aromatic group; the aryloxyl group; or the di-substituted amino group substituted by a group selected from an aromatic hydrocarbon group, an aromatic heterocyclic group, and a condensed polycyclic aromatic group, represented by R¹ to R⁸ in the general formula (2).

These substituents represented by R¹¹ to R¹⁸ may be unsubstituted, but may have a substituent. Examples of the substituent are the same as those shown in connection with the substituent that the aromatic hydrocarbon group, the aromatic heterocyclic group, or the condensed polycyclic aromatic group represented by Ar¹ to Ar⁵ in the general formula (1), may have. The same holds true of the embodiments that the substituent can adopt.

### (Preferred embodiments)

Preferred embodiments of the carbazole compound III will be described below. In the description of the preferred embodiments, the groups without the designation of "substituted"/"unsubstituted" may have a substituent or may be unsubstituted.

As A², a divalent group of an aromatic hydrocarbon, a divalent group of a condensed polycyclic aromatic, or a single bond is preferred; either a divalent group formed by removing two hydrogen atoms from benzene, biphenyl or naphthalene, or a single bond is more preferred; and either a divalent group formed by removing two hydrogen atoms from benzene, or a single bond is particularly preferred.

As Ar⁷, a phenyl group, a biphenylyl group, a naphthyl group, or an aromatic heterocyclic group is preferred; a naphthyl group, or an aromatic heterocyclic group is more preferred; and an aromatic heterocyclic group is particularly preferred. As the aromatic heterocyclic group, a triazinyl group, a quinazolinyl group, a benzoquinazolinyl group, a benzimidazolyl group, a pyridopyrimidinyl group, a naphthyridinyl group, a pyridyl group, a quinolyl group, or an isoquinolyl group is preferred; a quinazolinyl group or a benzoquinazolinyl group is more preferred; and a quinazolinyl group is particularly preferred. The quinazolinyl group or the benzoquinazolinyl group preferably has an aromatic hydrocarbon group as its substituent.

In connection with R¹¹ to R¹⁸, an embodiment is preferred in which two adjacent groups among R¹¹ to R¹⁴ are each an alkenyl group having 2 to 6 carbon atoms, an aromatic hydrocarbon group, an aromatic heterocyclic group, or a condensed polycyclic aromatic group, and these two groups are bound together via a single bond to form a condensed ring together with the benzene ring to which R¹¹ to R¹⁴ are bound. As the alkenyl group having 2 to 6 carbon atoms, the aromatic hydrocarbon group, the aromatic heterocyclic group, or the condensed polycyclic aromatic group in this case, a vinyl group or a phenyl group is preferred. As the ring formed from such two groups and the benzene ring bound to R¹¹ to R¹⁴, a naphthalene ring, a phenanthrene ring, or a triphenylene ring is preferred.

An embodiment is also preferred in which, as in general formulas (3a-1) to (3a-4) and (3b-1) indicated below, for example, one of R¹¹ to R¹⁴ is an aromatic hydrocarbon group, an aromatic heterocyclic group, or a condensed polycyclic aromatic group and, to a vacant site occurring upon elimination of some of R¹¹ to R¹⁴ from the benzene ring, such an aromatic hydrocarbon group, an aromatic heterocyclic group, or a condensed polycyclic aromatic group binds via a linking group, such as a substituted or unsubstituted methylene group, an oxygen atom, a sulfur atom, or a monoarylamino group, to form a ring. As the aromatic hydrocarbon group, the aromatic heterocyclic group, or the condensed polycyclic aromatic group in this case, a phenyl group, an indenyl group, an indolyl group, a benzofuranyl group, or a benzothienyl group is preferred. As the ring to be formed together with the benzene ring, a fluorene ring, a carbazole ring, a dibenzofuran ring, a dibenzothiophene ring, an indenoindole ring, an indenobenzofuran ring, an indenobenzothiophene ring, a benzofuroindole ring, a benzothienoindole ring, or an indoloindole ring is preferred.

In this case, R¹⁵ to R¹⁸ and the remainder of R¹¹ to R¹⁴ not contributing to ring formation may be the same or different, and are preferably hydrogen atoms or deuterium atoms.

As the embodiment in which to a vacant site occurring upon elimination of some of R¹¹ to R¹⁴ from the benzene ring, the remaining group of R¹¹ to R¹⁴ binds via a linking group to form a ring, the embodiment represented by any of the following general formulas (3a-1) to (3a-4) and (3b-1) is preferred. 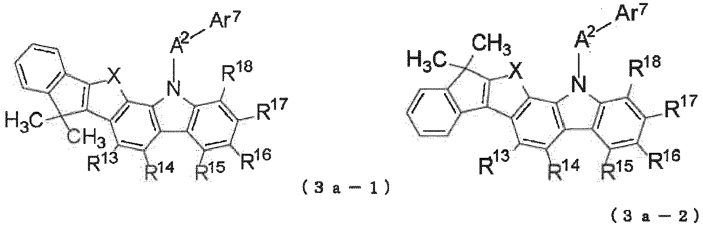 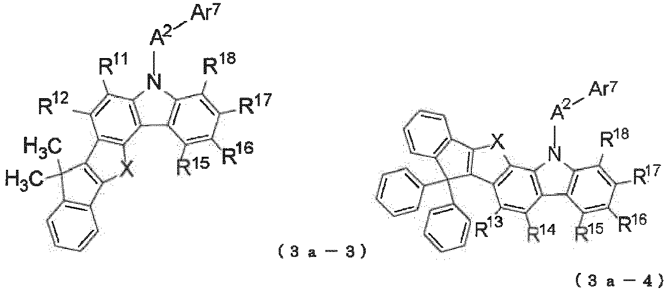 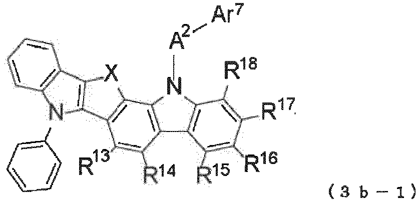 where
X represents a substituted or unsubstituted methylene group, an oxygen atom, a sulfur atom, or a monoarylamino group, and
A², Ar⁷, and R¹¹ to R¹⁸ have meanings as defined in the general formula (3).

The above general formula (3a-1) shows a structure in which to a position becoming a vacant site upon elimination of R¹¹, R¹² (an indenyl group having two methyl groups as substituents) adjacent to R¹¹ binds via the linking group X to form a condensed ring.

The above general formula (3a-2), like the general formula (3a-1), also shows a structure in which to a position becoming a vacant site upon elimination of R¹¹, R¹² (an indenyl group having two methyl groups as substituents) adjacent to R¹¹ binds via the linking group X to form a condensed ring.

The above general formula (3a-3) shows a structure in which to a position becoming a vacant site upon elimination of R¹⁴, R¹³ (an indenyl group having two methyl groups as substituents) adjacent to R¹⁴ binds via the linking group X to form a condensed ring.

The above general formula (3a-4) shows a structure in which to a position becoming a vacant site upon elimination of R¹¹, R¹² (an indenyl group having two phenyl groups as substituents) adjacent to R¹¹ binds via the linking group X to form a condensed ring.

The above general formula (3b-1) shows a structure in which to a position becoming a vacant site upon elimination of R¹¹, R¹² (an N-phenyl-substituted indolyl group) adjacent to R¹¹ binds via the linking group X to form a condensed ring.

In connection with R¹⁵ to R¹⁸, preferred is an embodiment in which the adjacent two groups or all the groups are vinyl groups, and the adjacent two vinyl groups bind to each other via a single bond to form a condensed ring, namely, an embodiment in which they form a naphthalene ring or a phenanthrene ring together with the benzene ring to which R¹⁵ to R¹⁸ are bound.

Also preferred is an embodiment in which one of R¹⁵ to R¹⁸ is an aromatic hydrocarbon group, an aromatic heterocyclic group, or a condensed polycyclic aromatic group, or in particular, is an aromatic hydrocarbon group. It is preferred that one of R¹⁵ to R¹⁸ be a fluorenyl group, a carbazolyl group, a dibenzofuranyl group, or a dibenzothienyl group. It is more preferred that R¹⁶ be a fluorenyl group, a carbazolyl group, a dibenzofuranyl group, or a dibenzothienyl group, and R¹⁵, R¹⁷ and R¹⁸ be hydrogen atoms. It is particularly preferred that R¹⁶ be a carbazolyl group, and R¹⁵, R¹⁷ and R¹⁸ be hydrogen atoms.

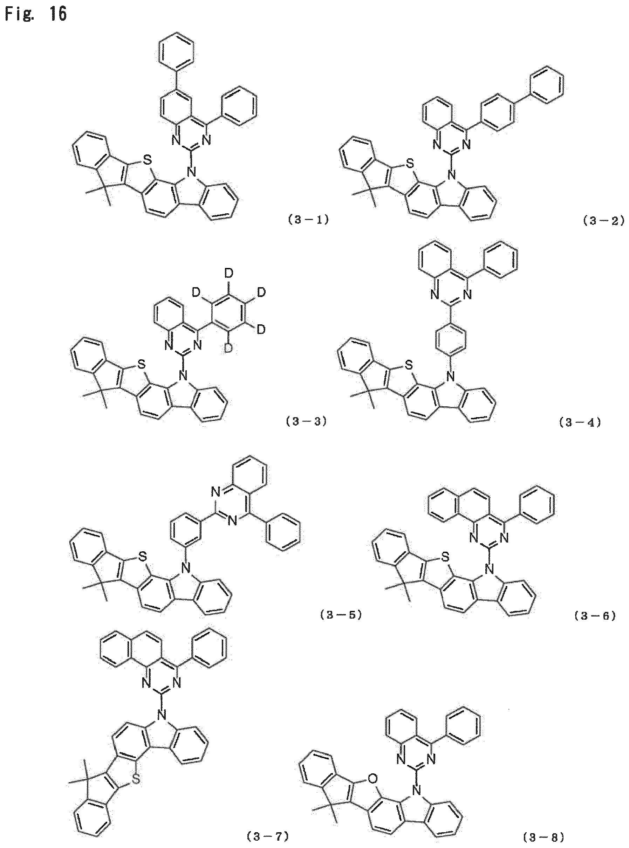

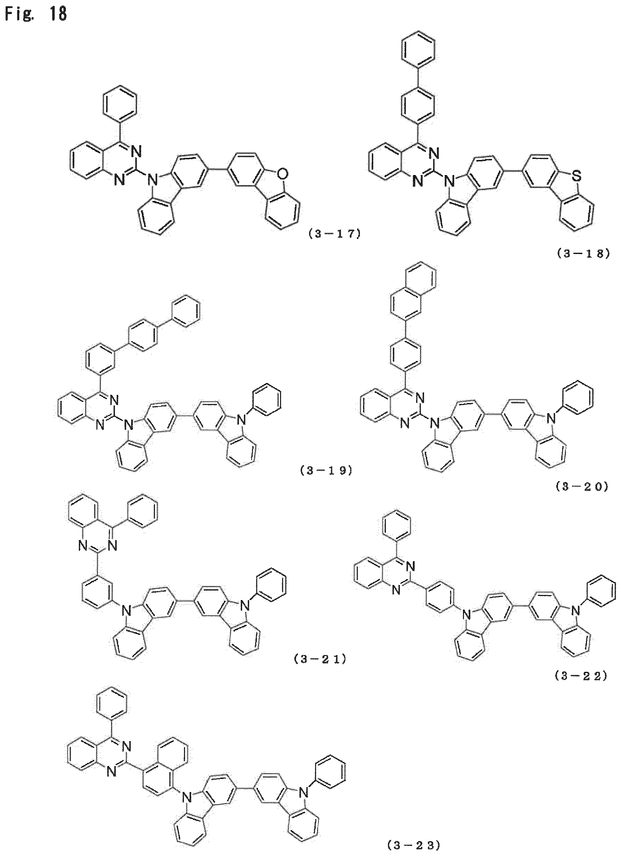

Preferred examples of the carbazole compound III are shown in Figs. 16 to 18, but the carbazole compound III is not limited to these compounds. In the concrete examples, Compounds 3-1 to 3-6, 3-8 to 3-10, 3-12, and 3-15 correspond to the aforementioned general formula (3a-1), Compound 3-14 corresponds to the aforementioned general formula (3a-2), Compound 3-7 corresponds to the aforementioned general formula (3a-3), Compound 3-11 corresponds to the aforementioned general formula (3a-4), and Compound 3-13 corresponds to the aforementioned general formula (3b-1).

The carbazole compound III can be synthesized in accordance with a publicly known method (see Patent Document 6) .

In the organic EL device of the present invention, the respective layers can take various forms, as long as they fulfill the conditions that the arylamine compound I is contained in the hole transport layer and that the indenoindole compound II or the carbazole compound III is contained in the luminous layer. The respective layers will be described in detail below by reference to Fig. 1.

### <Anode 2>

In the organic EL device of the present invention, the anode 2 is provided on the substrate 1. An electrode material having a high work function, such as ITO or gold, is used as the anode 2.

### <Hole injection layer 3>

The hole injection layer 3 can be provided, if necessary, between the anode 2 and the hole transport layer 4. For the hole injection layer 3, a publicly known material may be used.

Usable as the publicly known materials are, for example, materials such as triphenylamine derivatives of starburst type and various triphenylamine tetramers; porphyrin compounds typified by copper phthalocyanine; acceptor type heterocyclic compounds such as hexacyanoazatriphenylene; and coating type polymeric materials.

For the hole injection layer 3, the arylamine compound I is used preferably. A triarylamine compound V represented by a general formula (5) to be described later, or a triarylamine compound VI represented by a general formula (6) to be described later is also used preferably. When any of these (tri) arylamine compounds is used in the hole injection layer 3, the composition of the hole injection layer 3 and the composition of the hole transport layer 4 to be described later are different.

Moreover, the materials for general use in the hole injection layer can be further p-doped with tris(bromophenyl)aminium hexachloroantimonate or radialene derivatives (see WO2014/009310), and used in the hole injection layer. Polymeric compounds containing the structures of benzidine derivatives such as TPD in their partial structures can also be used in the hole injection layer.

When thin film formation is performed by a publicly known method such as vapor deposition, a spin coat method or an ink jet method with the use of any of the above materials, the hole injection layer 3 can be obtained. Each of the layers to be described below can similarly be obtained by thin film formation performed using a publicly known method such as vapor deposition, spin coating, or ink jetting.

### <Hole transport layer 4>

The hole transport layer 4 is provided above the anode 2 (or hole injection layer 3). In the present invention, the arylamine compound I represented by the general formula (1) is used for the hole transport layer 4.

Hole transporting materials, which can be mixed with or used concurrently with the arylamine compound I, can be exemplified below:
benzidine derivatives, for example,
   N,N'-diphenyl-N,N'-di(m-tolyl)benzidine (TPD),
   N,N'-diphenyl-N,N'-di(α-naphthyl)benzidine (NPD), and
   N,N,N' ,N' -tetrabiphenylylbenzidine;
triarylamine compounds having 2 to 6 triarylamine structures, the triarylamine structures being connected together by a single bond or a heteroatom-free divalent group, e.g.,
   1,1-bis[4-(di-4-tolylamino)phenyl]cyclohexane (TAPC),
   triarylamine compounds V represented by the general formula (5) to be described later, and
   triarylamine compounds VI represented by the general formula (6) to be described later; and
various triphenylamine trimers.

Of the above-mentioned hole transporting materials, the triarylamine compounds having 2 to 6 triarylamine structures, the triarylamine structures being connected together by a single bond or a heteroatom-free divalent group (may hereinafter be referred to simply as triarylamine compounds having 2 to 6 triarylamine structures) are preferred. More preferred are the triarylamine compounds having 2 triarylamine structures, the triarylamine structures being connected together by a single bond or a heteroatom-free divalent group (may hereinafter be referred to simply as triarylamine compounds having 2 triarylamine structures), or the triarylamine compounds having 4 triarylamine structures, the triarylamine structures being connected together by a single bond or a heteroatom-free divalent group (may hereinafter be referred to simply as triarylamine compounds having 4 triarylamine structures).

Besides, the materials for general use in the hole transport layer, which have been further p-doped with tris(bromophenyl)aminium hexachloroantimonate or radialene derivatives (see WO2014/009310), and polymeric compounds containing the structures of benzidine derivatives such as TPD in their partial structures can be used in the hole transport layer.

These materials may be subjected singly to film formation, but any of them may be mixed with other of them and subjected to film formation. The respective organic layers to be described later can also be formed as films in the same manner.

The hole transport layer 4 may have a structure in which layers formed from these materials used alone are stacked; a structure in which layers formed from their mixtures are stacked; or a structure in which a layer of any of them used alone and a layer of a mixture of them are stacked. The respective organic layers to be described below can have similar structures.

In the present invention, the hole transport layer 4 preferably has a two-layer structure composed of the first hole transport layer 4a located on the side of the anode 2 and the second hole transport layer 4b located on the side of the luminous layer 5 as shown in Fig. 1.

### (First hole transport layer 4a)

The first hole transport layer 4a is provided between the above-mentioned anode 2 (or hole injection layer 3) and the second hole transport layer 4b. In the present invention, the aforementioned hole transporting material can be contained in the first hole transport layer 4a.

Of the above-mentioned hole transporting materials, the triarylamine compounds having 2 to 6 triarylamine structures are used preferably. The triarylamine compounds having 2 triarylamine structures, and the triarylamine compounds having 4 triarylamine structures are used more preferably.

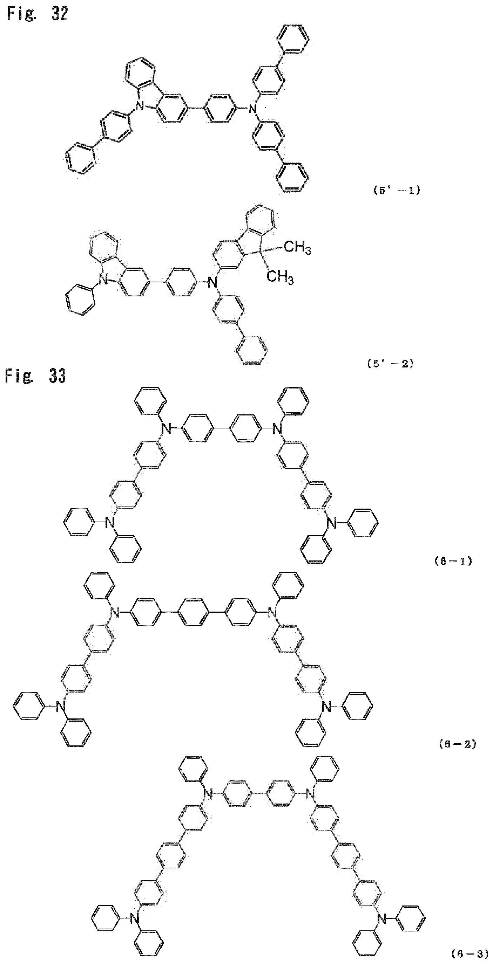

The triarylamine compounds having 2 triarylamine structures include an embodiment in which two benzene rings in the triarylamine structure are bound via a single bond, namely, an embodiment having a carbazole ring structure, for example, as in 5' -1 and 5'-2 of Fig. 32.

As the triarylamine compound having 2 triarylamine structures, the triarylamine compound V represented by the general formula (5) to be described later is preferred, because it is excellent in thin film stability and heat resistance in addition to hole transport properties, and its synthesis is easy.

As the triarylamine compound having 4 triarylamine structures, the triarylamine compound VI represented by the general formula (6) to be described later is preferred, because it is excellent in thin film stability and heat resistance as well as hole transport properties, and it is easy to synthesize.

By combining the triarylamine compound V or triarylamine compound VI having such a specific structure, as the material for the first hole transport layer 4a, with the second hole transport layer 4b containing the arylamine compound I to be described later, holes can be injected and transported into the luminous layer 5 with high efficiency, and the carrier balance is rendered more elaborate. As a result, the luminous efficiency is increased, the driving voltage is further lowered, and the durability becomes higher. An organic EL device with excellent durability, in particular, can be realized.

Triarylamine compound V represented by the following general formula (5):

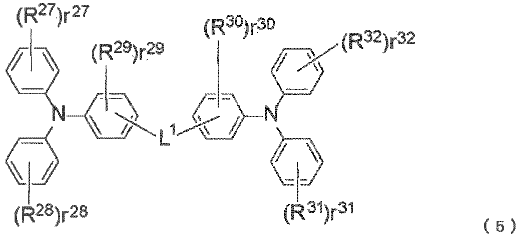

### R²⁷ to R³²:

R²⁷ to R³² may be the same or different, and each represents a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group. The alkyl group having 1 to 6 carbon atoms, the alkenyl group having 2 to 6 carbon atoms, and the alkyloxy group having 1 to 6 carbon atoms may be straight-chain or branched,

The alkyl group having 1 to 6 carbon atoms, the cycloalkyl group having 5 to 10 carbon atoms, or the alkenyl group having 2 to 6 carbon atoms, represented by R²⁷ to R³², can be exemplified by a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, or a 2-butenyl group.

The alkyl group having 1 to 6 carbon atoms, the cycloalkyl group having 5 to 10 carbon atoms, or the alkenyl group having 2 to 6 carbon atoms, represented by R²⁷ to R³², may be unsubstituted, but may have a substituent. The substituent can be exemplified by the following groups, in addition to a deuterium atom, a cyano group, and a nitro group:
a halogen atom, for example, a fluorine atom, a chlorine atom, a bromine atom, or an iodine atom;
an alkyloxy group having 1 to 6 carbon atoms, for example, a methyloxy group, an ethyloxy group, or a propyloxy group;
an alkenyl group, for example, a vinyl group or an allyl group;
an aryloxy group, for example, a phenyloxy group or a tolyloxy group;
an arylalkyloxy group, for example, a benzyloxy group or a phenethyloxy group;
an aromatic hydrocarbon group or a condensed polycyclic aromatic group, for example, a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, or a triphenylenyl group; and
an aromatic heterocyclic group, for example, a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, or a carbolinyl group.

The alkyloxy group having 1 to 6 carbon atoms and the alkenyl group may be straight-chain or branched. Any of these substituents may be unsubstituted, but may be further substituted by the above exemplary substituent. Moreover, these substituents may be present independently of each other so as not to form a ring. However, they may bind to each other via a single bond, a substituted or unsubstituted methylene group, an oxygen atom, or a sulfur atom to form a ring.

The alkyloxy group having 1 to 6 carbon atoms or the cycloalkyloxy group having 5 to 10 carbon atoms, represented by R²⁷ to R³², can be exemplified by a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

These groups represented by R²⁷ to R³² may be unsubstituted, but may have a substituent. Examples of the substituent are the same as those shown in connection with the alkyl group having 1 to 6 carbon atoms, the cycloalkyl group having 5 to 10 carbon atoms, or the alkenyl group having 2 to 6 carbon atoms, represented by R²⁷ to R³². The same holds true of the embodiments that the substituent can adopt.

Examples of the aromatic hydrocarbon group, the aromatic heterocyclic group, the condensed polycyclic aromatic group, or the aryloxy group, represented by R²⁷ to R³², can be the same as those shown in connection with the aromatic hydrocarbon group, the aromatic heterocyclic group, the condensed polycyclic aromatic group, or the aryloxy group, represented by R¹ to R⁸ in the aforementioned general formula (2).

These groups represented by R²⁷ to R³² may be unsubstituted, but may have a substituent. The substituent can be exemplified by the same ones as those shown in connection with the substituent that the aromatic hydrocarbon group, the aromatic heterocyclic group, or the condensed polycyclic aromatic group represented by Ar¹ to Ar⁵ in the general formula (1) may have. The same holds true of the embodiments that the substituent can adopt.

### r²⁷ to r³²:

r²⁷ to r³², respectively, show the numbers of the groups R²⁷s to R³²s bound to the aromatic ring. r²⁷, r²⁸, r³¹ and r³² each denote an integer of 0 to 5. r²⁹ and r³⁰ each denote an integer of 0 to 4. A case where r²⁷ to r³² are each 0 means that there are none of R²⁷ to R³² on the aromatic rings, namely, that none of the benzene rings are substituted by the groups represented by R²⁷ to R³².

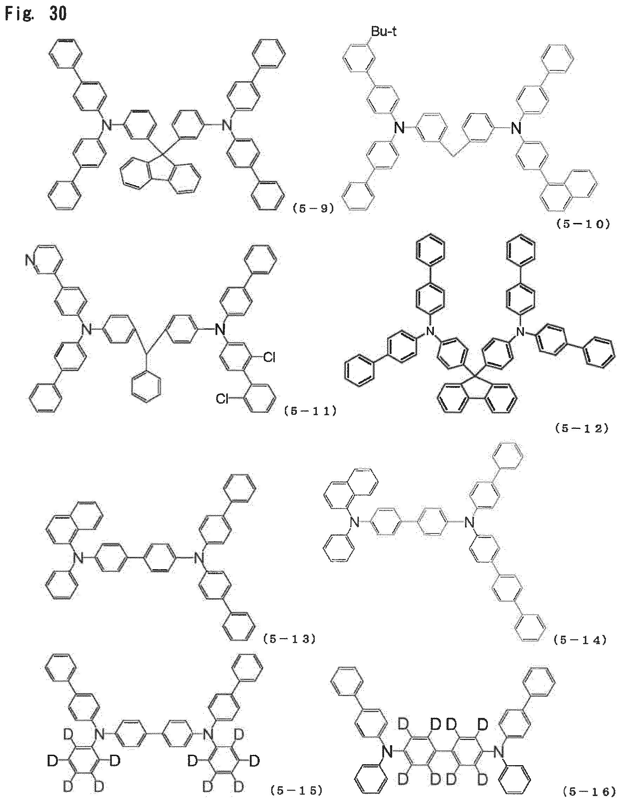

If r²⁷, r²⁸, r³¹ and r³² are each an integer of 2 to 5, or if r²⁹ and r³⁰ are each an integer of 2 to 4, a plurality of R²⁷s to R³²s are bound to the same benzene rings. In this case, the plurality of the groups bound may be the same or different. Moreover, they may be present independently of each other so as not to form a ring, but may bind to each other via a single bond, a substituted or unsubstituted methylene group, an oxygen atom, or a sulfur atom to form a ring. In Compounds 5-13 and 5-14 of Fig. 30, for example, a plurality of substituents (2 vinyl groups) are bound together via a single bond to form a naphthalene ring.

### L¹:

L¹ is a bridge group connecting the two triarylamine structures. L¹ represents a single bond, or a divalent group represented by any of the following structural formulas (B) to (G) :

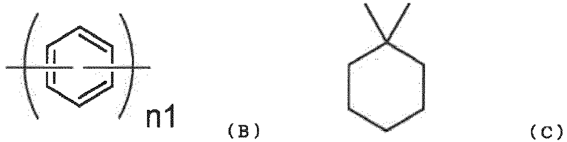

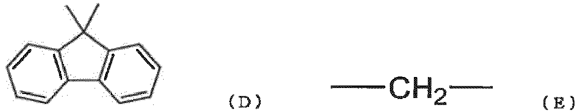

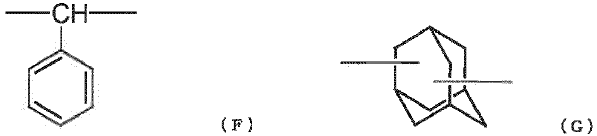

In the structural formula (B), n1 denotes an integer of 1 to 4.

### Preferred embodiments:

Preferred embodiments of the triarylamine compound V will be described below. In the description of the preferred embodiments, the groups without the designation of "substituted" or "unsubstituted" may have a substituent or may be unsubstituted.

R²⁷ to R³² may be the same or different, and a deuterium atom, a chlorine atom, an alkyl group having 1 to 6 carbon atoms, an alkenyl group having 2 to 6 carbon atoms, an aromatic hydrocarbon group, an aromatic heterocyclic group, or a condensed polycyclic aromatic group is preferred. A deuterium atom, a chlorine atom, a phenyl group, a biphenylyl group, a naphthyl group, a pyridyl group, or a vinyl group is more preferred. A deuterium atom, a phenyl group, a biphenylyl group, a naphthyl group, or a vinyl group is particularly preferred. An embodiment in which these groups bind to each other via a single bond to form a condensed aromatic ring, for example, as in Compounds 5-13 and 5-14 of Fig. 30, is also preferred. A deuterium atom, a phenyl group, or a biphenylyl group is most preferred.

R²⁷ to R³² may be the same or different. For these groups, an integer of 0 to 3 is preferred, and an integer of 0 to 2 is more preferred.

As L¹, a divalent group represented by any of the aforementioned structural formulas (C) to (F), or a single bond is preferred, a divalent group represented by the structural formula (B), or a single bond is more preferred, and a single bond is particularly preferred.

As n1 in the structural formula (B), an integer of 1 to 3 is preferred, and 2 or 3 is more preferred.

It is also preferred for the triarylamine compound V to have symmetry.

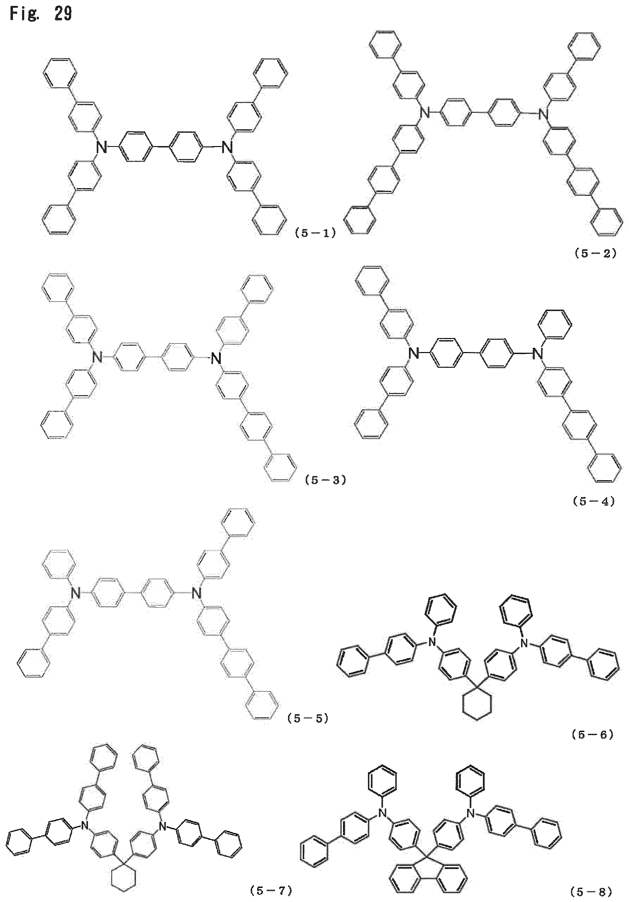

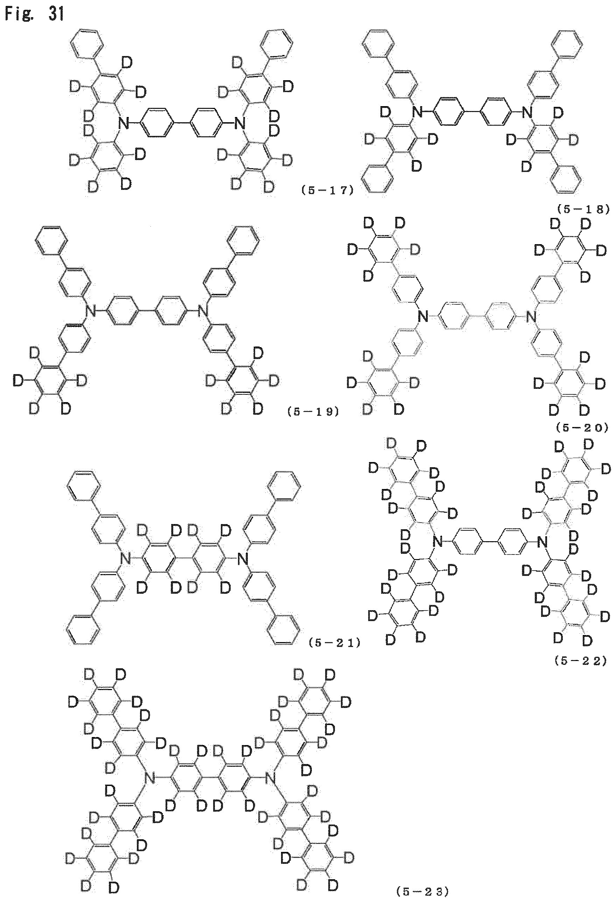

Preferred examples of the triarylamine compound V are shown in Figs. 29 to 31, but the triarylamine compounds V are not limited to these compounds. D represents a deuterium atom.

Of the above-described triarylamine compounds having 2 triarylamine structures, which are preferably used in the present invention, preferred examples of the compounds other than the triarylamine compounds V are shown in Fig. 32. However, the triarylamine compounds having 2 triarylamine structures are not limited to these compounds.

The triarylamine compound V can be synthesized in accordance with a method publicly known per se (see Patent Documents 1, 10 and 11).

Triarylamine compound VI represented by the following general formula (6):

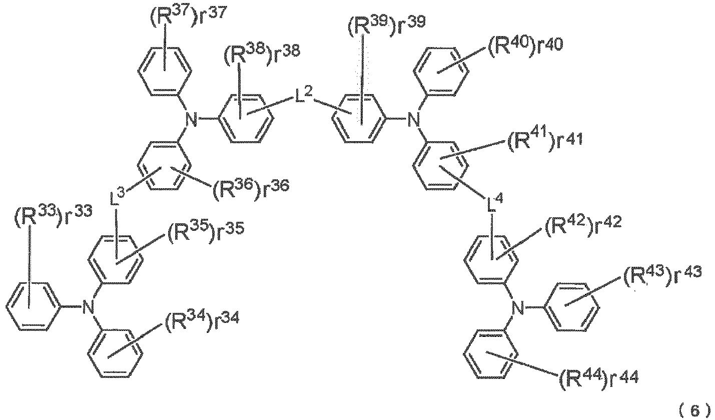

### R³³ to R⁴⁴:

R³³ to R⁴⁴ may be the same or different, and each represents a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group. The alkyl group having 1 to 6 carbon atoms, the alkenyl group having 2 to 6 carbon atoms, and the alkyloxy group having 1 to 6 carbon atoms may be straight-chain or branched.

The alkyl group having 1 to 6 carbon atoms, the cycloalkyl group having 5 to 10 carbon atoms, or the alkenyl group having 2 to 6 carbon atoms, represented by R³³ to R⁴⁴, can be exemplified by the same ones as those shown in connection with the alkyl group having 1 to 6 carbon atoms, the cycloalkyl group having 5 to 10 carbon atoms, or the alkenyl group having 2 to 6 carbon atoms, represented by R²⁷ to R³² in the general formula (5).

These groups, represented by R³³ to R⁴⁴, may be unsubstituted, but may have a substituent. Examples of the substituent are the same ones as those shown in connection with the substituent that the alkyl group having 1 to 6 carbon atoms, the cycloalkyl group having 5 to 10 carbon atoms, or the alkenyl group having 2 to 6 carbon atoms, represented by R²⁷ to R³² in the general formula (5), may have. The same holds true of the embodiments that the substituents can adopt.

The alkyloxy group having 1 to 6 carbon atoms or the cycloalkyloxy group having 5 to 10 carbon atoms, represented by R³³ to R⁴⁴, can be exemplified by the same ones as those shown in connection with the alkyloxy group having 1 to 6 carbon atoms or the cycloalkyloxy group having 5 to 10 carbon atoms, represented by R²⁷ to R³² in the general formula (5).

These groups, represented by R³³ to R⁴⁴, may be unsubstituted, but may have a substituent. Examples of the substituent are the same ones as those shown in connection with the substituent that the alkyl group having 1 to 6 carbon atoms, the cycloalkyl group having 5 to 10 carbon atoms, or the alkenyl group having 2 to 6 carbon atoms, represented by R²⁷ to R³² in the general formula (5), may have. The same holds true of the embodiments that the substituents can adopt.

Examples of the aromatic hydrocarbon group, the aromatic heterocyclic group, the condensed polycyclic aromatic group, or the aryloxy group, represented by R³³ to R⁴⁴, are the same ones as those shown in connection with the aromatic hydrocarbon group, the aromatic heterocyclic group, the condensed polycyclic aromatic group, or the aryloxy group, represented by R¹ to R⁸ in the aforementioned general formula (2).

These groups, represented by R³³ to R⁴⁴, may be unsubstituted, but may have a substituent. The substituent can be exemplified by the same ones as those shown in connection with the substituent that the aromatic hydrocarbon group, the aromatic heterocyclic group, or the condensed polycyclic aromatic group represented by Ar¹ to Ar⁵ in the general formula (1) may have. The same holds true of the embodiments that the substituent can adopt.

### r³³ to r⁴⁴:

r³³ to r⁴⁴, respectively, show the numbers of the groups R³³sto R⁴⁴sbound to the aromatic rings. r³³, r³⁴, r³⁷, r⁴⁰, r⁴³ and r⁴⁴ each denote an integer of 0 to 5. r³⁵, r³⁶, r³⁸, r³⁹, r⁴¹ and r⁴² each denote an integer of 0 to 4. A case where r³³ to r⁴⁴ are each 0 means that there are none of R³³ to R⁴⁴ on the aromatic rings, namely, that none of the aromatic rings are substituted by the groups represented by R³³ to R⁴⁴.

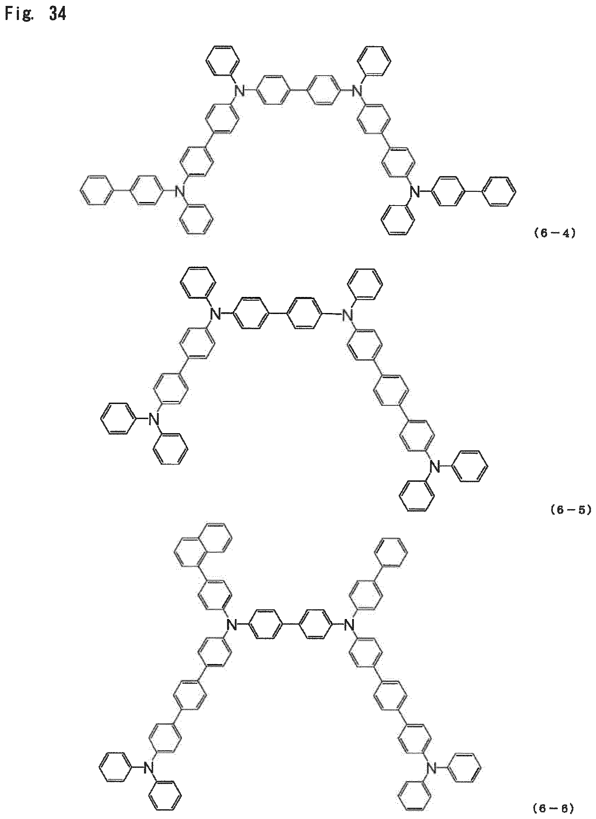

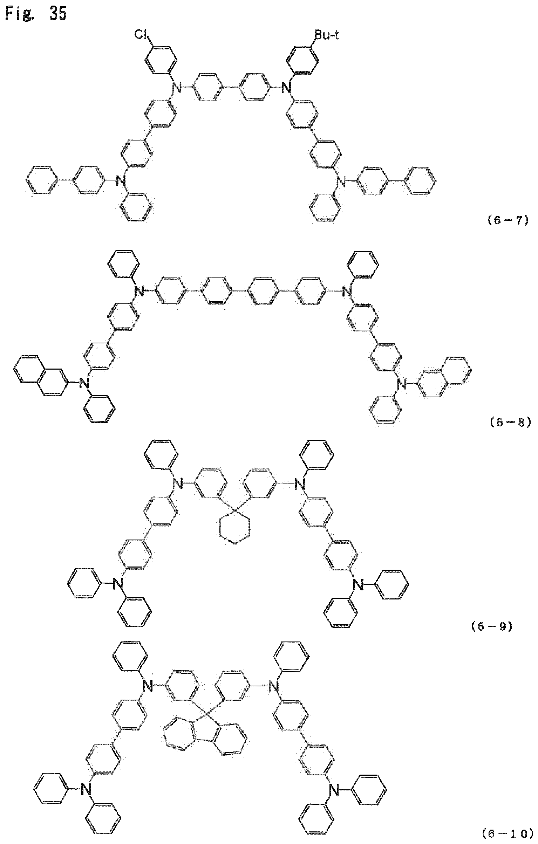

If r³³, r³⁴, r³⁷, r⁴⁰, r⁴³ and r⁴⁴ are each an integer of 2 to 5, or if r³⁵, r³⁶, r³⁸, r³⁹, r⁴¹ and r⁴² are each an integer of 2 to 4, a plurality of R³³s to R⁴⁴s are bound to the same benzene rings. In this case, the plurality of groups bound may be the same or different. Moreover, they may be present independently of each other so as not to form a ring, but may bind to each other via a single bond, a substituted or unsubstituted methylene group, an oxygen atom, or a sulfur atom to form a ring. In Compound 6-8 of Fig. 34 and Compound 6-12 of Fig. 35, for example, a plurality of substituents (2 vinyl groups) are bound together via a single bond to form a naphthalene ring.

### L² to L⁴:

L² to L⁴ are each a bridge group connecting the two triarylamine structures. L² to L⁴ may be the same or different, and each represents a single bond, or a divalent group represented by any of the following structural formulas (B') to (G).

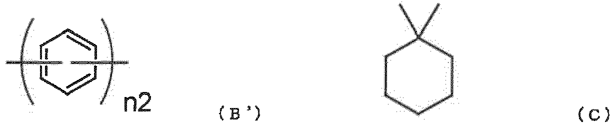

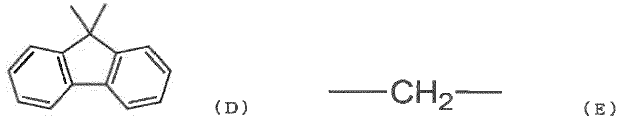

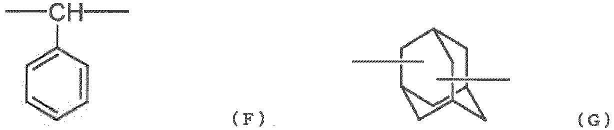

In the structural formula (B'), n2 denotes an integer of 1 to 3. The divalent group represented by any of the structural formulas (B') to (G) may be unsubstituted, but may be substituted by deuterium.

### Preferred embodiments:

Preferred embodiments of the triarylamine compound VI will be described below. In the description of the preferred embodiments, the groups without the designation of "substituted" or "unsubstituted" may have a substituent or may be unsubstituted.

R³³ to R⁴⁴ may be the same or different. As R³³ to R⁴⁴, a deuterium atom, a chlorine atom, an alkyl group having 1 to 6 carbon atoms, an alkenyl group having 2 to 6 carbon atoms, an aromatic hydrocarbon group, or a condensed polycyclic aromatic group is preferred; a deuterium atom, a chlorine atom, a tert-butyl group, a phenyl group, a biphenylyl group, a naphthyl group, or a vinyl group is more preferred; and a deuterium atom, a phenyl group, a biphenylyl group, a naphthyl group, or a vinyl group is particularly preferred. Formation of a condensed aromatic ring upon binding of these groups via a single bond, as in Compound 6-8 of Fig. 35, is also preferred. A deuterium atom, a phenyl group, or a biphenylyl group is most preferred.

r³³ to r⁴⁴ may be the same or different and, as r³³ to r⁴⁴, an integer of 0 to 3 is preferred, and an integer of 0 to 2 is more preferred.

L² to L⁴ may be the same or different. As L² to L⁴, the divalent group represented by the structural formula (B') or (D), or a single bond is preferred, and the divalent group represented by the structural formula (B'), or a single bond is more preferred.

As n2 in the structure formula (B'), 1or 2 is preferred, and 1 is more preferred.

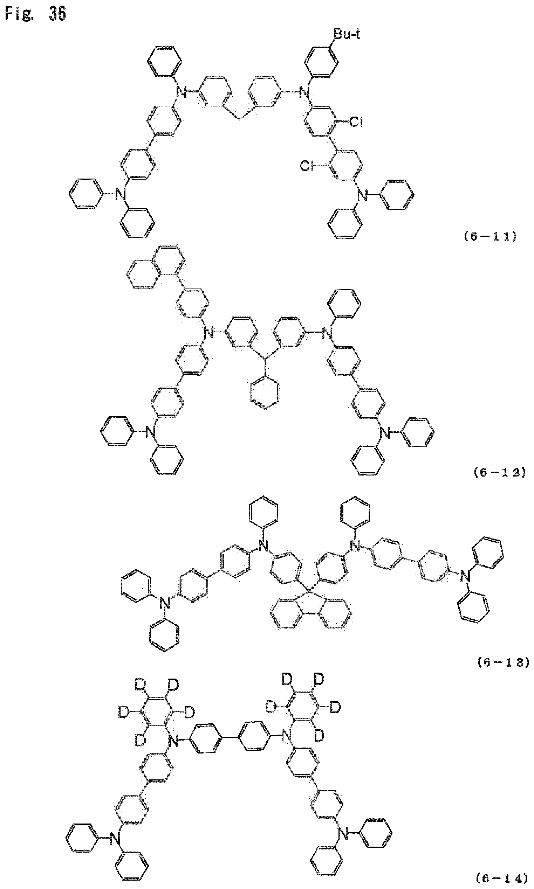

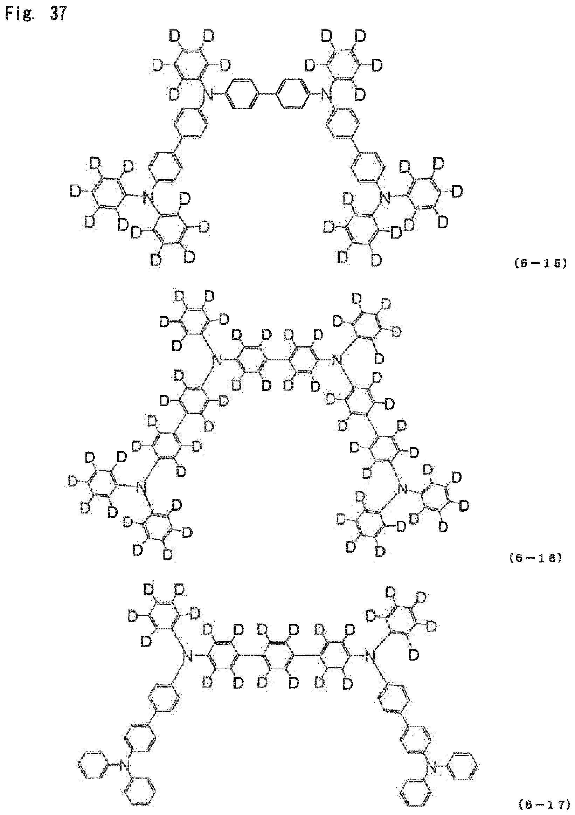

Preferred examples of the triarylamine compound VI are shown in Figs. 33 to 37, but the triarylamine compounds VI are not limited to these compounds. D represents a deuterium atom.

The triarylamine compound VI can be synthesized in accordance with a method publicly known per se (see Patent Documents 1, 10 and 11).

### (Second hole transport layer 4b)

In an embodiment in which the hole transport layer has a two-layer structure composed of the first hole transport layer 4a and the second hole transport layer 4b, the second hole transport layer 4b is provided on the first hole transport layer 4a. The composition of the second hole transport layer 4b is different from the composition of the first hole transport layer 4a. For the second hole transport layer 4b, the aforementioned publicly known hole transporting material may be used, but it is preferred to use the aforesaid arylamine compound I, because the arylamine compound I has high electron blocking performance.

### <Electron blocking layer>

An electron blocking layer (not shown) can be provided between the hole transport layer 4 and the luminous layer 5. For the electron blocking layer, the aforementioned arylamine compound I is used preferably. Furthermore, a publicly known compound having electron blocking action, for example, as exemplified below can be used:
Carbazole compounds, for example,
   4,4',4"-tri(N-carbazolyl)triphenylamine (TCTA),
   9,9-bis[4-(carbazol-9-yl)phenyl]fluorene,
   1,3-bis(carbazol-9-yl)benzene (mCP), and
   2,2-bis(4-carbazol-9-ylphenyl)adamantane (Ad-Cz); and

Triarylamine compounds having a triphenylsilyl group and a triarylamine structure, for example, 9-[4-(carbazol-9-yl)phenyl]-9-[4-(triphenylsilyl)phenyl]-9H-fluorene.

If the electron blocking layer is provided, the composition of the electron blocking layer and the composition of the hole transport layer 4 (the second hole transport layer 4b, if the hole transport layer has a two-layered structure) are different.

### <Luminous layer 5>

The luminous layer 5 is formed on the hole transport layer 4 (or the electron blocking layer). The luminous layer 5 includes the aforementioned indenoindole compound II or carbazole compound III.

For the luminous layer 5, a publicly known luminescent material may be used concurrently. As the publicly known luminescent material, use can be made of various metal complexes, such as metal complexes of quinolinol derivatives including Alq₃; anthracene derivatives; bisstyrylbenzene derivatives; pyrene derivatives; oxazole derivatives; and polyparaphenylenevinylene derivatives. In the luminous layer 5, a red light emitting material can be used preferably.

The luminous layer 5 may be composed of a host material and a dopant material.

As the host material, the above indenoindole compound II or the above carbazole compound III is used preferably. Other examples usable include the above luminescent materials; thiazole derivatives; benzimidazole derivatives; and polydialkylfluorene derivatives.

As the dopant material, there can be used quinacridone, coumarin, rubrene, perylene, pyrene, and derivatives thereof; benzopyran derivatives; indenophenanthrene derivatives; rhodamine derivatives; and aminostyryl derivatives.

Furthermore, a phosphorescent luminous body can be used as the luminescent material. As the phosphorescent luminous body, a phosphorescent luminous body in the form of a metal complex containing iridium, platinum or the like can be used. Concretely, a green phosphorescent luminous body such as Ir(ppy)₃; a blue phosphorescent luminous body such as FIrpic or FIr6; or a red phosphorescent luminous body such as Btp₂Ir(acac) is used. Of these materials, the red phosphorescent luminous body is preferred.

As the host material in this case, the above-mentioned indenoindole compound II or carbazole compound III is preferably used. In addition, the following hole injecting/transporting host material, for example, can be used:
A carbazole compound, for example, 4,4'-di(N-carbazolyl)biphenyl (CBP), TCTA, or mCP.

The following electron transporting host material, for example, is also usable:
p-bis(triphenylsilyl)benzene (UGH2), or 2,2',2"-(1,3,5-phenylene)-tris(1-phenyl-1H-benzimidazole) (TPBI) .

By using such a host material, a high performance organic EL device can be prepared.

Doping of the host material with the phosphorescent luminous body is preferably performed by codeposition in a range of 1 to 30% by weight based on the entire luminous layer in order to avoid concentration quenching.

Moreover, a material which emits delayed fluorescence, such as a CDCB derivative, for example, PIC-TRZ, CC2TA, PXZ-TRZ, or 4CzIPN, can be used as the luminescent material.

### <Hole blocking layer>

A hole blocking layer (not shown) can be provided on the luminous layer 5. For the hole blocking layer, a publicly known compound having hole blocking action can be used. Examples of the publicly known compound having hole blocking action include phenanthroline derivatives, e.g., bathocuproine (BCP); metal complexes of quinolinol derivatives, e.g., aluminum(III) bis(2-methyl-8-quinolinato)-4-phenylphenolate (BAlq); various rare earth complexes; triazole derivatives; triazine derivatives; and oxadiazole derivatives. These materials may also concurrently serve as the materials for the electron transport layer 6.

### <Electron transport layer 6>

The electron transport layer 6 is formed on the luminous layer 5 (or the hole blocking layer). For the electron transport layer 6, an anthracene derivative IV represented by the following general formula (4) is preferably used.

### Anthracene derivative IV:

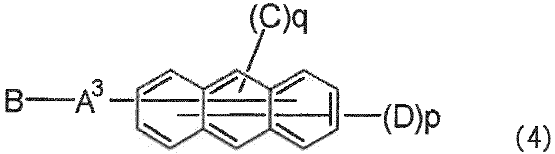

For the anthracene derivative IV, the following three embodiments, for example, are present:

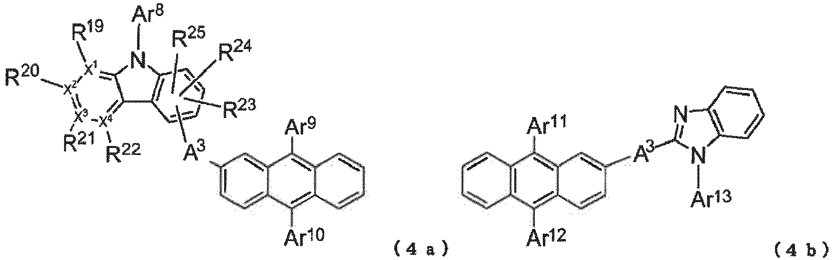

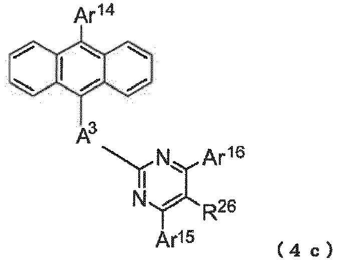

### A³:

A³ represents a divalent group of an aromatic hydrocarbon, a divalent group of an aromatic heterocycle, a divalent group of a condensed polycyclic aromatic, or a single bond.

Examples of the divalent group of an aromatic hydrocarbon, the divalent group of an aromatic heterocycle, or the divalent group of a condensed polycyclic aromatic, represented by A³, are the same groups as those shown in connection with the divalent group of an aromatic hydrocarbon, the divalent group of an aromatic heterocycle, or the divalent group of a condensed polycyclic aromatic represented by A¹ in the aforementioned general formula (2).

These divalent groups represented by A³ may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

### B:

B represents an aromatic heterocyclic group. Its examples include a pyridyl group, a pyrimidinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group.

The aromatic heterocyclic group represented by B may be unsubstituted, but may have a substituent. The substituent can be exemplified by the following groups, in addition to a deuterium atom, a cyano group, and a nitro group:
a halogen atom, for example, a fluorine atom, a chlorine atom, a bromine atom, or an iodine atom;
an alkyl group having 1 to 6 carbon atoms, for example, a methyl group, an ethyl group, an n-propyl group, an isopropyl group, an n-butyl group, an isobutyl group, a tert-butyl group, an n-pentyl group, an isopentyl group, a neopentyl group, or an n-hexyl group;
a cycloalkyl group having 5 to 10 carbon atoms, for example, a cyclopentyl group, a cyclohexyl group, a 1-adamantyl group, or a 2-adamantyl group;
an alkyloxy group having 1 to 6 carbon atoms, for example, a methyloxy group, an ethyloxy group, or a propyloxy group;
a cycloalkyloxy group having 5 to 10 carbon atoms, for example, a cyclopentyloxy group, a cyclohexyloxy group, a 1-adamantyloxy group, or a 2-adamantyloxy group;
an alkenyl group, for example, a vinyl group or an allyl group;
an aryloxy group, for example, a phenyloxy group or a tolyloxy group;
an arylalkyloxy group, for example, a benzyloxy group or a phenethyloxy group;
an aromatic hydrocarbon group or a condensed polycyclic aromatic group, for example, a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group;
an aromatic heterocyclic group, for example, a pyridyl group, a furyl group, a thienyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, or a carbolinyl group;
an aryloxy group, for example, a phenyloxy group, a biphenylyloxy group, a naphthyloxy group, an anthracenyloxy group, or a phenanthrenyloxy group;
an arylvinyl group, for example, a styryl group, or a naphthylvinyl group; and
an acyl group, for example, an acetyl group, or a benzoyl group.

The alkyl group having 1 to 6 carbon atoms, the alkyloxy group having 1 to 6 carbon atoms, and the alkenyl group may be straight-chain or branched. Any of these substituents may be unsubstituted, but may be further substituted by the above exemplary substituent. These substituents may be present independently of each other so as not to form a ring. However, they may bind to each other via a single bond, a substituted or unsubstituted methylene group, an oxygen atom, or a sulfur atom to form a ring.

### C:

C represents an aromatic hydrocarbon group, an aromatic heterocyclic group, or a condensed polycyclic aromatic group. If there are two of the groups C (q=2), the two Cs may be the same or different.

Examples of the aromatic hydrocarbon group, the aromatic heterocyclic group, or the condensed polycyclic aromatic group, represented by C, are the same ones as those shown in connection with the aromatic hydrocarbon group, the aromatic heterocyclic group, or the condensed polycyclic aromatic group represented by Ar¹ to Ar⁵ in the general formula (1).

These groups represented by C may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

### D:

Ds may be the same or different, and each represents a hydrogen atom, a deuterium atom, a fluorine atom, a chlorine atom, a cyano group, a trifluoromethyl group, an alkyl group having 1 to 6 carbon atoms, an aromatic hydrocarbon group, an aromatic heterocyclic group, or a condensed polycyclic aromatic group. The alkyl group having 1 to 6 carbon atoms may be straight-chain or branched.

The alkyl group having 1 to 6 carbon atoms, represented by D, can be exemplified by a methyl group, an ethyl group, an n-propyl group, an isopropyl group, an n-butyl group, an isobutyl group, a tert-butyl group, an n-pentyl group, an isopentyl group, a neopentyl group, and an n-hexyl group.

The alkyl group having 1 to 6 carbon atoms, represented by D, may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

Examples of the aromatic hydrocarbon group, the aromatic heterocyclic group, or the condensed polycyclic aromatic group, represented by D, are the same ones as those shown in connection with the aromatic hydrocarbon group, the aromatic heterocyclic group, or the condensed polycyclic aromatic group represented by Ar¹ to Ar⁵ in the general formula (1) .

These groups represented by D may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

### p, q:

In connection with p and q, provided that the sum of p and q is 9, p denotes 7 or 8, while q denotes 1 or 2.

A plurality of the groups D bound to the anthracene ring may be present independently of each other so as not to form a ring. However, they may bind to each other via a single bond, a substituted or unsubstituted methylene group, an oxygen atom, or a sulfur atom to form a ring.

If q is 2, the two groups C bound to the anthracene ring may be present independently of each other so as not to form a ring. However, they may bind to each other via a single bond, a substituted or unsubstituted methylene group, an oxygen atom, or a sulfur atom to form a ring.

### Ar⁸ to Ar¹⁶:

Ar^{B} to Ar¹⁶ may be the same or different, and each represents an aromatic hydrocarbon group, an aromatic heterocyclic group, or a condensed polycyclic aromatic group.

The aromatic hydrocarbon group, the aromatic heterocyclic group, or the condensed polycyclic aromatic group, represented by Ar⁸ to Ar¹⁶, can be exemplified by the same ones as those shown in connection with the aromatic hydrocarbon group, the aromatic heterocyclic group, or the condensed polycyclic aromatic group represented by Ar¹ to Ar⁵ in the general formula (1) .

These groups represented by Ar⁸ to Ar¹⁶ may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

### R¹⁹ to R²⁶:

R¹⁹ to R²⁶ may be the same or different, and each represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group. The alkyl group having 1 to 6 carbon atoms, the alkenyl group having 2 to 6 carbon atoms, and the alkyloxy group having 1 to 6 carbon atoms may be straight-chain or branched.

R¹⁹ to R²⁵ may be present independently of each other so as not to form a ring. However, they may bind to each other via a single bond, a substituted or unsubstituted methylene group, an oxygen atom, or a sulfur atom to form a ring.

The alkyl group having 1 to 6 carbon atoms, the cycloalkyl group having 5 to 10 carbon atoms, the alkenyl group having 2 to 6 carbon atoms, the alkyloxy group having 1 to 6 carbon atoms, or the cycloalkyloxy group having 5 to 10 carbon atoms, represented by R¹⁹ to R²⁶, can be exemplified by the same ones as those shown in connection with the alkyl group having 1 to 6 carbon atoms, the cycloalkyl group having 5 to 10 carbon atoms, the alkenyl group having 2 to 6 carbon atoms, the alkyloxy group having 1 to 6 carbon atoms, or the cycloalkyloxy group having 5 to 10 carbon atoms, represented by R¹ to R⁸ in the general formula (2) .

These substituents represented by R¹⁹ to R²⁶ may be unsubstituted, but may have a substituent. Examples of the substituent are the same as those shown in connection with the substituent that the alkyl group having 1 to 6 carbon atoms, the cycloalkyl group having 5 to 10 carbon atoms, or the alkenyl group having 2 to 6 carbon atoms, represented by R¹ to R⁸ in the general formula (2), may have. The same holds true of the embodiments that the substituent can adopt.

The aromatic hydrocarbon group, the aromatic heterocyclic group, or the condensed polycyclic aromatic group, represented by R¹⁹ to R²⁶, can be exemplified by the same ones as those shown in connection with the aromatic hydrocarbon group, the aromatic heterocyclic group, or the condensed polycyclic aromatic group represented by Ar¹ to Ar⁵ in the general formula (1) .

These groups represented by R¹⁹ to R²⁶ may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

The aryloxy group, represented by R¹⁹ to R²⁶, can be exemplified by the same groups as those shown in connection with the aryloxy group represented by R¹ to R⁸ in the general formula (2) .

These groups represented by R¹⁹ to R²⁶ may be unsubstituted, but may have a substituent. Examples of the substituent are the same as those shown in connection with the substituent that the aromatic hydrocarbon group, the aromatic heterocyclic group, or the condensed polycyclic aromatic group, represented by Ar¹ to Ar⁵ in the general formula (1), may have. The same holds true of the embodiments that the substituent can adopt.

### X¹ to X⁴:

X¹ to X⁴ each represents a carbon atom or a nitrogen atom, and only one of X¹ to X⁴ is a nitrogen atom. The nitrogen atom in this case does not have the hydrogen atom or the substituent of R¹⁹ to R²². That is, if X¹ is a nitrogen atom, R¹⁹ is not present; if X² is a nitrogen atom, R²⁰ is not present; if X³ is a nitrogen atom, R²¹ is not present; and if X⁴ is a nitrogen atom, R²² is not present.

### Preferred embodiments:

Preferred embodiments of the anthracene derivative IV will be described below. In the description of the preferred embodiments, the groups without the designation of "substituted" or "unsubstituted" may have a substituent or may be unsubstituted.

The anthracene derivative IV is preferably represented by the aforementioned general formula (4a), (4b) or (4c).

The anthracene derivative IV represented by the general formula (4a) is more preferably represented by the following general formula (4a'): 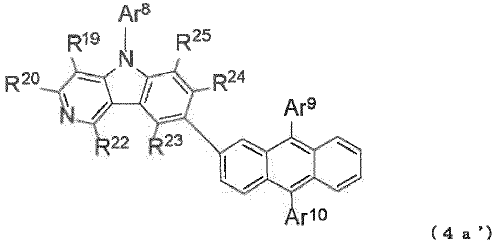 where
Ar⁸ to Ar¹⁰, R¹⁹, R²⁰ and R²² to R²⁵ have meanings as defined in the general formula (4).

As A³, a single bond, a divalent group of an aromatic hydrocarbon, or a divalent group of a condensed polycyclic aromatic is preferred; a single bond, or a divalent group formed by removing two hydrogen atoms from benzene, biphenyl, terphenyl, naphthalene, anthracene, fluorene, or phenanthrene is more preferred; and a single bond, or a divalent group formed by removing two hydrogen atoms from benzene, biphenyl, naphthalene, fluorene, or phenanthrene is particularly preferred. In the general formulas (4b) and (4c), the divalent group of an aromatic hydrocarbon is particularly preferred.

As B, a nitrogen-containing aromatic heterocyclic group is preferred; a pyridyl group, a pyrimidinyl group, a pyrrolyl group, a quinolyl group, an isoquinolyl group, an indolyl group, a carbazolyl group, a benzoxazolyl group, a benzothiazolyl group, a quinoxalinyl group, a benzimidazolyl group, a pyrazolyl group, or a carbolinyl group is more preferred; and a pyridyl group, a pyrimidinyl group, a quinolyl group, an isoquinolyl group, an indolyl group, a benzimidazolyl group, a pyrazolyl group, or a carbolinyl group is particularly preferred.

As C, an aromatic hydrocarbon group or a condensed polycyclic aromatic group is preferred; and a phenyl group, a biphenylyl group, a naphthyl group, an anthracenyl group, a phenanthrenyl group, or a fluorenyl group is more preferred. If there are two of Cs, the,two Cs may be the same or different.

Ds may be the same or different, but a hydrogen atom, a deuterium atom, a fluorine atom, a chlorine atom, a cyano group, a trifluoromethyl group, or an alkyl group having 1 to 6 carbon atoms is preferred, and a hydrogen atom is more preferred.

As Ar⁸, an aromatic hydrocarbon group or a condensed polycyclic aromatic group is preferred; a phenyl group, a biphenylyl group, a naphthyl group, an anthracenyl group, a phenanthrenyl group, or a fluorenyl group is more preferred; and a phenyl group is particularly preferred.

Ar⁹ and Ar¹⁰ may be the same or different. As Ar⁹ and Ar¹⁰, an aromatic hydrocarbon group or a condensed polycyclic aromatic group is preferred; a phenyl group, a biphenylyl group, a naphthyl group, an anthracenyl group, a phenanthrenyl group, or a fluorenyl group is more preferred; and a phenyl group is particularly preferred.

Ar¹¹ and Ar¹² may be the same or different. As Ar¹¹ and Ar¹², an aromatic hydrocarbon group or a condensed polycyclic aromatic group is preferred; a phenyl group, a biphenylyl group, a naphthyl group, an anthracenyl group, a phenanthrenyl group, or a fluorenyl group is more preferred; and a phenyl group, a naphthyl group, or an anthracenyl group is particularly preferred.

As Ar¹³, an aromatic hydrocarbon group or a condensed polycyclic aromatic group is preferred; a phenyl group, a biphenylyl group, a naphthyl group, an anthracenyl group, a phenanthrenyl group, or a fluorenyl group is more preferred; and a phenyl group is particularly preferred.

As Ar¹⁴, an aromatic hydrocarbon group, a condensed polycyclic aromatic group, a pyridyl group, a quinolyl group, an isoquinolyl group, or a carbolinyl group is preferred; a phenyl group, a biphenylyl group, a naphthyl group, an anthracenyl group, a phenanthrenyl group, a fluorenyl group, a pyridyl group, a quinolyl group, an isoquinolyl group, or a carbolinyl group is more preferred; and a phenyl group, a naphthyl group, or an anthracenyl group is particularly preferred.

Ar¹⁵ and Ar¹⁶ may be the same or different. As Ar¹⁵ and Ar¹⁶, an aromatic hydrocarbon group, a condensed polycyclic aromatic group, a pyridyl group, a quinolyl group, an isoquinolyl group, or a carbolinyl group is preferred; a phenyl group, a biphenylyl group, a naphthyl group, an anthracenyl group, a phenanthrenyl group, a fluorenyl group, a pyridyl group, a quinolyl group, an isoquinolyl group, or a carbolinyl group is more preferred; and a phenyl group, a naphthyl group, a phenanthrenyl group, a pyridyl group, or a carbolinyl group is particularly preferred. If these groups have a substituent, as the substituent, an aromatic hydrocarbon group, a condensed polycyclic aromatic group, or an aromatic heterocyclic group is preferred, and a phenyl group, a naphthyl group, a phenanthrenyl group, or a pyridyl group is preferred.

R¹⁹ to R²² may be the same or different. As R¹⁹ to R²², a hydrogen atom, a deuterium atom, an aromatic hydrocarbon group, or an aromatic heterocyclic group is preferred; and a hydrogen atom, a deuterium atom, a phenyl group, or a pyridyl group is preferred.

R²³ to R²⁵ are preferably hydrogen atoms.

As R²⁶, a hydrogen atom is preferred.

Of X¹ to X⁴, X³ being a nitrogen atom is preferred.

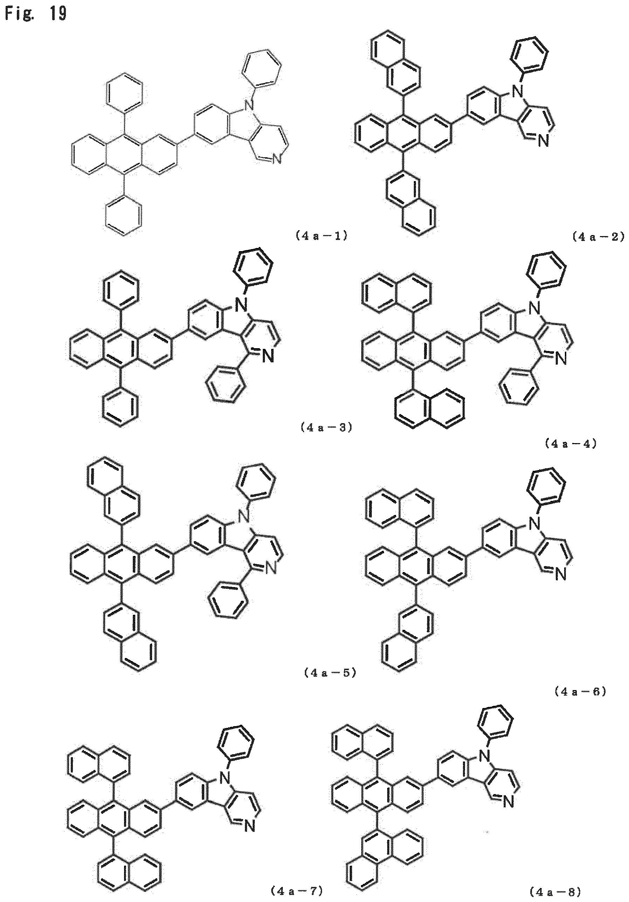

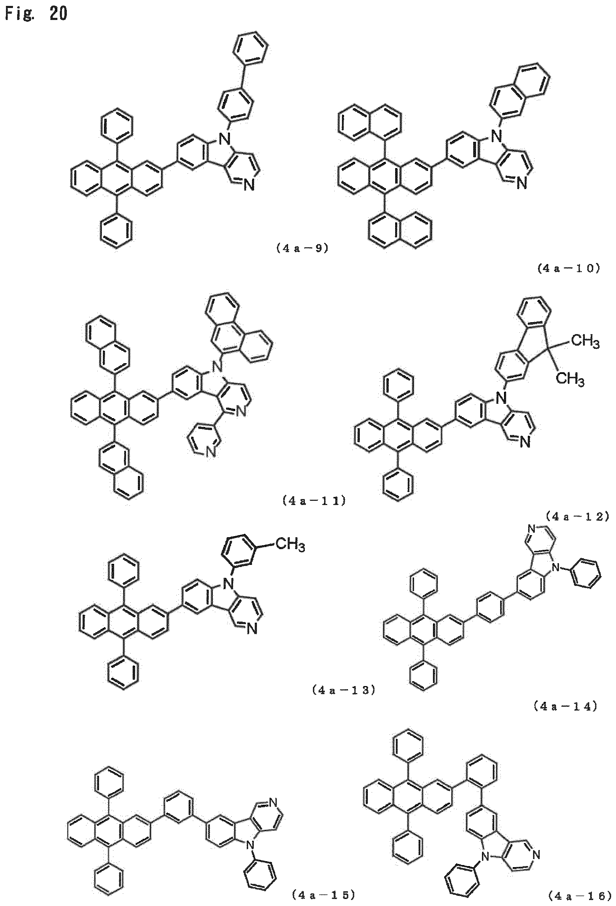

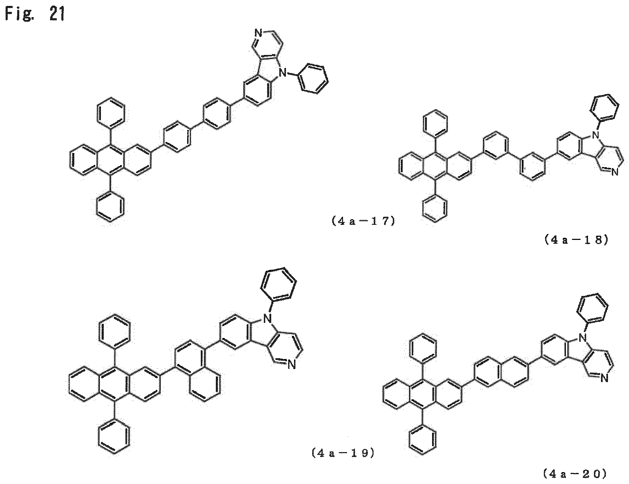

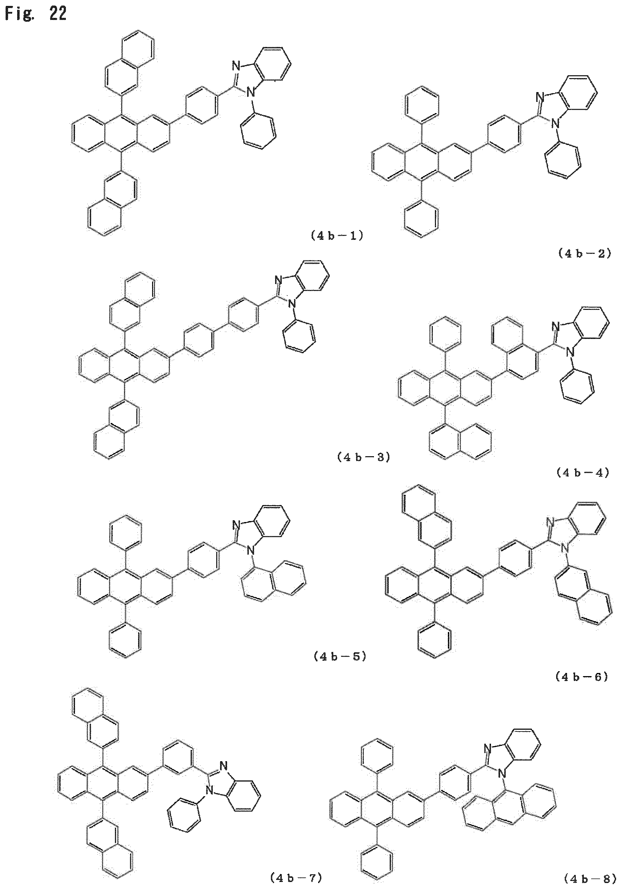

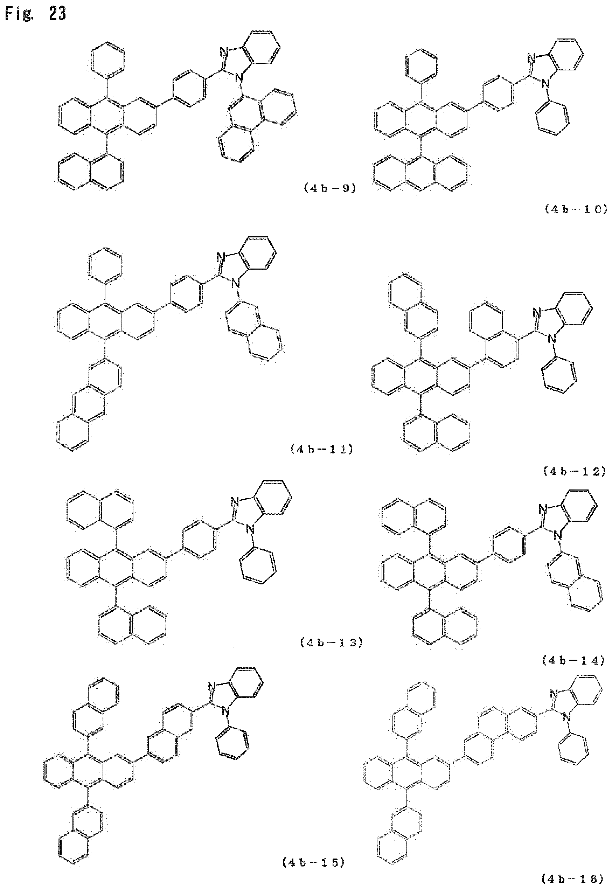

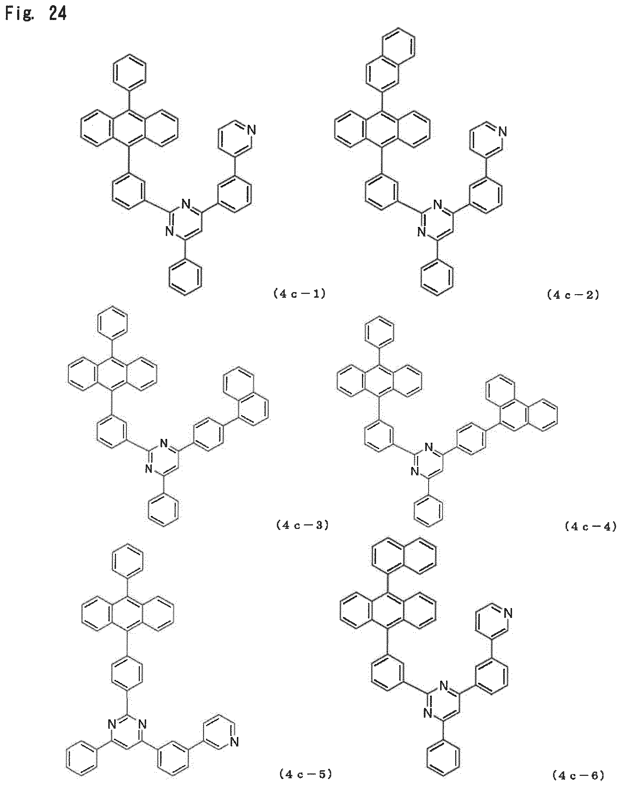

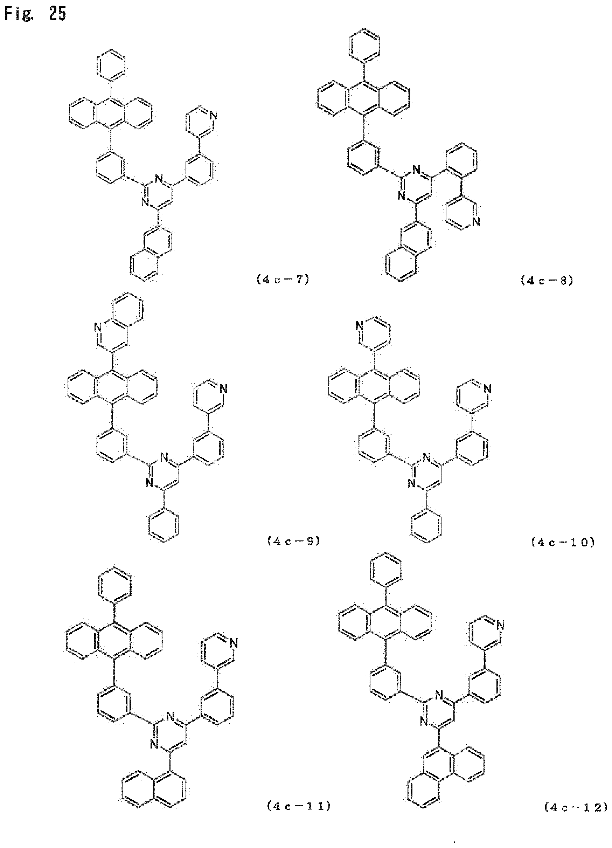

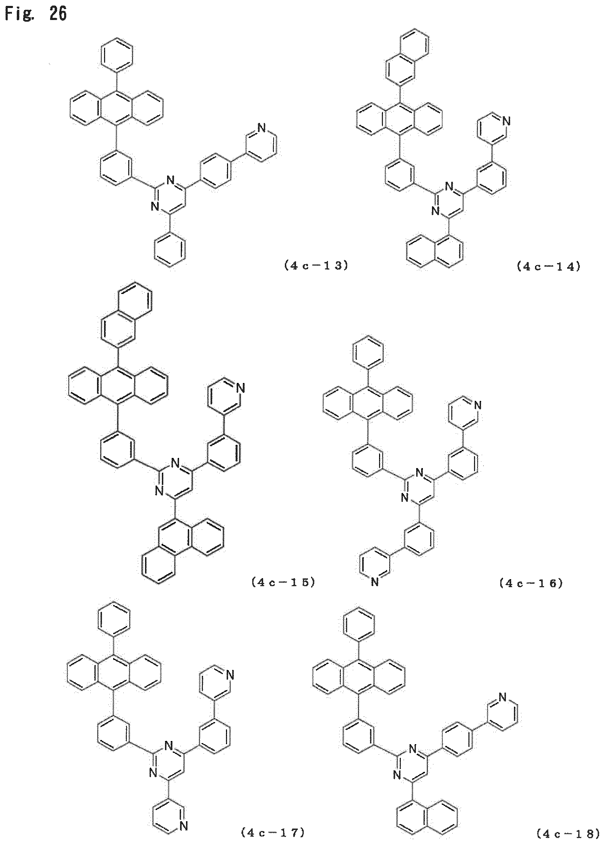

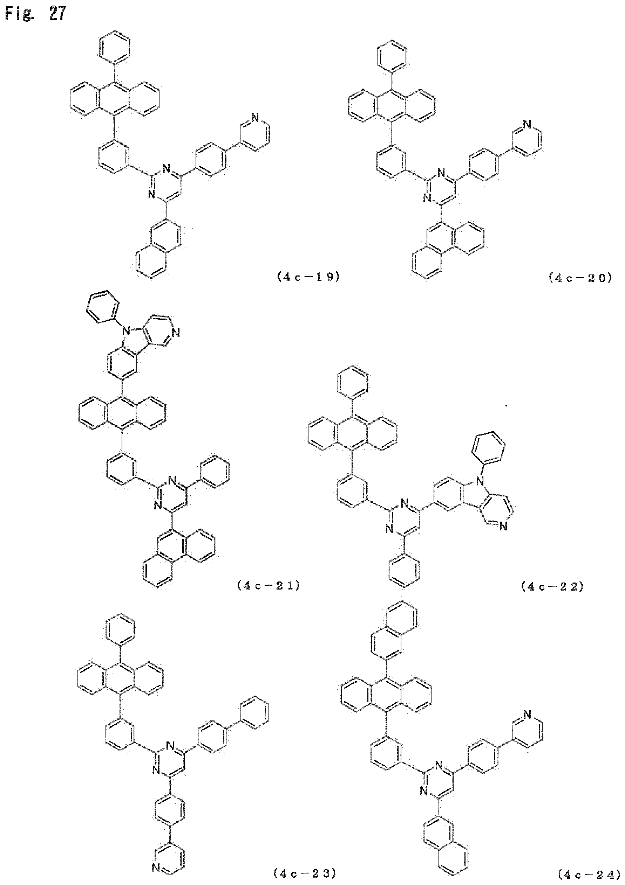

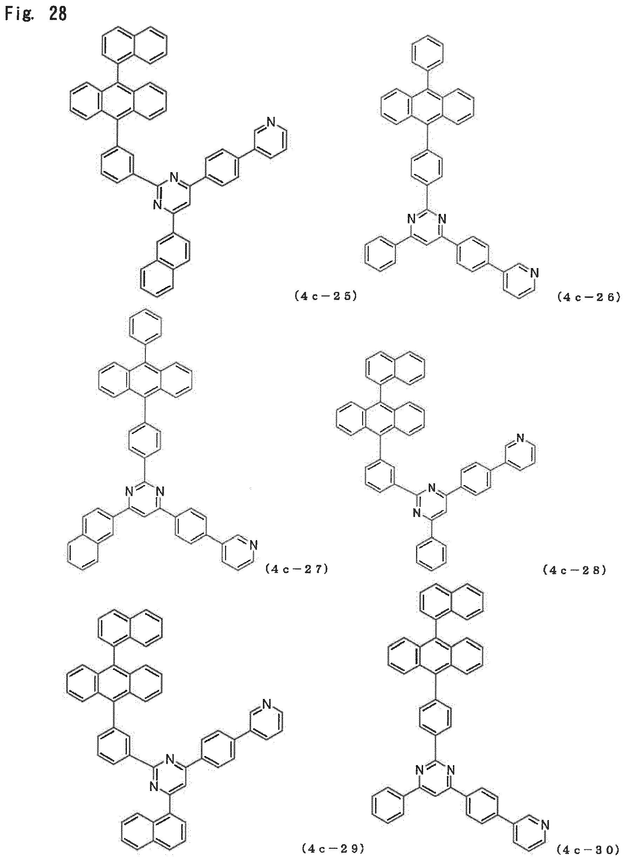

Examples of the preferred compound among the anthracene derivatives IV are shown in Figs. 19 to 28, but the anthracene derivatives IV are not limited to these compounds.

The above-mentioned anthracene derivatives IV can be synthesized by a method publicly known per se (see Patent Documents 7 to 9).

For the electron transport layer 6, a publicly known material with electron transport properties may be used, as long as this material does not impair the effects of the present invention. Examples of the publicly known material with electron transport properties include various metal complexes such as metal complexes of quinolinol derivatives including Alq₃ and BAIq; triazole derivatives; triazine derivatives; oxadiazole derivatives; pyridine derivatives; pyrimidine derivatives; benzimidazole derivatives; thiadiazole derivatives; benzotriazole derivatives; carbodiimide derivatives; quinoxaline derivatives; pyridoindole derivatives; phenanthroline derivatives; and silole derivatives.

### <Electron injection layer 7>

For the electron injection layer 7, there can be used an alkali metal salt such as lithium fluoride or cesium fluoride; an alkaline earth metal salt such as magnesium fluoride; or a metal oxide such as aluminum oxide. However, such a material can be omitted in the suitable selection of the electron transport layer and the cathode.

### <Cathode 8>

For the cathode 8, an electrode material with a low work function such as aluminum, or an alloy having a lower work function, such as a magnesium-silver alloy, a magnesium-indium alloy, or an aluminum-magnesium alloy, is used as an electrode material.

### Examples

The embodiments of the present invention will now be described more specifically by the following Examples, but the present invention is in no way limited to these Examples:

### <Synthesis Example 1: Compound 1-9>

Synthesis of bis(biphenyl-4-yl)-(1,1':2',1ʹʹ:3ʹ,1ʹʹʹ-quaterphenyl)-5'-amine:
A nitrogen-purged reaction vessel was charged with

| | | |
|---|---|---|
| | bis(biphenyl-4-yl)amine | 23.5 g, |
| | 5'-bromo-1,1':3',1"-terphenyl | 23.8 g, |
| | t-butoxysodium | 9.2 g, |
| and | | |
| | toluene | 240 ml. |

A nitrogen gas was passed through the charge for 30 minutes under ultrasonic irradiation to obtain a mixture. To the mixture,

| | | |
|---|---|---|
| | palladium acetate | 0.33 g, |
| and | | |
| | a 50% (w/v) toluene solution of t-butylphosphine | |
| | | 1.0mL |

were added, followed by heating and stirring the mixture for 4 hours at 85°C to obtain a reaction liquid. From the reaction liquid after stirring, insolubles were removed by filtration, and then concentration was performed. Acetone was added to the residue, solids were dispersed and washed, and the solids were collected by filtration. As a result, 34.0 g (yield 85%) of bis(biphenyl-4-yl)-(1,1':3',1"-terphenyl)-5'-amine was obtained as a white powder.

To a nitrogen-purged reaction vessel, 15.0 g of the resulting bis(biphenyl-4-yl)-(1,1':3',1"-terphenyl)-5'-amine and 300 mL of dichloromethane were added. The mixture was cooled in an ice bath to obtain a mixture. To the mixture, 2.9 g of N-bromosuccinimide was slowly added, and 2.0 g of N-bromosuccinimide was further added 2 hours later to prepare a reaction liquid. The reaction liquid was stirred for 3 hours at 10°C or lower, whereafter the reaction liquid was heated to room temperature and stirred for 2 days. Water was added to the reaction liquid after stirrig, and methanol was further added to precipitate solids. The precipitated solids were collected by filtration. The resulting solids were dissolved with heating in toluene, and the solution was subjected to adsorption purification using silica gel. The filtrate was concentrated, and methanol was added to precipitate solids, which were collected by filtration. As a result, 16.0 g (yield 93%) of bis(biphenyl-4-yl)-(2'-bromo-1,1':3',1"-terphenyl)-5'-amine was obtained as a white powder.

A nitrogen-purged reaction vessel was charged with

| | | |
|---|---|---|
| | bis(biphenyl-4-yl)-(2'-bromo-1,1':3',1"-terphenyl)-5'-amine | 10.0 g, |
| | toluene | 80 mL, |
| | ethanol | 40 mL, |
| and | | |
| | phenylboronic acid | 2.3 g. |

Then, an aqueous solution of 3.0 g of potassium carbonate dissolved in 30 mL of water was added, and a nitrogen gas was passed through the mixture for 30 minutes under ultrasonic irradiation to obtain a mixture. To the mixture, 0.37 g of tetrakistriphenylphosphine palladium was added to obtain a reaction liquid. The reaction liquid was heated, and stirred for 1 day at 72°C. The reaction liquid after stirring was cooled to room temperature, and an organic layer was collected by a liquid separating operation. The organic layer was subjected to adsorption purification using silica gel, and then to adsorption purification using activated carbon. Recrystallization using THF and acetone was performed. As a result, 4.0 g (yield 40%) of Compound 1-9 was obtained as a white powder.

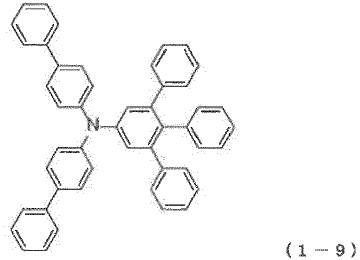

In connection with the resulting white powder, its structure was identified using NMR. In ¹H-NMR (CDC1₃), the following signals of 35 hydrogens were detected:

| | |
|---|---|
| δ (ppm) = | 7.56-7.65 (6H) |
| | 7.27-7.49 (12H) |
| | 7.00-7.15 (13H) |
| | 6.87-6.90 (4H) |

### <Synthesis Example 2: Compound 1-10>

Synthesis of bis(biphenyl-4-yl)-(1,1':2' ,1":4",1" ':3' ,1""-quinquephenyl)-5'-amine:
Reactions were performed under the same conditions as in Synthesis Example 1, except that
4-biphenylboronic acid
   was used instead of
phenylboronic acid.
As a result, 13.2 g (yield 74%) of Compound 1-10 was obtained as a white powder.

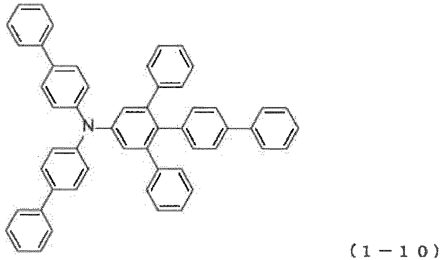

In connection with the resulting white powder, its structure was identified using NMR. In ¹H-NMR (CDC1₃), the following signals of 39 hydrogens were detected:

| | |
|---|---|
| δ (ppm) = | 7.54-7.65 (8H) |
| | 7.27-7.49 (17H) |
| | 7.09-7.16 (10H) |
| | 6.93-6.95 (4H) |

### <Synthesis Example 3: Compound 1-13>

Synthesis of bis(biphenyl-4-yl)-4"-(1-naphthalenyl)-(1,1' :2' ,1":3' ,1"'-quaterphenyl)-4'-amine:
Reactions were performed under the same conditions as in Synthesis Example 1, except that
4-(1-naphthalenyl)phenylboronic acid
   was used instead of
phenylboronic acid.
As a result, 8.7 g (yield 73%) of Compound 1-13 was obtained as a white powder.

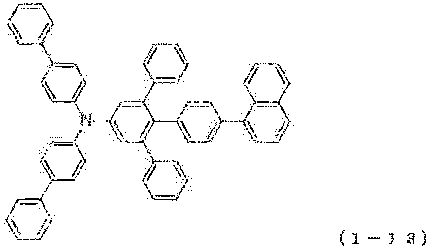

In connection with the resulting white powder, its structure was identified using NMR. In ¹H-NMR (CDC1₃), the following signals of 41 hydrogens were detected:

| | |
|---|---|
| δ (ppm) = | 7.82-7.92 (2H) |
| | 7.59-7.70 (8H) |
| | 7.12-7.53 (27H) |
| | 6.99-7.02 (4H) |

### <Synthesis Example 4: Compound 1-39>

Synthesis of bis [4-(1-naphthalenyl)phenyl]-(1,1':2',1":3',1"'-quaterphenyl)-5'-amine:
Reactions were performed under the same conditions as in Synthesis Example 1, except that
bis[4-(1-naphthalenyl)phenyl]amine
   was used instead of
bis(biphenyl-4-yl)amine.
As a result, 4.3 g (yield 43%) of Compound 1-39 was obtained as a white powder.

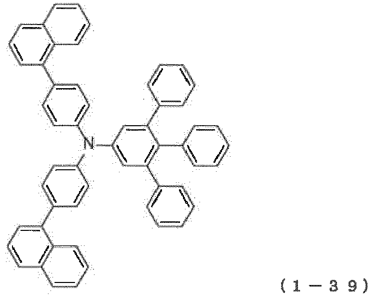

In connection with the resulting white powder, its structure was identified using NMR. In ¹H-NMR (CDC1₃), the following signals of 39 hydrogens were detected:

| | |
|---|---|
| δ (ppm) = | 8.09-8.11 (2H) |
| | 7.88-7.96 (4H) |
| | 7.41-7.60 (17H) |
| | 7.02-7.21 (12H) |
| | 6.89-6.93 (4H) |

### <Synthesis Example 5: Compound 1-40>

Synthesis of bis[4-(1-naphthalenyl)phenyl]-(1,1' :2',1":4",1'" :3' ,1""-quinquephenyl)-5'-amine:
Reactions were performed under the same conditions as in Synthesis Example 4, except that
4-biphenylboronic acid
   was used instead of
phenylboronic acid.
As a result, 9.9 g (yield 82%) of Compound 1-40 was obtained as a white powder.

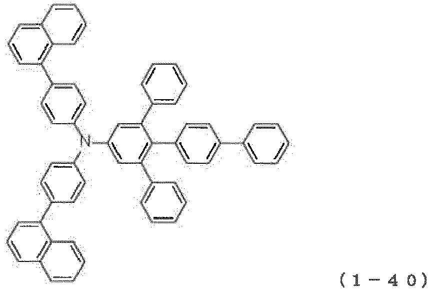

In connection with the resulting white powder, its structure was identified using NMR. In ¹H-NMR (CDC1₃), the following signals of 43 hydrogens were detected:

| | |
|---|---|
| δ (ppm) = | 8.09-8.11 (2H) |
| | 7.88-7.96 (4H) |
| | 7.14-7.60 (35H) |
| | 6.95-6.98 (2H) |

### <Synthesis Example 6: Compound 1-49>

Synthesis of bis[4-(2-naphthalenyl)phenyll-(1,1':2',1":31,1"'-quaterphenyl)-5'-amine:
Reactions were performed under the same conditions as in Synthesis Example 1, except that
bis[4-(2-naphthalenyl)phenyl]amine
   was used instead of
bis(biphenyl-4-yl)amine.
As a result, 4.4 g (yield 44%) of Compound 1-49 was obtained as a white powder.

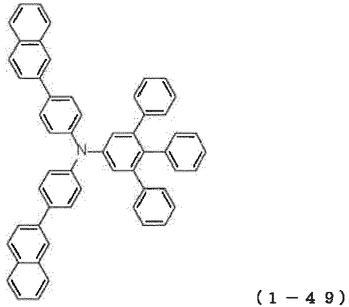

In connection with the resulting white powder, its structure was identified using NMR. In ¹H-NMR (CDC1₃), the following signals of 39 hydrogens were detected:

| | |
|---|---|
| δ (ppm) = | 8.08 (2H) |
| | 7.71-7.86 (12H) |
| | 7.32-7.56 (13H) |
| | 6.89-7.17 (16H) |

### <Synthesis Example 7: Compound 1-50>

Synthesis of bis[4-(2-naphthalenyl)phenyl]-(1,1":2',1":4",1"':3',1""-quinquephenyl)-5'-amine:
Reactions were performed under the same conditions as in Synthesis Example 6, except that
4-biphenylboronic acid
   was used instead of
phenylboronic acid.
As a result, 8.5 g (yield 70%) of Compound 1-50 was obtained as a white powder.

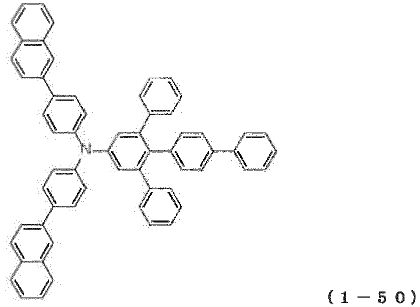

In connection with the resulting white powder, its structure was identified using NMR. In ¹H-NMR (CDC1₃), the following signals of 43 hydrogens were detected:

| | |
|---|---|
| δ (ppm) = | 8.09 (2H) |
| | 7.71-7.96 (11H) |
| | 7.31-7.57 (16H) |
| | 7.11-7.16 (10H) |
| | 6.94-6.97 (4H) |

### <Synthesis Example 8: Compound 1-69>

Synthesis of (biphenyl-4-yl)-(2'-phenyl-1,1':3',1"-terphenyl-5'-yl)-(9,9-dimethylfluoren-2-yl)amine:
Reactions were performed under the same conditions as in Synthesis Example 1, except that
(biphenyl-4-yl)-9,9-dimethylfluoren-2-amine
   was used instead of
bis(biphenyl-4-yl) amine.
As a result, 11.2 g (yield 64%) of Compound 1-69 was obtained as a white powder.

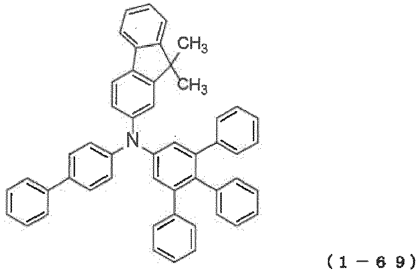

In connection with the resulting white powder, its structure was identified using NMR. In ¹H-NMR (CDC1₃), the following signals of 39 hydrogens were detected:

| | |
|---|---|
| δ (ppm) = | 7.53-7.69 (5H) |
| | 7.21-7.48 (14H) |
| | 6.96-7.12 (12H) |
| | 6.81-6.90 (2H) |
| | 1.51 (6H) |

### <Synthesis Example 9: Compound 1-75>

Synthesis of bis [9,9-dimethylfluoren-2-yl]-(1,1':2',1":3',1'"-quaterphenyl)-5'-amine:
Reactions were performed under the same conditions as in Synthesis Example 1, except that
bis(9, 9-dimethylfluoren-2-yl) amine
   was used instead of
bis(biphenyl-4-yl)amine.
As a result, 7.7 g (yield 77%) of Compound 1-75 was obtained as a white powder.

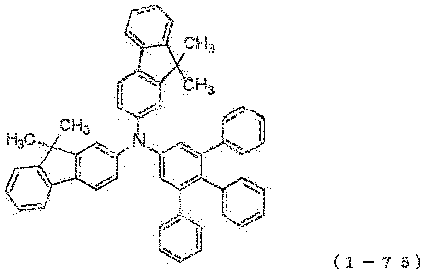

In connection with the resulting white powder, its structure was identified using NMR. In ¹H-NMR (CDC1₃) , the following signals of 43 hydrogens were detected:

| | |
|---|---|
| δ (ppm) = | 7.68-7.72 (4H) |
| | 7.28-7.47 (12H) |
| | 6.90-7.13 (15H) |
| | 1.53 (12H) |

### <Synthesis Example 10: Compound 1-81>

Synthesis of (biphenyl-4-yl)-(9,9-diphenylfluoren-2-yl)-(1,1' :2' ,1":3' ,1"'-quaterphenyl)-5'-amine:
Reactions were performed under the same conditions as in Synthesis Example 1, except that
(biphenyl-4-yl)-(9,9-diphenylfluoren-2-yl)amine
   was used instead of
bis(biphenyl-4-yl)amine.
As a result, 7.3 g (yield 56%) of Compound 1-81 was obtained as a white powder.

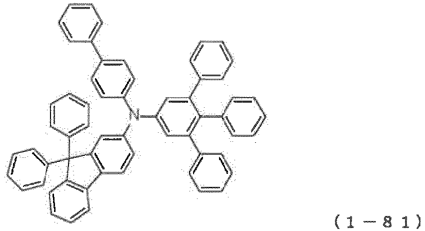

In connection with the resulting white powder, its structure was identified using NMR. In ¹H-NMR (CDCl₃), the following signals of 43 hydrogens were detected:

| | |
|---|---|
| δ (ppm) = | 7.70-7.75 (2H) |
| | 7.61-7.64 (2H) |
| | 7.15-7.54 (28H) |
| | 6.97-7.02 (7H) |
| | 6.85-6.87 (4H) |

### <Synthesis Example 11: Compound 1-90>

Synthesis of (biphenyl-4-yl)-(1,1':2',1":4",1"':3',1""-quinquephenyl)-5'-yl-9,9-spirobi[9H-fluoren]-2-amine:
Reactions were performed under the same conditions as in Synthesis Example 1, except that
(biphenyl-4-yl)-9,9-spirobi[9H-fluoren]-2-amine
   was used instead of
bis(biphenyl-4-yl)amine, and
4-biphenylboronic acid
   was used instead of
phenylboronic acid.
As a result, 10.8 g (yield 99%) of Compound 1-90 was obtained as a white powder.

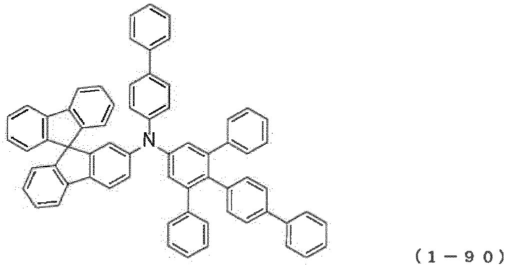

In connection with the resulting white powder, its structure was identified using NMR. In ¹H-NMR (CDC1₃), the following signals of 45 hydrogens were detected:

| | |
|---|---|
| δ (ppm) = | 7.77-7.87 (4H) |
| | 7.06-7.62 (30H) |
| | 6.81-6.92 (9H) |
| | 6.70-6.73 (2H) |

The glass transition points of the compounds obtained in the Synthesis Examples were measured by a high sensitivity differential scanning calorimeter (DSC3100SA, manufactured by Bruker AXS).

| | Glass transition point (°C) |
|---|---|
| Compound 1-9 of Synthesis Example 1 | 110 |
| Compound 1-10 of Synthesis Example 2 | 124 |
| Compound 1-13 of Synthesis Example 3 | 121 |
| Compound 1-39 of Synthesis Example 4 | 121 |
| Compound 1-40 of Synthesis Example 5 | 131 |
| Compound 1-49 of Synthesis Example 6 | 121 |
| Compound 1-50 of Synthesis Example 7 | 133 |
| Compound 1-69 of Synthesis Example 8 | 121 |
| Compound 1-75 of Synthesis Example 9 | 135 |
| Compound 1-81 of Synthesis Example 10 | 140 |
| Compound 1-90 of Synthesis Example 11 | 159 |

The arylamine compound I represented by the general formula (1) had a glass transition point of 100°C or higher, demonstrating that it was stable in a thin film state.

Using each of the compounds obtained in the Synthesis Examples, a vapor deposited film with a film thickness of 100 nm was prepared on an ITO substrate, and its work function was measured using an ionization potential measuring device (PYS-202, manufactured by Sumitomo Heavy Industries, Ltd.).

| | Work function (eV) |
|---|---|
| Compound 1-9 of Synthesis Example 1 | 5.71 |
| Compound 1-10 of Synthesis Example 2 | 5.72 |
| Compound 1-13 of Synthesis Example 3 | 5.73 |
| Compound 1-39 of Synthesis Example 4 | 5.75 |
| Compound 1-40 of Synthesis Example 5 | 5.75 |
| Compound 1-49 of Synthesis Example 6 | 5.69 |
| Compound 1-50 of Synthesis Example 7 | 5.69 |
| Compound 1-69 of Synthesis Example 8 | 5.62 |
| Compound 1-75 of Synthesis Example 9 | 5.58 |
| Compound 1-81 of Synthesis Example 10 | 5.69 |
| Compound 1-90 of Synthesis Example 11 | 5.66 |

The arylamine compound I represented by the general formula (1) showed a suitable energy level as compared with a work function of 5.4 eV which an ordinary hole transport material such as NPD or TPD has. Thus, this arylamine compound I was found to have satisfactory hole transport capability.

### <Synthesis Example 12: Compound 2-1>

Synthesis of 7,7-dimethyl-12-(4-phenylquinazolin-2-yl)-7,12-dihydrobenzo[4,5]thieno[3,2-g]indeno[1,2-b]indole:
A nitrogen-purged reaction vessel was charged with

| | | |
|---|---|---|
| | 7,7-dimethyl-7,12-dihydrobenzo[4,5]thieno[3,2-g]indeno[1,2-b]indole | 4.9 g, |
| | 2-chloro-4-phenylquinazoline | 5.7 g, |
| | tris(dibenzylideneacetone)dipalladium | 0.3 g, |
| | tri-tert-butylphosphonium tetrafluoroborate | 0.4 g, |
| | tert-butoxysodium | 4.0 g, |
| and | | |
| | xylene | 74 mL. |

The charge was heated, stirred under reflux for 12 hours to obtain a reaction liquid. The reaction liquid was cooled to room temperature, and then ethyl acetate and water were added. An organic layer was collected by a liquid separating operation. The organic layer collected was concentrated to obtain a crude product. The crude product was purified by column chromatography. As a result, 3.0 g (yield 38%) of Compound 2-1 was obtained as a powder.

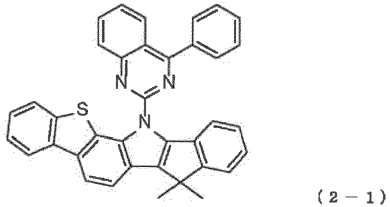

### <Synthesis Example 13: Compound 2-2>

Synthesis of 7,7-dimethyl-12-(4-phenylbenzo[h]quinazolin-2-yl)-7,12-dihydrobenzo[4,5]thieno[3,2-g]indeno[1,2-b]indole:
Reactions were performed under the same conditions as in Synthesis Example 12, except that
2-chloro-4-phenylbenzo[h]quinazoline
   was used instead of
2-chloro-4-phenylquinazoline.
As a result, 3.2 g (yield 38%) of Compound 2-2 was obtained as a powder.

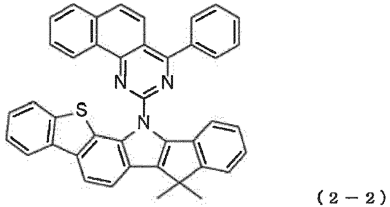

### <Synthesis Example 14: Compound 2-3>

Synthesis of 12-(4,7-diphenylquinazolin-2-yl)-7,7-dimethyl-7,12-dihydrobenzo[4,5]thieno[3,2-g]indeno[1,2-b]indole:
Reactions were performed under the same conditions as in Synthesis Example 12, except that
2-chloro-4,7-diphenylquinazoline
   was used instead of
2-chloro-4-phenylquinazoline.
As a result, 3.3 g (yield 38%) of Compound 2-3 was obtained as a powder.

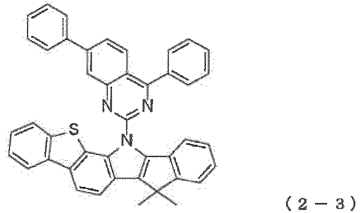

### <Synthesis Example 15: Compound 2-4>

Synthesis of 12-(4,6-diphenylquinazolin-2-yl)-7,7-dimethyl-7,12-dihydrobenzo[4,5]thieno[3,2-g]indeno[1,2-b]indole:
Reactions were performed under the same conditions as in Synthesis Example 12, except that
2-chloro-4,6-diphenylquinazoline
   was used instead of
2-chloro-4-phenylquinazoline.
As a result, 3.3 g (yield 38%) of Compound 2-4 was obtained as a powder.

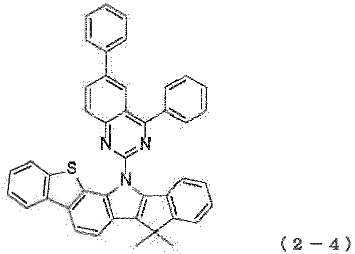

### <Synthesis Example 16: Compound 2-5>

Synthesis of 13,13-dimethyl-8-(4-phenylquinazolin-2-yl)-8,13-dihydrobenzo[4,5]thieno[3,2-e]indeno[1,2-b]indole:
Reactions were performed under the same conditions as in Synthesis Example 12, except that
13,13-dimethyl-8,13-dihydrobenzo[4,5]thieno[3,2-e]ind eno[1,2-
b]indole
   was used instead of
7,7-dimethyl-7,12-dihydrobenzo[4,5]thieno[3,2-g]inden o [1,2 -
b]indole.
As a result, 3.0 g (yield 38%) of Compound 2-5 was obtained as a powder.

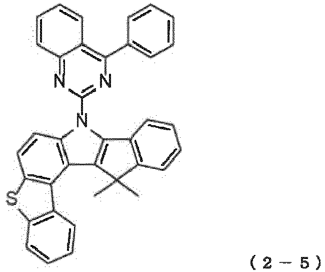

### <Synthesis Example 17: Compound 2-6>

Synthesis of 8-(4,6-diphenylquinazolin-2-yl)-13,13-dimethyl-8,13-dihydrobenzo[4,5]thieno[3,2-e]indeno[1,2-b]indole:
Reactions were performed under the same conditions as in Synthesis Example 16, except that
2-chloro-4,6-diphenylquinazoline
   was used instead of
2-chloro-4-phenylquinazoline.
As a result, 3.3 g (yield 38%) of Compound 2-6 was obtained as a powder.

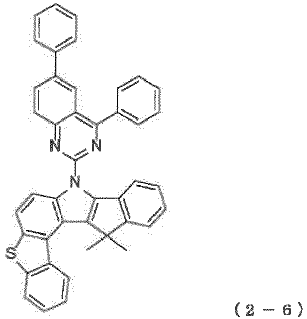

### <Synthesis Example 18: Compound 2-7>

Synthesis of 7,7,13,13-tetramethyl-5-(4-phenylquinazolin-2-yl)-7, 13-dihydro-5H-diindeno[1,2-b:1' ,2'-f]indole:
Reactions were performed under the same conditions as in Synthesis Example 12, except that
7,7,13,13-tetramethyl-7,13-dihydro-5H-diindeno[1,2-b:1',2'-f]indole
   was used instead of
7,7-dimethyl-7,12-dihydrobenzo[4,5]thieno[3,2-g]inden o[1,2 -
b]indole.
As a result, 3.0 g (yield 38%) of Compound 2-7 was obtained as a powder.

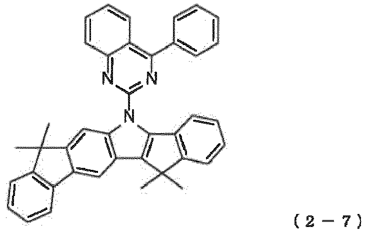

### <Synthesis Example 19: Compound 2-8>

Synthesis of 7,7,13,13-tetramethyl-5- [3-(4-phenylquinazolin-2-yl)phenyl]-7,13-dihydro-5H-diindeno[1,2-b:1',2'-f]indole: Reactions were performed under the same conditions as in Synthesis Example 18, except that
2-(3-bromophenyl)-4-phenylquinazoline
   was used instead of
2-chloro-4-phenylquinazoline.
As a result, 3.4 g (yield 38%) of Compound 2-8 was obtained as a powder.

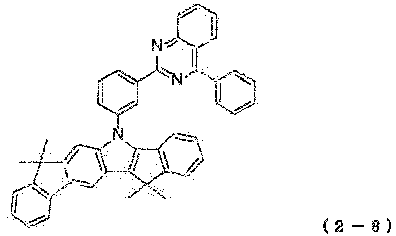

### <Synthesis Example 20: Compound 2-9>

Synthesis of 7,7-dimethyl-12-(4-phenylbenzo[h]quinazolin-2-yl)-7,12-dihydrobenzofuro[3,2-g]indeno[1,2-b]indole:
Reactions were performed under the same conditions as in Synthesis Example 13, except that
7,7-dimethyl-7,12-dihydrobenzofuro[3,2-g]indeno[1,2-b ] indole
   was used instead of
7,7-dimethyl-7,12-dihydrobenzo[4,5]thieno[3,2-g]inden o [1, 2 -
b]indole.
As a result, 3.0 g (yield 38%) of Compound 2-9 was obtained as a powder.

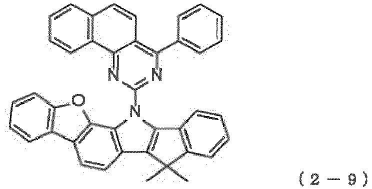

### <Synthesis Example 21: Compound 2-10>

Synthesis of 12-(4,6-diphenylbenzo[h]quinazolin-2-yl)-7,7-dimethyl-7,12-dihydrobenzofuro[3,2-g]indeno[1,2-b]indole:
Reactions were performed under the same conditions as in Synthesis Example 20, except that
2-chloro-4,6-diphenylbenzo[h]quinazoline
   was used instead of
2-chloro-4-phenylbenzo[h]quinazoline.
As a result, 3.5 g (yield 38%) of Compound 2-10 was obtained as a powder.

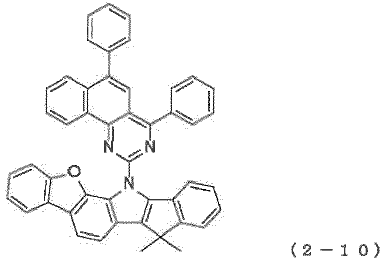

### <Synthesis Example 22: Compound 2-11>

Synthesis of 13,13-dimethyl-8-(4-phenylquinazolin-2-yl)-8,13-dihydrobenzofuro[3,2-e]indeno[1,2-b]indole:
Reactions were performed under the same conditions as in Synthesis Example 12, except that
13,13-dimethyl-8,13-dihydrobenzofuro[3,2-e]indeno[1,2 -b]indole
   was used instead of
7,7-dimethyl-7,12-dihydrobenzo[4,5]thieno[3,2-g]inden o[1,2-
b]indole.
As a result, 3.0 g (yield 38%) of Compound 2-11 was obtained as a powder.

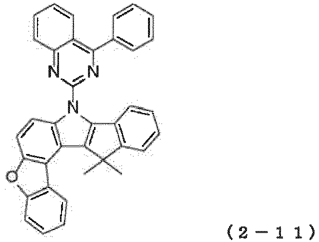

### <Synthesis Example 23: Compound 2-12>

Synthesis of 13,13-dimethyl-8-(4,6-diphenylquinazolin-2-yl)-8,13-dihydrobenzofuro[3,2-e]indeno[1,2-b]indole:
Reactions were performed under the same conditions as in Synthesis Example 22, except that
2-chloro-4,6-diphenylquinazoline
   was used instead of
2-chloro-4-phenylquinazoline.
As a result, 3.2 g (yield 38%) of Compound 2-12 was obtained as a powder.

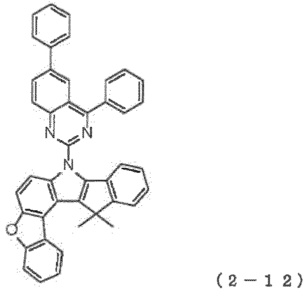

### <Synthesis Example 24: Compound 3-1>

Synthesis of 13-(4,6-diphenylquinazolin-2-yl)-7,7-dimethyl-7,13-dihydroindeno[2' ,1':4,5]thieno[2,3-a]carbazole:
Reactions were performed under the same conditions as in Synthesis Example 23, except that
7, 7-dimethyl-7,13-dihydroindeno[2' ,1' :4,5]thieno[2,3-a] carbazole
   was used instead of
13,13-dimethyl-8,13-dihydrobenzofuro[3,2-e]indeno[1,2 -b]indole.
As a result, 7.0 g (yield 38%) of Compound 3-1 was obtained as a powder.

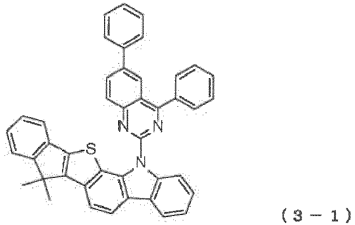

### <Synthesis Example 25: Compound 3-2>

Synthesis of 13-[4-(biphenyl-4-yl)quinazolin-2-yl] -7,7-dimethyl-7,13-dihydroindeno[2' ,1' :4,5]thieno[2,3-a]carbazole:
Reactions were performed under the same conditions as in Synthesis Example 24, except that
4-(biphenyl-4-yl)-2-chloroquinazoline
   was used instead of
2-chloro-4,6-diphenylquinazoline.
As a result, 6.7 g (yield 37%) of Compound 3-2 was obtained as a powder.

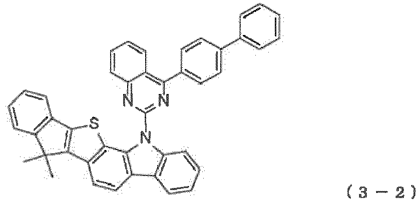

### <Synthesis Example 26: Compound 3-3>

Synthesis of 7,7-dimethyl-13-[4-(phenyl-d₅)quinazolin-2-yl]-7,13-dihydroindeno[2',1':4,5]thieno[2,3-a]carbazole:
Reactions were performed under the same conditions as in Synthesis Example 24, except that
2-chloro-4-(phenyl-d₅)quinazoline
   was used instead of
2-chloro-4,6-diphenylquinazoline.
As a result, 8.4 g (yield 32%) of Compound 3-3 was obtained as a powder.

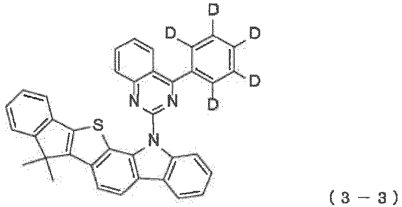

### <Synthesis Example 27: Compound 3-4>

Synthesis of 7,7-dimethyl-13- [4-(4-phenylquinazolin-2-yl)phenyl]-7,13-dihydroindeno[2',1':4,5]thieno[2,3-a] carbazole:
Reactions were performed under the same conditions as in Synthesis Example 24, except that
2-(4-bromophenyl)-4-phenylquinazoline
   was used instead of
2-chloro-4,6-diphenylquinazoline.
As a result, 5.2 g (yield 28%) of Compound 3-4 was obtained as a powder.

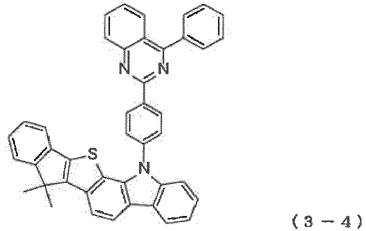

### <Synthesis Example 28: Compound 3-5>

Synthesis of 7,7-dimethyl-13-[3-(4-phenylquinazolin-2-yl)phenyl] - 7,13-dihydroindeno[2',1':4,5]thieno[2,3-a]carbazole:
Reactions were performed under the same conditions as in Synthesis Example 24, except that
2-(3-bromophenyl)-4-phenylquinazoline
   was used instead of
2-chloro-4,6-diphenylquinazoline.
As a result, 8.4 g (yield 32%) of Compound 3-5 was obtained as a powder.

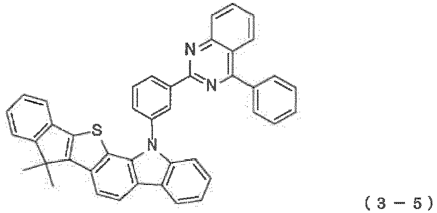

### <Synthesis Example 29: Compound 3-6>

Synthesis of 7,7-dimethyl-13-(4-phenylbenzo[h]quinazolin-2-yl)-7,13-dihydroindeno[2',1':4,5]thieno[2,3-a]carbazole:
Reactions were performed under the same conditions as in Synthesis Example 24, except that
2-chloro-4-phenylbenzo[h]quinazoline
   was used instead of
2-chloro-4,6-diphenylquinazoline.
As a result, 8.4 g (yield 32%) of Compound 3-6 was obtained as a powder.

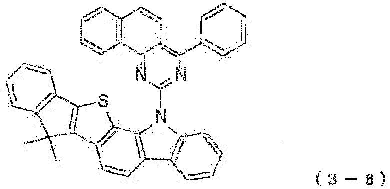

### <Synthesis Example 30: Compound 3-7>

Synthesis of 8,8-dimethyl-5-(4-phenylbenzo[h]quinazolin-2-yl)-5,8-dihydroindeno[2',1':4,5]thieno[3,2-c]carbazole:
Reactions were performed under the same conditions as in Synthesis Example 29, except that
8,8-dimethyl-5,8-dihydroindeno[2',1':4,5]thieno[3,2-c ] carbazole
   was used instead of
7,7-dimethyl-7,13-dihydroindeno[2',1':4,5]thieno[2,3-a] carbazole.
As a result, 9.3 g (yield 35%) of Compound 3-7 was obtained as a powder.

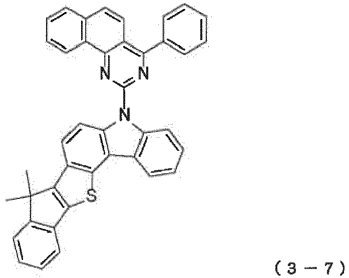

### <Synthesis Example 31: Compound 3-8>

Synthesis of 7,7-dimethyl-13-(4-phenylquinazolin-2-yl)-7,13-dihydroindeno[2',1':4,5]furo[2,3-a]carbazole:
Reactions were performed under the same conditions as in Synthesis Example 12, except that
7,7-dimethyl-7,13-dihydroindeno[2',1':4,5]furo[2,3-a] carbazole
   was used instead of
7,7-dimethyl-7,12-dihydrobenzo[4,5]thieno[3,2-g]indeno[1,2-b]indole.
As a result, 6.2 g (yield 32%) of Compound 3-8 was obtained as a powder.

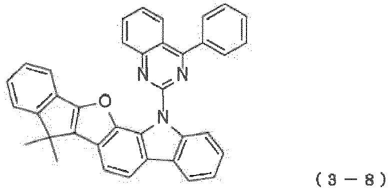

### <Synthesis Example 32: Compound 3-9>

Synthesis of 7,7-dimethyl-13-(4-phenylbenzo[h]quinazolin-2-yl)-7,13-dihydroindeno[2',1':4,5]furo[2,3-a]carbazole:
Reactions were performed under the same conditions as in Synthesis Example 31, except that
2-chloro-4-phenylbenzo[h]quinazoline
   was used instead of
2-chloro-4-phenylquinazoline.
As a result, 8.6 g (yield 30%) of Compound 3-9 was obtained as a powder.

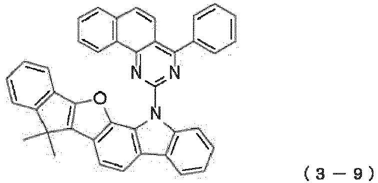

### <Synthesis Example 33: Compound 3-10>

Synthesis of 13-(4,6-diphenylquinazolin-2-yl)-7,7-dimethyl-7,13-dihydroindeno[2',1':4,5]furo[2,3-a]carbazole:
Reactions were performed under the same conditions as in Synthesis Example 31, except that
2-chloro-4,6-diphenylquinazoline
   was used instead of
2-chloro-4-phenylquinazoline.
As a result, 7.2 g (yield 29%) of Compound 3-10 was obtained as a powder.

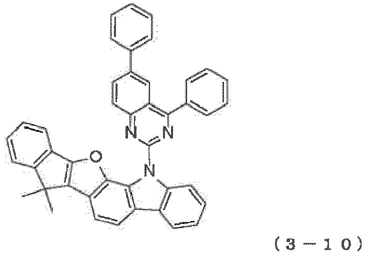

### <Synthesis Example 34: Compound 3-11>

Synthesis of 7,7-diphenyl-13-(4-phenylquinazolin-2-yl)-7,13-dihydroindeno[2',1' :4,5]thieno[2,3-a]carbazole:
Reactions were performed under the same conditions as in Synthesis Example 12, except that
7,7-diphenyl-7,13-dihydroindeno[2',1':4,5]thieno[2,3-a] carbazole
   was used instead of
7,7-dimethyl-7,12-dihydrobenzo[4,5]thieno[3,2-g]indeno[1,2-b]indole.
As a result, 6.7 g (yield 37%) of Compound 3-11 was obtained as a powder.

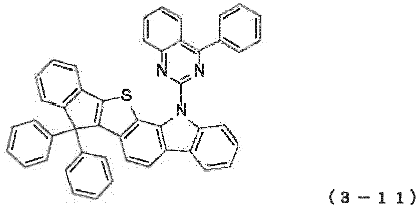

### <Synthesis Example 35: Compound 3-12>

Synthesis of 9,9-dimethyl-15-(4-phenylquinazolin-2-yl)-9,15-dihydrobenzo[a]indeno[2',1' :4,5]thieno[3,2-i]carbazole
Reactions were performed under the same conditions as in Synthesis Example 12, except that
9, 9-dimethyl-9,15-dihydrobenzo[a]indeno[2',1':4,5]thieno[3,2-i]carbazol e
   was used instead of
7,7-dimethyl-7,12-dihydrobenzo[4,5]thieno[3,2-g]indeno[1,2-b]indole.
As a result, 4.8 g (yield 42%) of Compound 3-12 was obtained as a powder.

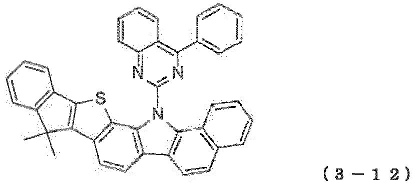

### <Synthesis Example 36: Compound 3-13>

Synthesis of 7-phenyl-13-(4-phenylquinazolin-2-yl)-7,13-dihydroindolo[2',3' :4,5]thieno[2,3-a]carbazole:
Reactions were performed under the same conditions as in Synthesis Example 12, except that
7-phenyl-7,13-dihydroindolo[2' ,3' :4,5]thieno[2,3-a]ca rbazole
   was used instead of
7,7-dimethyl-7,12-dihydrobenzo[4,5]thieno[3,2-g]indeno[1,2-b]indole.
As a result, 4.3 g (yield 43%) of Compound 3-13 was obtained as a powder.

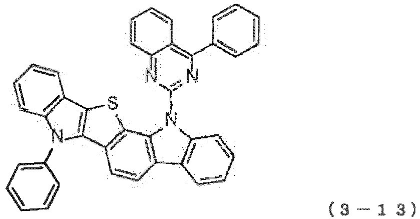

### <Synthesis Example 37: Compound 3-14>

Synthesis of 12,12-dimethyl-1- (4-phenylquinazolin-2-yl)-1,12-dihydroindeno[1',2':4,5]thieno[2,3-a]carbazole:
Reactions were performed under the same conditions as in Synthesis Example 12, except that
12,12-dimethyl-1,12-dihydroindeno[1',2':4,5]thieno[2,3-a]carbazole
   was used instead of
7,7-dimethyl-7, 12-dihydrobenzo[4,5]thieno[3,2-g]indeno[1,2-b]indole.
As a result, 6.3 g (yield 44%) of Compound 3-14 was obtained as a powder.

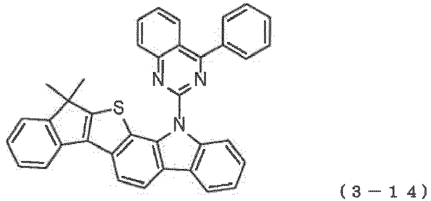

### <Synthesis Example 38: Compound 3-15>

Synthesis of 7,7-dimethyl-13-(naphthalen-2-yl)-7,13-dihydroindeno[2',1':4,5]thieno[2,3-a]carbazole:
Reactions were performed under the same conditions as in Synthesis Example 24, except that
2-bromonaphthalene
   was used instead of
2-chloro-4,6-diphenylquinazoline.
As a result, 5.4 g (yield 47%) of Compound 3-15 was obtained as a powder.

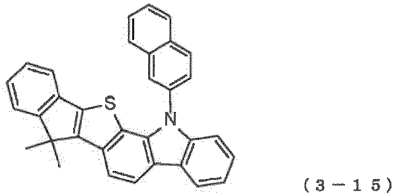

### <Device Example 1>

An organic EL device was prepared, as shown in Fig. 1, by vapor-depositing the hole injection layer 3, the first hole transport layer 4a, the second hole transport layer 4b, the luminous layer 5, the electron transport layer 6, the electron injection layer 7, and the cathode (aluminum electrode) 8 in this order on an ITO electrode formed beforehand as the transparent anode 2 on the glass substrate 1.

Concretely, the glass substrate 1 having a 150 nm thick ITO film formed thereon was ultrasonically cleaned in isopropyl alcohol for 20 minutes, and dried for 10 minutes on a hot plate heated at 200°C. Then, the glass substrate with the ITO film was treated with UV ozone for 15 minutes, and mounted within a vacuum deposition machine. Then, the pressure inside the vacuum deposition machine was reduced to 0.001 Pa or lower.

Then, HIM-1 of the following structural formula was vapor-deposited so as to cover the transparent anode 2, whereby the hole injection layer 3 with a film thickness of 5 nm was formed.

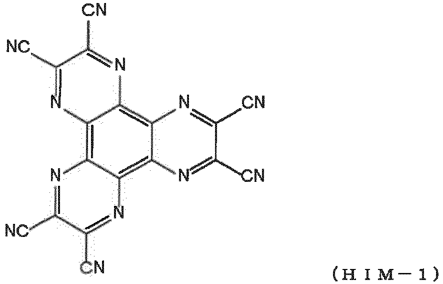

A triarylamine compound 5-1 of the following structural formula was vapor-deposited on the hole injection layer 3 to form the first hole transport layer 4a with a film thickness of 60 nm.

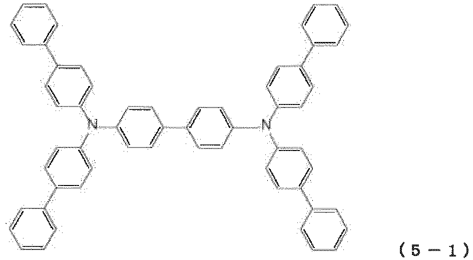

Compound 1-69 of Synthesis Example 8 was vapor-deposited on the first hole transport layer 4a to form the second hole transport layer 4b with a film thickness of 5 nm.

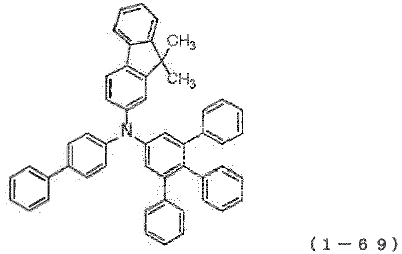

A compound EMD-1 and Compound 2-2 of Synthesis Example 13, represented by the following structural formulas, were binary vapor deposited on the second hole transport layer 4b at such vapor deposition rates that EMD-1:Compound 2-2=5:95, whereby the luminous layer 5 with a film thickness of 20 nm was formed.

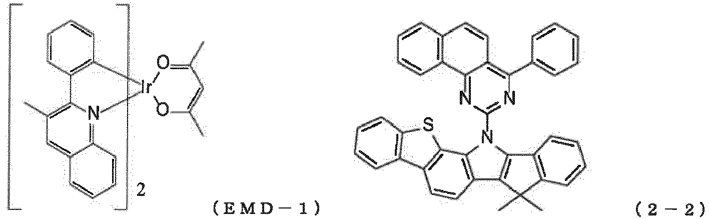

An anthracene derivative 4a-1 and ETM-1, of the following structural formulas, were binary vapor deposited on the luminous layer 5 at such vapor deposition rates that the vapor deposition rate ratio would be derivative 4a-1:ETM-1=50:50, whereby the electron transport layer 6 with a film thickness of 30 nm was formed.

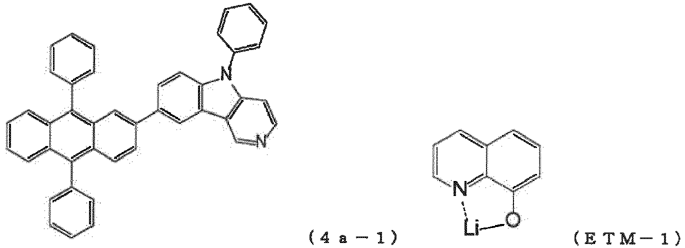

Lithium fluoride was vapor deposited on the electron transport layer 6 to form the electron injection layer 7 with a film thickness of 1 nm.

Finally, aluminum was vapor deposited to a film thickness of 100 nm to form the cathode 8.

### <Device Example 2>

An organic EL device was prepared under the same conditions as in Device Example 1, except that Carbazole Compound 3-16 of the following structural formula was used, instead of Compound 2-2 of Synthesis Example 13, as the material for the luminous layer 5, and that EMD-1 and Compound 3-16 were binary vapor deposited at such vapor deposition rates that the vapor deposition rate ratio would be EMD-1:Compound 3-16=5:95.

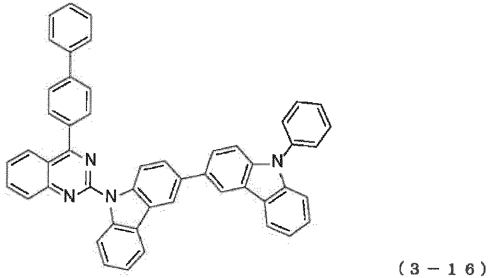

### <Device Example 3>

An organic EL device was prepared under the same conditions as in Device Example 1, except that Compound 1-75 of Synthesis Example 9 was used, instead of Compound 1-69 of Synthesis Example 8, as the material for the second hole transport layer 4b.

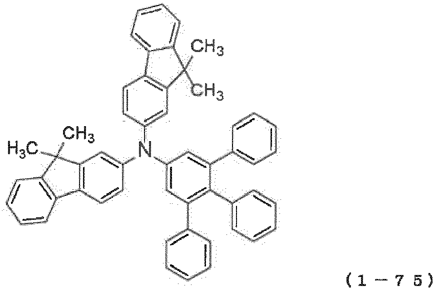

### <Device Example 4>

An organic EL device was prepared under the same conditions as in Device Example 2, except that Compound 1-75 of Synthesis Example 9 was used, instead of Compound 1-69 of Synthesis Example 8, as the material for the second hole transport layer 4b.

### <Device Example 5>

An organic EL device was prepared under the same conditions as in Device Example 1, except that Compound 1-81 of Synthesis Example 10 was used, instead of Compound 1-69 of Synthesis Example 8, as the material for the second hole transport layer 4b.

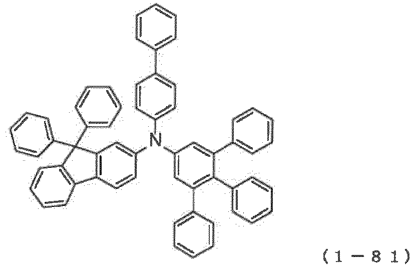

### <Device Example 6>

An organic EL device was prepared under the same conditions as in Device Example 2, except that Compound 1-81 of Synthesis Example 10 was used, instead of Compound 1-69 of Synthesis Example 8, as the material for the second hole transport layer 4b.

### <Device Example 7>

An organic EL device was prepared under the same conditions as in Device Example 1, except that Compound 1-90 of Synthesis Example 11 was used, instead of Compound 1-69 of Synthesis Example 8, as the material for the second hole transport layer 4b.

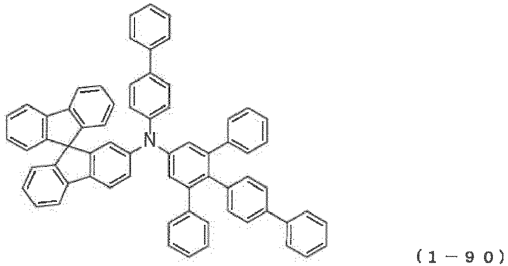

### <Device Example 8>

An organic EL device was prepared under the same conditions as in Device Example 2, except that Compound 1-90 of Synthesis Example 11 was used, instead of Compound 1-69 of Synthesis Example 8, as the material for the second hole transport layer 4b.

### <Device Comparative Example 1>

An organic EL device was prepared under the same conditions as in Device Example 1, except that Compound 5'-2 of the following structural formula was used, instead of Compound 5-1, as the material for the first hole transport layer 4a, and that Compound 5'-2 of the following structural formula was used, instead of Compound 1-69 of Synthesis Example 8, as the material for the second hole transport layer 4b. In this case, the first hole transport layer 4a and the second hole transport layer 4b function as the integral hole transport layer 4 (film thickness 65 nm).

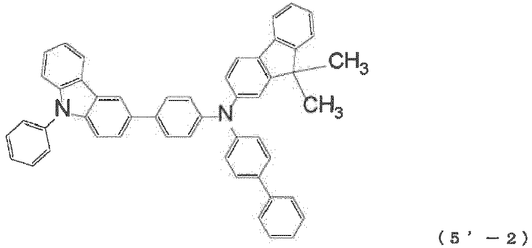

### <Device Comparative Example 2>

An organic EL device was prepared under the same conditions as in Device Example 2, except that Compound 5'-2 was used, instead of Compound 5-1, as the material for the first hole transport layer 4a, and that Compound 5'-2 was used, instead of Compound 1-69 of Synthesis Example 8, as the material for the second hole transport layer 4b. In this case, the first hole transport layer 4a and the second hole transport layer 4b function as the integral hole transport layer 4 (film thickness 65 nm).

### <Device Comparative Example 3>

An organic EL device was prepared under the same conditions as in Device Example 1, except that Compound HTM-1 of the following structural formula was used, instead of Compound 1-69 of Synthesis Example 8, as the material for the second hole transport layer 4b.

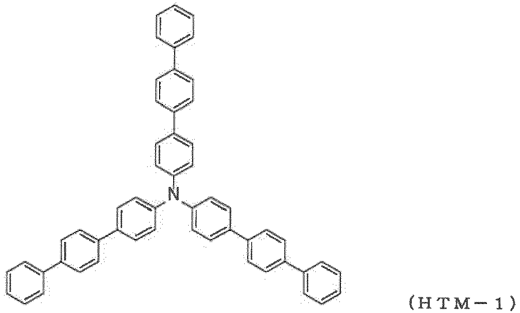

### <Device Comparative Example 4>

An organic EL device was prepared under the same conditions as in Device Example 2, except that Compound HTM-1 was used, instead of Compound 1-69 of Synthesis Example 8, as the material for the second hole transport layer 4b.

Each of the organic EL devices prepared in Device Examples 1 to 8 and Device Comparative Examples 1 to 4 was measured for the light emission characteristics when a direct current voltage was applied at normal temperature in the atmosphere. The results of the measurements are shown in Table 1.

Each of the organic EL devices prepared in Device Examples 1 to 8 and Device Comparative Examples 1 to 4 was measured for the device lifetime. Concretely, the device lifetime was measured as the period of time until the emission luminance attenuated to 6790 cd/m² (corresponding to 97%, with the initial luminance taken as 100%: 97% attenuation) when constant current driving was performed, with the emission luminance at the start of light emission (initial luminance) being set at 7000 cd/m². The results are shown in Table 1.

**Table 1**

| | First hole transport layer | Second hole transport layer | Luminous layer | Electron transport layer | Voltage [V] (@10mA/ cm²) | Luminance [cd/m²] (@10mA/ cm²) | Luminous efficiency [cd/A] (@10mA/cm²) | Power efficiency [lm/W] (@10mA/cm²) | Device lifetime (hrs) 97% atten. |
|---|---|---|---|---|---|---|---|---|---|
| Device Ex. 1 | Comp. 5-1 | Comp. 1-69 | EMD-1/ Comp.2-2 | Comp. 4a-1/ ETM-1 | 3.73 | 2677 | 26.74 | 22.52 | 145 |
| Device Ex. 2 | Comp. 5-1 | Comp. 1-69 | EMD-1/ Comp.3-16 | Comp. 4a-1/ ETM-1 | 4.10 | 2611 | 26.11 | 20.01 | 320 |
| Device Ex. 3 | Comp. 5-1 | Comp. 1-75 | EMD-1/ Comp.2-2 | Comp. 4a-1/ ETM-1 | 3.53 | 2713 | 27.10 | 24.12 | 110 |
| Device Ex. 4 | Comp. 5-1 | Comp. 1-75 | EMD-1/ Comp.3-16 | Comp. 4a-1/ ETM-1 | 4.03 | 2655 | 26.58 | 20.72 | 250 |
| Device Ex. 5 | Comp. 5-1 | Comp. 1-81 | EMD-1/ Comp.2-2 | Comp. 4a-1/ ETM-1 | 3.90 | 2885 | 28.82 | 23.22 | 200 |
| Device Ex. 6 | Comp. 5-1 | Comp. 1-81 | EMD-1/ Comp.3-16 | Comp. 4a-1/ ETM-1 | 4.20 | 2809 | 28.12 | 21.04 | 385 |
| Device Ex. 7 | Comp. 5-1 | Comp. 1-90 | EMD-1/ Comp.2-2 | Comp. 4a-1/ ETM-1 | 3.85 | 2829 | 28.31 | 23.10 | 168 |
| Device Ex. 8 | Comp. 5-1 | Comp. 1-90 | EMD-1/ Comp.3-16 | Comp. 4a-1/ ETM-1 | 4.18 | 2781 | 27.68 | 20.81 | 329 |
| Device Comp. Ex. 1 | Comp. 5'-2 | Comp. 5'-2 | EMD-1/ Comp.2-2 | Comp. 4a-1/ ETM-1 | 3.95 | 2222 | 22.22 | 17.67 | 50 |
| Device Comp. Ex. 2 | Comp. 5'-2 | Comp. 5'-2 | EMD-1/ Comp.3-16 | Comp. 4a-1/ ETM-1 | 3.79 | 2033 | 20.34 | 16.86 | 32 |
| Device Comp. Ex. 3 | Comp. 5-1 | HTM-1 | EMD-1/ Comp.2-2 | Comp. 4a-1/ ETM-1 | 4.33 | 2598 | 26.01 | 18.82 | 61 |
| Device Comp. Ex. 4 | Comp. 5-1 | HTM-1 | EMD-1/ Comp.3-16 | Comp. 4a-1/ ETM-1 | 4.55 | 2517 | 25.19 | 17.77 | 46 |

| | | | | | | | | | |
|---|---|---|---|---|---|---|---|---|---|
| Ex. = Example, Comp. = Compound, atten. = attenuation Comp. Ex. = Comparative Example, Comp. = Compound, atten. = attenuation | | | | | | | | | |

The luminous efficiency upon passage of a current at a current density of 10 mA/cm² was 20.34 to 26.01 cd/A in Device Comparative Examples 1 to 4, while it was 26.11 to 28.82 cd/A in Device Examples 1 to 8, demonstrating higher efficiencies.

The power efficiency was 16.86 to 18.82 lm/W in Device Comparative Examples 1 to 4, while it was 20.01 to 24.12 lm/W in Device Examples 1 to 8, showing higher efficiencies.

The device lifetime was 32 to 61 hours in Device Comparative Examples 1 to 4, while it was 110 to 385 hours in Device Examples 1 to 8, demonstrating much longer lifetimes.

As evident from the above results, the organic EL device using the arylamine compound I represented by the general formula (1) and the indenoindole compound II represented by the general formula (2) or the carbazole compound represented by the general formula (3) is improved in the carrier balance within the device, and uses the materials suitable for the characteristics of luminescent materials. Hence, the organic EL device of the present invention has succeeded in proving to be an organic EL device having a higher luminous efficiency, a lower driving voltage, and a longer lifetime than known organic EL devices.

### Industrial Applicability

The organic EL device of the present invention, as described above, can attain a high luminous efficiency and a high power efficiency, can lower a practical driving voltage, and can improve durability. Thus, this organic EL device can be put to uses such as domestic electrical appliances and illumination.

### Explanations of Letters or Numerals

1 glass substrate, 2 transparent anode, 3 hole injection layer, 4 hole transport layer, 4a first hole transport layer, 4b second hole transport layer, 5 luminous layer, 6 electron transport layer, 7 electron injection layer, 8 cathode.

## Claims

1. An organic electroluminescent device having at least an anode (2), a hole transport layer (4a, 4b) , a luminous layer (5), an electron transport layer (6), and a cathode (8) in this order, wherein
the hole transport layer contains an arylamine compound represented by the following general formula (1), and
the luminous layer contains an indenoindole compound represented by the following general formula (2) or a carbazole compound represented by the following general formula (3): 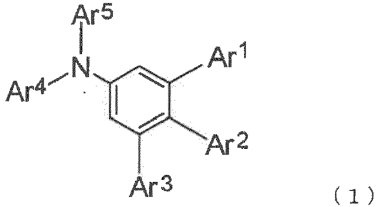 where
Ar¹ to Ar⁵ may be identical or different, and each represents an aromatic hydrocarbon group, an aromatic heterocyclic group, or a condensed polycyclic aromatic group. 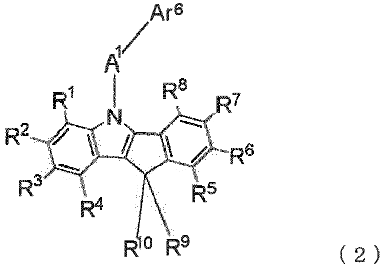 where
A¹ represents a divalent group of an aromatic hydrocarbon, a divalent group of an aromatic heterocycle, a divalent group of a condensed polycyclic aromatic, or a single bond,
Ar⁶ represents an aromatic hydrocarbon group, an aromatic heterocyclic group, or a condensed polycyclic aromatic group,
R¹ to R⁸ may be identical or different, and each represents a hydrogen atom; a deuterium atom; a fluorine atom; a chlorine atom; a cyano group; a nitro group; an alkyl group having 1 to 6 carbon atoms; a cycloalkyl group having 5 to 10 carbon atoms; an alkenyl group having 2 to 6 carbon atoms; an alkyloxy group having 1 to 6 carbon atoms; a cycloalkyloxy group having 5 to 10 carbon atoms; an aromatic hydrocarbon group; an aromatic heterocyclic group; a condensed polycyclic aromatic group; an aryloxyl group; or a di-substituted amino group substituted by a group selected from an aromatic hydrocarbon group, an aromatic heterocyclic group, and a condensed polycyclic aromatic group; R¹ to R⁴ may bind to each other via a single bond, a substituted or unsubstituted methylene group, an oxygen atom, or a sulfur atom to form a ring; R⁵ to R⁸ may bind to each other via a single bond, a substituted or unsubstituted methylene group, an oxygen atom, or a sulfur atom to form a ring; furthermore, to a vacant site occurring upon elimination of some of R¹ to R⁴ from a benzene ring, the other group of R¹ to R⁴ may bind via a substituted or unsubstituted methylene group, an oxygen atom, a sulfur atom, or a monoarylamino group to form a ring; and to a vacant site occurring upon elimination of some of R⁵ to R⁸ from a benzene ring, the other group of R⁵ to R⁸ may bind via a substituted or unsubstituted methylene group, an oxygen atom, a sulfur atom, or a monoarylamino group to form a ring, and
R⁹ and R¹⁰ may be identical or different, and each represents an alkyl group having 1 to 6 carbon atoms, an aromatic hydrocarbon group, an aromatic heterocyclic group, or a condensed polycyclic aromatic group, and may bind to each other via a single bond, a substituted or unsubstituted methylene group, an oxygen atom, or a sulfur atom to form a ring. 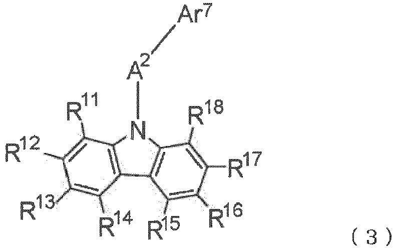 where
A² represents a divalent group of an aromatic hydrocarbon, a divalent group of an aromatic heterocycle, a divalent group of a condensed polycyclic aromatic, or a single bond,
Ar⁷ represents an aromatic hydrocarbon group, an aromatic heterocyclic group, or a condensed polycyclic aromatic group,
R¹¹ to R¹⁸ may be identical or different, and each represents a hydrogen atom; a deuterium atom; a fluorine atom; a chlorine atom; a cyano group; a nitro group; an alkyl group having 1 to 6 carbon atoms; a cycloalkyl group having 5 to 10 carbon atoms; an alkenyl group having 2 to 6 carbon atoms; an alkyloxy group having 1 to 6 carbon atoms; a cycloalkyloxy group having 5 to 10 carbon atoms; an aromatic hydrocarbon group; an aromatic heterocyclic group; a condensed polycyclic aromatic group; an aryloxyl group; or a di-substituted amino group substituted by a group selected from an aromatic hydrocarbon group, an aromatic heterocyclic group, and a condensed polycyclic aromatic group; R¹¹ to R¹⁴ may bind to each other via a single bond, a substituted or unsubstituted methylene group, an oxygen atom, or a sulfur atom to form a ring; R¹⁵ to R¹⁸ may bind to each other via a single bond, a substituted or unsubstituted methylene group, an oxygen atom, or a sulfur atom to form a ring; furthermore, to a vacant site occurring upon elimination of some of R¹¹ to R¹⁴ from a benzene ring, the other group of R¹¹ to R¹⁴ may bind via a substituted or unsubstituted methylene group, an oxygen atom, a sulfur atom, or a monoarylamino group to form a ring; and to a vacant site occurring upon elimination of some of R¹⁵ to R¹⁸ from a benzene ring, the other group of R¹⁵ to R¹⁸ may bind via a substituted or unsubstituted methylene group, an oxygen atom, a sulfur atom, or a monoarylamino group to form a ring.

2. The organic electroluminescent device according to claim 1, wherein
the electron transport layer contains an anthracene derivative represented by the following general formula (4): 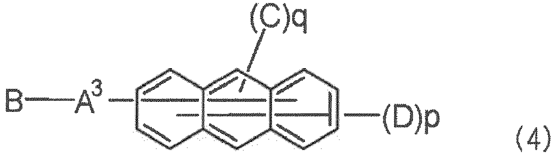 where
A³ represents a divalent group of an aromatic hydrocarbon, a divalent group of an aromatic heterocycle, a divalent group of a condensed polycyclic aromatic, or a single bond,
B represents an aromatic heterocyclic group,
C represents an aromatic hydrocarbon group, an aromatic heterocyclic group, or a condensed polycyclic aromatic group and, if there are two of Cs, the two Cs may be identical or different,
Ds may be identical or different, and each represents a hydrogen atom, a deuterium atom, a fluorine atom, a chlorine atom, a cyano group, a trifluoromethyl group, an alkyl group having 1 to 6 carbon atoms, an aromatic hydrocarbon group, an aromatic heterocyclic group, or a condensed polycyclic aromatic group, and
provided that a sum of p and q is 9, p denotes 7 or 8, while q denotes 1 or 2.

3. The organic electroluminescent device according to claim 2, wherein
the anthracene derivative is represented by the following general formula (4a): 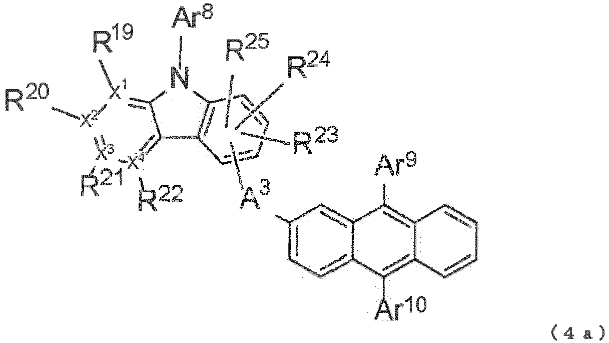 where
A³ has a meaning as defined in the general formula (4),
Ar⁸ to Ar¹⁰ may be identical or different, and each represents an aromatic hydrocarbon group, an aromatic heterocyclic group, or a condensed polycyclic aromatic group,
R¹⁹ to R²⁵ may be identical or different, and each represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group, and may bind to each other via a single bond, a substituted or unsubstituted methylene group, an oxygen atom, or a sulfur atom to form a ring, and
X¹ to X⁴ may be identical or different, and each represents a carbon atom or a nitrogen atom, and only one of X¹ to X⁴ is a nitrogen atom with the proviso that the nitrogen atom in this case does not have the hydrogen atom or substituent of R¹⁹ to R²².

4. The organic electroluminescent device according to claim 2, wherein
the anthracene derivative is represented by the following general formula (4b): 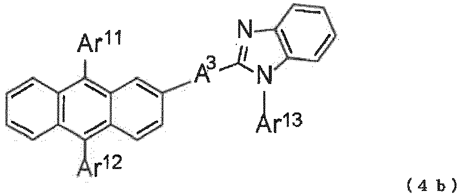 where
A³ has a meaning as defined in the general formula (4), and
Ar¹¹ to Ar¹³ may be identical or different, and each represents an aromatic hydrocarbon group, an aromatic heterocyclic group, or a condensed polycyclic aromatic group.

5. The organic electroluminescent device according to claim 2, wherein
the anthracene derivative is represented by the following general formula (4c): 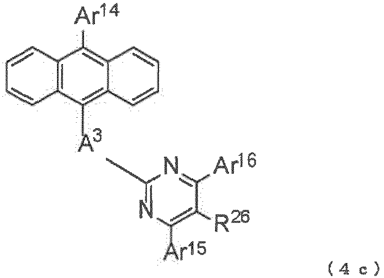 where
A³ has a meaning as defined in the general formula (4),
Ar¹⁴ to Ar¹⁶ may be identical or different, and each represents an aromatic hydrocarbon group, an aromatic heterocyclic group, or a condensed polycyclic aromatic group, and
R²⁶ represents a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group, or an aryloxyl group.

6. The organic electroluminescent device according to claim 1, wherein
the hole transport layer has a two-layer structure composed of a first hole transport layer (4a) and a second hole transport layer (4b), and the second hole transport layer is located beside the luminous layer and contains the arylamine compound represented by the general formula (1).

7. The organic electroluminescent device according to claim 1, wherein
the luminous layer contains a red light emitting material.

8. The organic electroluminescent device according to claim 1, wherein
the luminous layer contains a phosphorescence-emitting material.

9. The organic electroluminescent device according to claim 8, wherein
the phosphorescence-emitting material is a metal complex containing iridium or platinum.

## Patentansprüche

1. Organische Elektrolumineszenzvorrichtung, aufweisend zumindest eine Anode (2), eine Lochtransportschicht (4a,4b), eine Leuchtschicht (5), eine Elektrontransportschicht (6) und eine Kathode (8) in dieser Reihenfolge, wobei
die Lochtransportschicht eine Arylaminverbindung, dargestellt durch die nachstehende allgemeine Formel (1), enthält und
die Leuchtschicht eine Indenoindolverbindung, dargestellt durch die nachstehende allgemeine Formel (2), oder eine Carbazolverbindung, dargestellt durch die nachstehende allgemeine Formel (3), enthält: 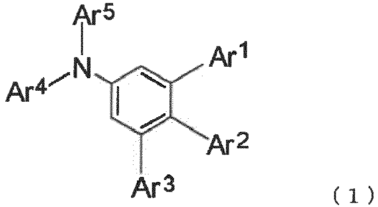 worin
Ar¹ bis Ar⁵ gleich oder verschieden sein können und jeweils eine aromatische Kohlenwasserstoffgruppe, eine aromatische heterocyclische Gruppe oder eine kondensierte polycyclische aromatische Gruppe darstellen. 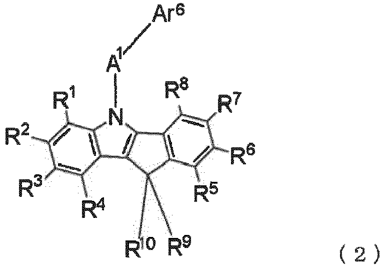 worin
A¹ eine zweiwertige Gruppe eines aromatischen Kohlenwasserstoffs, eine zweiwertige Gruppe eines aromatischen Heterocyclus, eine zweiwertige Gruppe eines kondensierten polycyclischen Aromaten oder eine Einfachbindung darstellt,
Ar⁶ eine aromatische Kohlenwasserstoffgruppe, eine aromatische heterocyclische Gruppe oder eine kondensierte polycyclische aromatische Gruppe darstellt,
R¹ bis R⁸ gleich oder verschieden sein können und jeweils ein Wasserstoffatom; ein Deuteriumatom; ein Fluoratom; ein Chloratom; eine Cyanogruppe; eine Nitrogruppe; eine Alkylgruppe mit 1 bis 6 Kohlenstoffatomen; eine Cycloalkylgruppe mit 5 bis 10 Kohlenstoffatomen; eine Alkenylgruppe mit 2 bis 6 Kohlenstoffatomen; eine Alkyloxygruppe mit 1 bis 6 Kohlenstoffatomen; eine Cycloalkyloxygruppe mit 5 bis 10 Kohlenstoffatomen; eine aromatische Kohlenwasserstoffgruppe; eine aromatische heterocyclische Gruppe; eine kondensierte polycyclische aromatische Gruppe; eine Aryloxygruppe; oder eine disubstituierte Aminogruppe, welche mit einer Gruppe, ausgewählt aus einer aromatischen Kohlenwasserstoffgruppe, einer aromatischen heterocyclischen Gruppe und einer kondensierten polycyclischen aromatischen Gruppe, substituiert ist, darstellen; R¹ bis R⁴ aneinander über eine Einfachbindung, eine substituierte oder unsubstituierte Methylengruppe, ein Sauerstoffatom oder ein Schwefelatom binden können, um einen Ring zu bilden; R⁵ bis R⁸ aneinander über eine Einfachbindung, eine substituierte oder unsubstituierte Methylengruppe, ein Sauerstoffatom oder ein Schwefelatom binden können, um einen Ring zu bilden; weiterhin an eine frei Stelle, welche bei Eliminierung eines Teils von R¹ bis R⁴ von einem Benzolring auftritt, die weitere Gruppe von R¹ bis R⁴ über eine substituierte oder unsubstituierte Methylengruppe, ein Sauerstoffatom, ein Schwefelatom oder eine Monoarylaminogruppe binden kann, um einen Ring zu bilden; und an freie Stelle, welche bei Eliminierung eines Teils von R⁵ bis R⁸ von einem Benzolring auftritt, die weitere Gruppe von R⁵ bis R⁸ über eine substituierte oder unsubstituierte Methylengruppe, ein Sauerstoffatom, ein Schwefelatom oder eine Monoarylaminogruppe binden kann, um einen Ring zu bilden, und
R⁹ und R¹⁰ gleich oder verschieden sein können und jeweils eine Alkylgruppe mit 1 bis 6 Kohlenstoffatomen, eine aromatische Kohlenwasserstoffgruppe, eine aromatische heterocyclische Gruppe oder eine kondensierte polycyclische aromatische Gruppe darstellen und aneinander über eine Einfachbindung, eine substituierte oder unsubstituierte Methylengruppe, ein Sauerstoffatom oder ein Schwefelatom binden können, um einen Ring zu bilden. 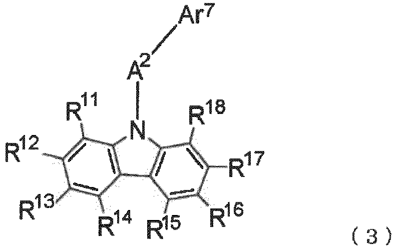 worin
A² eine zweiwertige Gruppe eines aromatischen Kohlenwasserstoffs, eine zweiwertige Gruppe eines aromatischen Heterocyclus, eine zweiwertige Gruppe eines kondensierten polycyclischen Aromaten oder eine Einfachbindung darstellt,
Ar⁷ eine aromatische Kohlenwasserstoffgruppe, eine aromatische heterocyclische Gruppe oder eine kondensierte polycyclische aromatische Gruppe darstellt,
R¹¹ bis R¹⁸ gleich oder verschieden sein können und jeweils ein Wasserstoffatom; ein Deuteriumatom; ein Fluoratom; ein Chloratom; eine Cyanogruppe; eine Nitrogruppe; eine Alkylgruppe mit 1 bis 6 Kohlenstoffatomen; eine Cycloalkylgruppe mit 5 bis 10 Kohlenstoffatomen; eine Alkenylgruppe mit 2 bis 6 Kohlenstoffatomen; eine Alkyloxygruppe mit 1 bis 6 Kohlenstoffatomen; eine Cycloalkyloxygruppe mit 5 bis 10 Kohlenstoffatomen; eine aromatische Kohlenwasserstoffgruppe; eine aromatische heterocyclische Gruppe; eine kondensierte polycyclische aromatische Gruppe; eine Aryloxygruppe; oder eine disubstituierte Aminogruppe, welche mit einer Gruppe, ausgewählt aus einer aromatischen Kohlenwasserstoffgruppe, einer aromatischen heterocyclischen Gruppe und einer kondensierten polycyclischen aromatischen Gruppe, substituiert ist, darstellen;
R¹¹ bis R¹⁴ aneinander über eine Einfachbindung, eine substituierte oder unsubstituierte Methylengruppe, ein Sauerstoffatom oder ein Schwefelatom binden können, um einen Ring zu bilden; R¹⁵ bis R¹⁸ aneinander über eine Einfachbindung, eine substituierte oder unsubstituierte Methylengruppe, ein Sauerstoffatom oder ein Schwefelatom binden können, um einen Ring zu bilden; weiterhin an eine freie Stelle, welche bei Eliminierung eines Teils von R¹¹ bis R¹⁴ von einem Benzolring auftritt, die weitere Gruppe von R¹¹ bis R¹⁴ über eine substituierte oder unsubstituierte Methylengruppe, ein Sauerstoffatom, ein Schwefelatom oder eine Monoarylaminogruppe binden kann, um einen Ring zu bilden; und an eine freie Stelle, welche bei Eliminierung eines Teils von R¹⁵ bis R¹⁸ von einem Benzolring auftritt, die weitere Gruppe von R¹⁵ bis R¹⁸ über eine substituierte oder unsubstituierte Methylengruppe, ein Sauerstoffatom, ein Schwefelatom oder eine Monoarylaminogruppe binden kann, um einen Ring zu bilden.

2. Organische Elektrolumineszenzvorrichtung nach Anspruch 1, wobei
die Elektrontransportschicht ein Anthracenderivat, dargestellt durch die nachstehende allgemeine Formel (4), enthält: 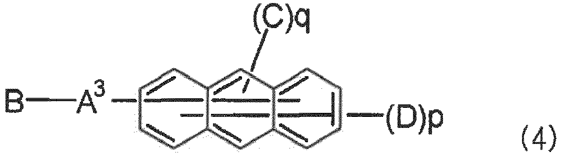 worin
A³ eine zweiwertige Gruppe eines aromatischen Kohlenwasserstoffs, eine zweiwertige Gruppe eines aromatischen Heterocyclus, eine zweiwertige Gruppe eines kondensierten polycyclischen Aromaten oder eine Einfachbindung darstellt,
B eine aromatische heterocyclische Gruppe darstellt,
C eine aromatische Kohlenwasserstoffgruppe, eine aromatische heterocyclische Gruppe oder eine kondensierte polycyclische aromatische Gruppe darstellt und, falls zwei Cs vorhanden sind, die zwei Cs gleich oder verschieden sein können,
Ds gleich oder verschieden sein können und jeweils ein Wasserstoffatom, ein Deuteriumatom, ein Fluoratom, ein Chloratom, eine Cyanogruppe, eine Trifluormethylgruppe, eine Alkylgruppe mit 1 bis 6 Kohlenstoffatomen, eine aromatische Kohlenwasserstoffgruppe, eine aromatische heterocyclische Gruppe oder eine kondensierte polycyclische aromatische Gruppe darstellen und
vorausgesetzt ist, dass eine Summe von p und q 9 ist, p 7 oder 8 bezeichnet, während q 1 oder 2 bezeichnet.

3. Organische Elektrolumineszenzvorrichtung nach Anspruch 2, wobei
das Anthracenderivat durch die nachstehende allgemeine Formel (4a) dargestellt ist: 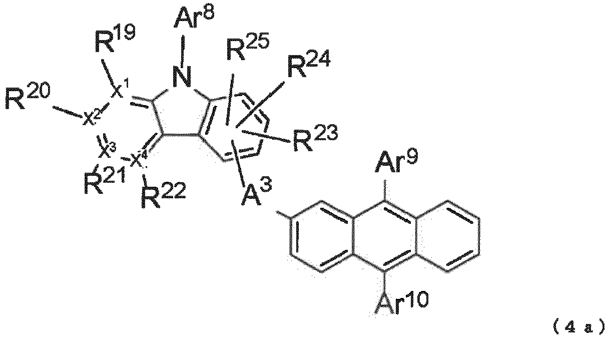 worin
A³ eine Bedeutung, wie in der allgemeinen Formel (4) definiert, aufweist,
Ar⁸ bis Ar¹⁰ gleich oder verschieden sein können und jeweils eine aromatische Kohlenwasserstoffgruppe, eine aromatische heterocyclische Gruppe oder eine kondensierte polycyclische aromatische Gruppe darstellen,
R¹⁹ bis R²⁵ gleich oder verschieden sein können und jeweils ein Wasserstoffatom, ein Deuteriumatom, ein Fluoratom, ein Chloratom, eine Cyanogruppe, eine Nitrogruppe, eine Alkylgruppe mit 1 bis 6 Kohlenstoffatomen, eine Cycloalkylgruppe mit 5 bis 10 Kohlenstoffatomen, eine Alkenylgruppe mit 2 bis 6 Kohlenstoffatomen, eine Alkyloxygruppe mit 1 bis 6 Kohlenstoffatomen, eine Cycloalkyloxygruppe mit 5 bis 10 Kohlenstoffatomen, eine aromatische Kohlenwasserstoffgruppe, eine aromatische heterocyclische Gruppe, eine kondensierte polycyclische aromatische Gruppe oder eine Aryloxygruppe darstellen und aneinander über eine Einfachbindung, eine substituierte oder unsubstituierte Methylengruppe, ein Sauerstoffatom oder ein Schwefelatom binden können, um einen Ring zu bilden, und
X¹ bis X⁴ gleich oder verschieden sein können und jeweils ein Kohlenstoffatom oder ein Stickstoffatom darstellen und nur eines von X¹ bis X⁴ ein Stickstoffatom mit der Maßgabe ist, dass das Stickstoffatom in diesem Fall nicht das Wasserstoffatom oder den Substituenten von R¹⁹ bis R²² aufweist.

4. Organische Elektrolumineszenzvorrichtung nach Anspruch 2, wobei
das Anthracenderivat durch die nachstehende allgemeine Formel (4b) dargestellt ist: 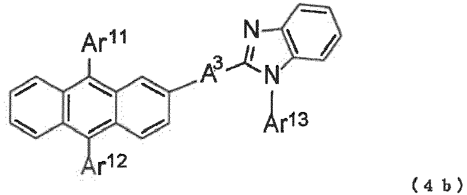 worin
A³ eine Bedeutung, wie in der allgemeinen Formel (4) definiert, aufweist und
Ar¹¹ bis Ar¹³ gleich oder verschieden sein können und jeweils eine aromatische Kohlenwasserstoffgruppe, eine aromatische heterocyclische Gruppe oder eine kondensierte polycyclische aromatische Gruppe darstellen.

5. Organische Elektrolumineszenzvorrichtung nach Anspruch 2, wobei
das Anthracenderivat durch die nachstehende allgemeine Formel (4c) dargestellt ist: 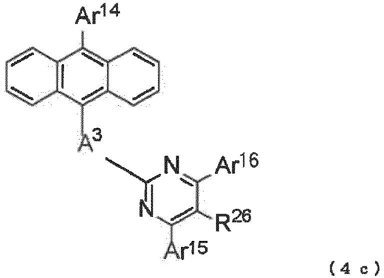 worin
A³ eine Bedeutung, wie in der allgemeinen Formel (4) definiert, aufweist,
Ar¹⁴ bis Ar¹⁶ gleich oder verschieden sein können und jeweils eine aromatische Kohlenwasserstoffgruppe, eine aromatische heterocyclische Gruppe oder eine kondensierte polycyclische aromatische Gruppe darstellen und
R²⁶ ein Wasserstoffatom, ein Deuteriumatom, ein Fluoratom, ein Chloratom, eine Cyanogruppe, eine Nitrogruppe, eine Alkylgruppe mit 1 bis 6 Kohlenstoffatomen, eine Cycloalkylgruppe mit 5 bis 10 Kohlenstoffatomen, eine Alkenylgruppe mit 2 bis 6 Kohlenstoffatomen, eine Alkyloxygruppe mit 1 bis 6 Kohlenstoffatomen, eine Cycloalkyloxygruppe mit 5 bis 10 Kohlenstoffatomen, eine aromatische Kohlenwasserstoffgruppe, eine aromatische heterocyclische Gruppe, eine kondensierte polycyclische aromatische Gruppe oder eine Aryloxygruppe darstellt.

6. Organische Elektrolumineszenzvorrichtung nach Anspruch 1, wobei
die Lochtransportschicht eine Zweischichtstruktur, zusammengesetzt aus einer ersten Lochtransportschicht (4a) und einer zweiten Lochtransportschicht (4b), aufweist und sich die zweite Lochtransportschicht neben der Leuchtschicht befindet und die Arylaminverbindung, dargestellt durch die allgemeine Formel (1), enthält.

7. Organische Elektrolumineszenzvorrichtung nach Anspruch 1, wobei
die Leuchtschicht ein Material, welches rotes Licht emittiert, enthält.

8. Organische Elektrolumineszenzvorrichtung nach Anspruch 1, wobei
die Leuchtschicht ein Material, welches Phosphoreszenz emittiert, enthält.

9. Organische Elektrolumineszenzvorrichtung nach Anspruch 8, wobei
das Material, welches Phosphoreszenz emittiert, ein iridium- oder platinhaltiger Metallkomplex ist.

## Revendications

1. Dispositif électroluminescent organique, comprenant au moins une anode (2), une couche de transport de trous (4a, 4b), une couche lumineuse (5), une couche de transport d'électrons (6) et une cathode (8), en cet ordre, dans lequel
la couche de transport de trous contient un composé arylamine représenté par la formule générale (1) suivante, et
la couche lumineuse contient un composé indénoindole représenté par la formule générale (2) suivante ou un composé carbazole représenté par la formule générale (3) suivante : 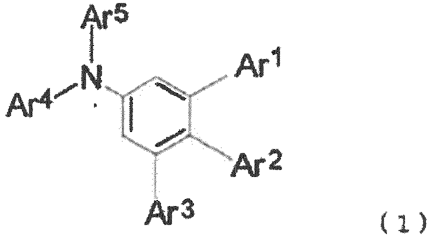 dans laquelle
Ar¹ à Ar⁵ peuvent être identiques ou différents, et chacun représente un groupe hydrocarboné aromatique, un groupe hétérocyclique aromatique, ou un groupe aromatique polycyclique condensé ; 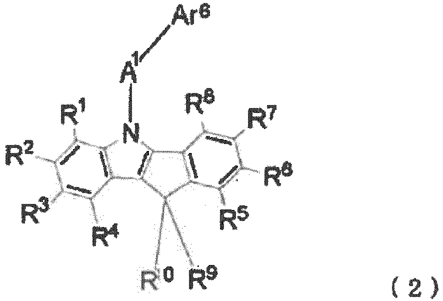 dans laquelle
A¹ représente un groupe bivalent d'un hydrocarbure aromatique, un groupe bivalent d'un hétérocyclique aromatique, un groupe bivalent d'un aromatique polycyclique condensé, ou une simple liaison ;
Ar⁶ représente un groupe hydrocarboné aromatique, un groupe hétérocyclique aromatique, ou un groupe aromatique polycyclique condensé ;
R¹ à R⁸ peuvent être identiques ou différents et chacun représente un atome d'hydrogène ; un atome de deutérium ; un atome de fluor ; un atome de chlore ; un groupe cyano ; un groupe nitro ; un groupe alkyle ayant 1 à 6 atomes de carbone ; un groupe cycloalkyle ayant 5 à 10 atomes de carbone ; un groupe alcényle ayant 2 à 6 atomes de carbone ; un groupe alkyloxy ayant 1 à 6 atomes de carbone ; un groupe cycloalkyloxy ayant 5 à 10 atomes de carbone ; un groupe hydrocarboné aromatique ; un groupe hétérocyclique aromatique ; un groupe aromatique polycyclique condensé ; un groupe aryloxy ; ou un groupe amino disubstitué substitué par un groupe choisi parmi un groupe hydrocarboné aromatique, un groupe hétérocyclique aromatique, et un groupe aromatique polycyclique condensé ; R¹ à R⁴ peuvent être liés l'un à l'autre via une simple liaison, un groupe méthylène substitué ou non substitué, un atome d'oxygène, ou un atome de soufre pour former un cycle ; R⁵ à R⁸ peuvent être liés l'un à l'autre via une simple liaison, un groupe méthylène substitué ou non substitué, un atome d'oxygène, ou un atome de soufre pour former un cycle ; en outre, en un site vacant produit par élimination de l'un des R¹ à R⁴ d'un cycle benzène, l'autre groupe R¹ à R⁴ peut se lier via un groupe méthylène substitué ou non substitué, un atome d'oxygène, un atome de soufre, ou un groupe monoarylamino pour former un cycle ; et en un site vacant produit par élimination de l'un des R⁵ à R⁸ d'un cycle benzène, l'autre groupe R⁵ à R⁸ peut se lier via un groupe méthylène substitué ou non substitué, un atome d'oxygène, un atome de soufre, ou un groupe monoarylamino pour former un cycle, et
R⁹ et R¹⁰ peuvent être identiques ou différents, et chacun représente un groupe alkyle ayant 1 à 6 atomes de carbone, un groupe hydrocarboné aromatique, un groupe hétérocyclique aromatique, ou un groupe aromatique polycyclique condensé, et ils peuvent être liés l'un à l'autre via une simple liaison, un groupe méthylène substitué ou non substitué, un atome d'oxygène, ou un atome de soufre pour former un cycle ; 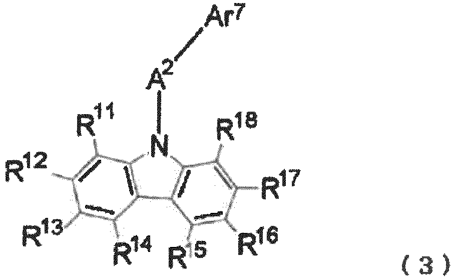 dans laquelle
A² représente un groupe bivalent d'un hydrocarbure aromatique, un groupe bivalent d'un hétérocyclique aromatique, un groupe bivalent d'un aromatique polycyclique condensé, ou une simple liaison ;
Ar⁷ représente un groupe hydrocarboné aromatique, un groupe hétérocyclique aromatique, ou un groupe aromatique polycyclique condensé ;
R¹¹ à R¹⁸ peuvent être identiques ou différents, et chacun représente un atome d'hydrogène ; un atome de deutérium ; un atome de fluor ; un atome de chlore ; un groupe cyano ; un groupe nitro ; un groupe alkyle ayant 1 à 6 atomes de carbone ; un groupe cycloalkyle ayant 5 à 10 atomes de carbone ; un groupe alcényle ayant 2 à 6 atomes de carbone ; un groupe alkyloxy ayant 1 à 6 atomes de carbone ; un groupe cycloalkyloxy ayant 5 à 10 atomes de carbone ; un groupe hydrocarboné aromatique ; un groupe hétérocyclique aromatique ; un groupe aromatique polycyclique condensé ; un groupe aryloxy ; ou un groupe amino disubstitué substitué par un groupe choisi parmi un groupe hydrocarboné aromatique, un groupe hétérocyclique aromatique, et un groupe aromatique polycyclique condensé ; R¹¹ à R¹⁴ peuvent être liés l'un à l'autre via une simple liaison, un groupe méthylène substitué ou non substitué, un atome d'oxygène ou un atome de soufre pour former un cycle ; R¹⁵ à R¹⁸ peuvent être liés l'un à l'autre via une simple liaison, un groupe méthylène substitué ou non substitué, un atome d'oxygène, ou un atome de soufre pour former un cycle ; en outre, en un site vacant produit par élimination de l'un des R¹¹ à R¹⁴ d'un cycle benzène, l'autre groupe R¹¹ à R¹⁴ peut se lier via un groupe méthylène substitué ou non substitué, un atome d'oxygène, un atome de soufre, ou un groupe monoarylamino pour former un cycle ; et en un site vacant produit par élimination de l'un des R¹⁵ à R¹⁸ d'un cycle benzène, l'autre groupe R¹⁵ à R¹⁸ peut se lier via un groupe méthylène substitué ou non substitué, un atome d'oxygène, un atome de soufre, ou un groupe monoarylamino pour former un cycle.

2. Dispositif électroluminescent organique selon la revendication 1, dans lequel
la couche de transport d'électrons contient un dérivé anthracène représenté par la formule générale (4) suivante : 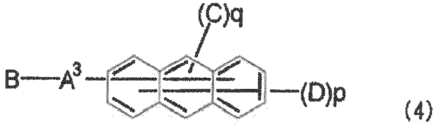 dans laquelle
A³ représente un groupe bivalent d'un hydrocarbure aromatique, un groupe bivalent d'un hétérocyclique aromatique, un groupe bivalent d'un aromatique polycyclique condensé, ou une simple liaison ;
B représente un groupe hétérocyclique aromatique,
C représente un groupe hydrocarboné aromatique, un groupe hétérocyclique aromatique, ou un groupe aromatique polycyclique condensé, et s'il y a deux C, les deux C peuvent être identiques ou différents ;
les D peuvent être identiques ou différents et chacun représente un atome d'hydrogène, un atome de deutérium, un atome de fluor, un atome de chlore, un groupe cyano, un groupe trifluorométhyle, un groupe alkyle ayant 1 à 6 atomes de carbone, un groupe hydrocarboné aromatique, un groupe hétérocyclique aromatique, ou un groupe aromatique polycyclique condensé,
pourvu que la somme de p et de q fasse 9, p représente 7 ou 8 alors que q représente 1 ou 2.

3. Dispositif électroluminescent organique selon la revendication 2, dans lequel le dérivé anthracène est représenté par la formule générale (4a) suivante : 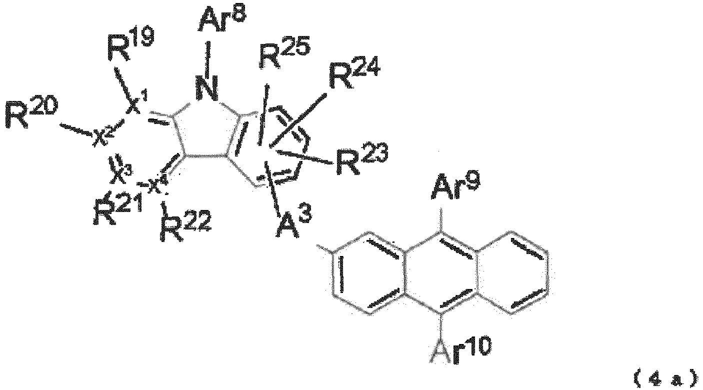 dans laquelle
A³ a la signification définie dans la formule générale (4) ;
Ar⁸ à Ar¹⁰ peuvent être identiques ou différents, et chacun représente un groupe hydrocarboné aromatique, un groupe hétérocyclique aromatique, ou un groupe aromatique polycyclique condensé ;
R¹⁹ à R²⁵ peuvent être identiques ou différents et chacun représente un atome d'hydrogène, un atome de deutérium, un atome de fluor, un atome de chlore, un groupe cyano, un groupe nitro, un groupe alkyle ayant 1 à 6 atomes de carbone, un groupe cycloalkyle ayant 5 à 10 atomes de carbone, un groupe alcényle ayant 2 à 6 atomes de carbone, un groupe alkyloxy ayant 1 à 6 atomes de carbone, un groupe cycloalkyloxy ayant 5 à 10 atomes de carbone, un groupe hydrocarboné aromatique, un groupe hétérocyclique aromatique, un groupe aromatique polycyclique condensé, ou un groupe aryloxy, et ils peuvent être liés l'un à l'autre via une simple liaison, un groupe méthylène substitué ou non substitué, un atome d'oxygène, ou un atome de soufre pour former un cycle, et
X¹ à X⁴ peuvent être identiques ou différents, et chacun représente un atome de carbone ou un atome d'azote, et uniquement l'un de X¹ à X⁴ est un atome d'azote avec la condition que l'atome d'azote ne porte pas d'atome d'hydrogène ni de substituant R¹⁹ à R²².

4. Dispositif électroluminescent organique selon la revendication 2, dans lequel
le dérivé anthracène est représenté par la formule générale (4b) suivante : 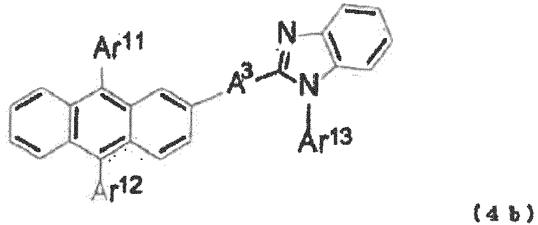 dans laquelle
A³ a la signification définie dans la formule générale (4), et
Ar¹¹ à Ar¹³ peuvent être identiques ou différents, et chacun représente un groupe hydrocarboné aromatique, un groupe hétérocyclique aromatique, ou un groupe aromatique polycyclique condensé.

5. Dispositif électroluminescent organique selon la revendication 2, dans lequel
le dérivé anthracène est représenté par la formule générale (4c) suivante : 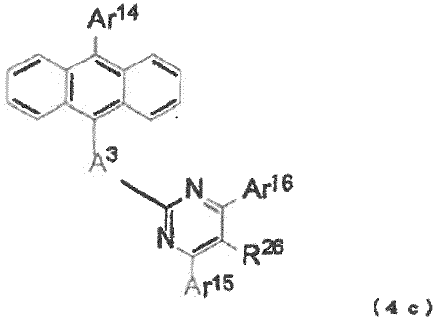 dans laquelle
A³ a la signification définie dans la formule générale (4) ;
Ar¹⁴ à Ar¹⁶ peuvent être identiques ou différents, et chacun représente un groupe hydrocarboné aromatique, un groupe hétérocyclique aromatique, ou un groupe aromatique polycyclique condensé, et
R²⁶ représente un atome d'hydrogène, un atome de deutérium, un atome de fluor, un atome de chlore, un groupe cyano, un groupe nitro, un groupe alkyle ayant 1 à 6 atomes de carbone, un groupe cycloalkyle ayant 5 à 10 atomes de carbone, un groupe alcényle ayant 2 à 6 atomes de carbone, un groupe alkyloxy ayant 1 à 6 atomes de carbone, un groupe cycloalkyloxy ayant 5 à 10 atomes de carbone, un groupe hydrocarboné aromatique, un groupe hétérocyclique aromatique, un groupe aromatique polycyclique condensé, ou un groupe aryloxy.

6. Dispositif électroluminescent organique selon la revendication 1, dans lequel
la couche de transport de trous présente une structure bicouche composée d'une première couche de transport de trous (4a) et d'une deuxième couche de transport de trous (4b), et la deuxième couche de transport de trous étant située à côté de la couche lumineuse et contenant le composé arylamine représenté par la formule générale (1).

7. Dispositif électroluminescent organique selon la revendication 1, dans lequel
la couche lumineuse contient un matériau émettant de la lumière rouge.

8. Dispositif électroluminescent organique selon la revendication 1, dans lequel
la couche lumineuse contient un matériau émettant de la phosphorescence.

9. Dispositif électroluminescent organique selon la revendication 8, dans lequel
le matériau émettant de la phosphorescence est un complexe métallique contenant de l'iridium ou du platine.
